# EUROPÄISCHE PATENTANMELDUNG

(11) **EP 4 656 604 A1**
(43) Veröffentlichungstag der Anmeldung: **03.12.2025**
(21) Anmeldenummer: 25177929.4
(22) Anmeldetag: 21.05.2025
(51) Int. Cl.: C03C 3/068, C03C 3/087, C03C 3/091, C03C 13/04, G02B 5/00, C03C 3/112

(54) **WIEDERZIEHBARES GLAS, LICHTLEITELEMENT MIT DIESEM GLAS UND DESSEN ANWENDUNGEN**

(30) Priorität: 27.05.2024 DE 102024114790
(71) Anmelder: SCHOTT AG, 55122 Mainz (DE)
(72) Erfinder: SCHREDER, Bianca, 55122 Mainz (DE)
(74) Vertreter: Schott Corporate IP

(57) **Zusammenfassung**

Die Erfindung betrifft wiederziehbares Glas, insbesondere für Lichtleitelemente (1) wie Glasfasern. Insbesondere betrifft die Erfindung Gläser, die hochtransparent sind, ein Verfahren zu deren Herstellung und deren Verwendungen. Die Gläser der Erfindung werden vorzugsweise als Kernglas in einem Licht- und/oder Bildleiter (1) verwendet. Außerdem betrifft die Erfindung einen Licht- und/oder Bildleiter (1), der das erfindungsgemäße Glas als Kernglas (2) und ein Mantelglas (3) umfasst. Die Erfindung betrifft auch die Verwendung eines solchen Glases für die Bereiche Medizintechnik, insbesondere für endoskopische Anwendungen, Abbildung, Projektion, Telekommunikation, optische Nachrichtentechnik, Mobile Drive, Lasertechnologie und Desinfektion sowie optische Elemente bzw. Vorformen (so genannte "Preformen") solcher optischen Elemente.

## Beschreibung

Die Erfindung betrifft wiederziehbares Glas, insbesondere für Glasfasern, Lichtleitelemente mit diesem Glas und Verwendungen des Glases und/oder der Lichtleitelemente. Insbesondere betrifft die Erfindung Gläser, die hochtransparent sind, ein Verfahren zu deren Herstellung und ihre Verwendungen. Die Gläser der Erfindung werden vorzugsweise als Kernglas in einem Licht- und/oder Bildleiter verwendet. Außerdem betrifft die Erfindung einen Licht- und/oder Bildleiter, der das erfindungsgemäße Glas als Kernglas und ein Mantelglas umfasst. Die Erfindung betrifft auch die Verwendung eines solchen Glases für die Bereiche Abbildung, Projektion, Telekommunikation, optische Nachrichtentechnik, Mobile Drive, Lasertechnologie und Desinfektion sowie optische Elemente bzw. Vorformen (so genannte "Preformen") solcher optischen Elemente.

### Technischer Hintergrund

Faseroptische Lichtleiter finden zunehmend größere Verbreitung bei der Lichtübertragung in den unterschiedlichsten technischen und medizinischen Bereichen, z.B. in der allgemeinen Industrietechnik, der Beleuchtungs- und Verkehrstechnik, der Automobilindustrie, in der Medizintechnik wie Dentalmedizin oder Endoskopie usw. Aufgrund ihrer guten thermischen und chemischen Widerstandsfähigkeit werden in der Regel faseroptische Lichtleiter aus Glas eingesetzt, die aus zu Faserbündeln zusammengesetzten Einzelfasern bestehen. Die einzelne Lichtleitfaser leitet das Licht durch Totalreflexion. Die am weitesten verbreiteten Lichtleitfasern sind die Stufenindexfasern, die aus einem Kern aus Kernglas bestehen, wobei das Kernglas eine konstante Brechzahl über seinen Querschnitt besitzt. Das Kernglas ist von einem Mantel aus Mantelglas umgeben, das eine niedrigere Brechzahl als das Kernglas besitzt. An der Grenzfläche zwischen Kern- und Mantelglas tritt die Totalreflexion auf.

Die Lichtmenge, die in eine solche Faser eingekoppelt werden kann, ist proportional dem Quadrat der numerischen Apertur (NA) der Faser und der Querschnittsfläche des Faserkerns. Die NA entspricht dem Sinus des Winkelbereichs innerhalb dessen Licht von der Faser aufgenommen werden kann. Der Winkelbereich wird auch als Öffnungswinkel angegeben.

Neben der numerischen Apertur spielt auch die Dämpfung des Lichts in der Faser eine große Rolle. Deshalb können nur Kerngläser mit geringer Dämpfung Verwendung finden. Die Rohstoffe zum Erschmelzen solcher Kerngläser sind auf Grund ihrer hohen Reinheit recht teuer, was zu erheblichen Herstellungskosten solcher Fasern bzw. daraus hergestellter Licht- und/oder Bildleiter führen kann. Weiterhin sollten toxische Bestandteile wie PbO, CdO, As₂O₃, BeO, HgO, Tl₂O, ThO₂ aus Gründen des Umweltschutzes nicht mehr verwendet werden.

Insbesondere bei mobilen Anwendungen ist weiterhin die Zuverlässigkeit der Faser von Bedeutung, d.h. die Alterungsbeständigkeit bei Temperaturwechselbeanspruchungen zwischen ca. - 50°C und 110°C, die Beständigkeit gegenüber mechanischen Beanspruchungen, insbesondere die Schwingungsfestigkeit sowie die chemische Beständigkeit gegenüber Umwelteinflüssen und Reinigungsprozeduren. Dabei sind insbesondere die Klimabeständigkeit und die Beständigkeit des Kerns gegenüber alkalischen Lösungen von Bedeutung. Von Bedeutung ist auch die Dichte der Fasern, da diese einen direkten Einfluss auf die Nutzlast und den Treibstoffverbrauch eines Flugzeugs oder Automobils ausübt. Die Dichte einer Stufenindexfaser wird hauptsächlich durch die Dichte des Kernglases bestimmt.

Die Herstellung optischer Stufenfasern aus Mehrkomponentengläsern erfolgt entweder über das sogenannte Doppeltiegel- oder Stab-Rohr-Verfahren. In beiden Fällen werden Kern- und Mantelglas auf Temperaturen erhitzt, die einen Viskositätsbereich zwischen 10⁵ bis 10⁶ dPas entsprechen, und dabei zu einer Faser ausgezogen. Damit eine stabile Faser niedriger Dämpfung hergestellt werden kann, müssen zum einen das Kern- und Mantelglas in einer Reihe von Eigenschaften wie Viskositätsverlauf, thermischer Ausdehnung, Kristallisationsneigung u.a.m. kompatibel zueinander sein und zum anderen eine hohe Reinheit aufweisen, die, wie erwähnt, durch reine Rohstoffe und vor allem durch das Herstellungsverfahren gewährleistet wird. Insbesondere darf es in der Grenzfläche zwischen Faserkern und Fasermantel nicht zu Reaktionen zwischen Kern- und Mantelglas, z.B. Diffusion oder Kristallisation, kommen, was eine Totalreflexion des im Faserkern geführten Lichtes stört und dadurch die Dämpfung erhöht. Darüber hinaus wird auch die mechanische Festigkeit der Faser durch Kristallisation beeinträchtigt.

Vor diesem Hintergrund beschreibt die WO 2013/104748 A1 hochtransmittive Gläser, die als Kerngläser in Lichtleitern verwendet werden können. Dort wird ein besonderer Fokus auf eine niedrige Dämpfung im nahen IR-Bereich gelegt. Insbesondere wird eine geringe Dämpfung bei einer Wellenlänge von 1050 nm erzielt.

Zunehmend spielt jedoch auch eine hohe Transmission (geringe Dämpfung) bei niedrigeren Wellenlängen für verschiedene Anwendungen eine Rolle. So wird beispielsweise bei einem als Strahlenhärtung bezeichneten Prozess unter anderem UV-A Strahlung eingesetzt, um Werkstoffe wie Lacke, Druckfarben oder Klebstoffe auszuhärten. Neben Flächenhärtung gibt es auch die sogenannte Punkthärtung, bei der die Aushärtungsstrahlung zielgenau an den gewünschten Wirkort geleitet wird, ohne dass andere Teile der herzustellenden Produkte der Bestrahlung ausgesetzt werden. UV-Punkthärtung wird beispielsweise bei der Herstellung von Herz-Kreislauf-Kathetern oder Oxygenatoren eingesetzt, um eine Klebeverbindung unterschiedlicher Materialien herzustellen. Eine hohe Transmission bei niedrigen Wellenlängen spielt zudem beispielsweise auch bei der Desinfektion oder Entkeimung mittels UV-Strahlung eine zunehmende Rolle.

Insofern besteht ein Bedarf an entsprechenden Gläsern, insbesondere für Licht- und/oder Bildleiteranwendungen.

Lichtleitelemente werden hierin auch kurz Lichtleiter genannt. Solche werden oft auch als Lichtleitfasern bezeichnet und entsprechend mit "LLF" abgekürzt und Bildleiter können auch als "BL" (deutsch) oder "IG" ("image guide" - englisch) abgekürzt sein. Für bestimmte Bildleiter wird häufig auch die Abkürzung "LFB" verwendet (aus dem Englischen "Leached Fiber Bundle").

Bildleiter umfassen dabei ggü. Lichtleitern Lichtleitfasern, die derart geordnet angeordnet sind, dass ein eingangsseitiges Bild im Wesentlichen ungestört an die Ausgangseite eines Bildleiters übertragen werden kann. Häufig wird in diesem Zusammenhang auch von einer 1:1 Ordnung oder Anordnung von Lichtleitfasern eines Bildleiters bzw. in einem Bildleiter gesprochen.

### Zusammenfassung der Offenbarung

In einem ersten Aspekt betrifft die Erfindung ein Glas umfassend SiO₂ sowie mindestens eine der beiden Komponenten Gd₂O₃ und Y₂O₃, wobei das Verhältnis der Summe der Gewichtsanteile von Gd₂O₃ und Y₂O₃ zum Gewichtsanteil von SiO₂ mindestens 0,01 beträgt, wobei der Anteil an Ta₂O₅ höchstens 10 Gew.-% beträgt, wobei der Anteil an ZrO₂ mindestens 0,1 Gew.-% beträgt und wobei das Verhältnis von wobei das Verhältnis des Gewichtsanteils an B₂O₃ zum Gewichtsanteil an SiO₂ höchstens 0,50 beträgt.

Vorteilhaft weist dieses Glas bei einer Probendicke von 25 mm und einer Wellenlänge von 380 nm eine Reintransmission von mindestens 0,900 auf.

In einem dritten Aspekt betrifft die Erfindung einen Glasartikel oder insbesondere ein Lichtleitelement, insbesondere eine Glasfaser, umfassend oder bestehend aus einem Glas (insbesondere einem Glas der Erfindung) umfassend SiO₂ sowie mindestens eine der beiden Komponenten Gd₂O₃ und Y₂O₃, wobei das Verhältnis der Summe der Gewichtsanteile von Gd₂O₃ und Y₂O₃ zum Gewichtsanteil von SiO₂ mindestens 0,01 beträgt, wobei der Anteil an Ta₂O₅ höchstens 10 Gew.-% beträgt, wobei der Anteil an ZrO₂ mindestens 0,1 Gew.-% beträgt und wobei das Verhältnis von wobei das Verhältnis des Gewichtsanteils an B₂O₃ zum Gewichtsanteil an SiO₂ höchstens 0,50 beträgt.

In einem vierten Aspekt betrifft die Erfindung ein Verfahren zur Herstellung eines Glases (insbesondere eines Glases der Erfindung) umfassend SiO₂ sowie mindestens eine der beiden Komponenten Gd₂O₃ und Y₂O₃, wobei das Verhältnis der Summe der Gewichtsanteile von Gd₂O₃ und Y₂O₃ zum Gewichtsanteil von SiO₂ mindestens 0,01 beträgt, wobei der Anteil an Ta₂O₅ höchstens 10 Gew.-% beträgt, wobei der Anteil an ZrO₂ mindestens 0,1 Gew.-% beträgt und wobei das Verhältnis von wobei das Verhältnis des Gewichtsanteils an B₂O₃ zum Gewichtsanteil an SiO₂ höchstens 0,50 beträgt,, wobei das Verfahren die folgenden Schritte umfasst:
- Schmelzen der Glasrohstoffe,
- Kühlen des erhaltenen Glases, wobei insbesondere ein Glas der Erfindung erhalten wird.

In einem fünften Aspekt betrifft die Erfindung ein Verfahren zur Herstellung eines Glasartikels (insbesondere eines Lichtleitelements) umfassend SiO₂ sowie mindestens eine der beiden Komponenten Gd₂O₃ und Y₂O₃, wobei das Verhältnis der Summe der Gewichtsanteile von Gd₂O₃ und Y₂O₃ zum Gewichtsanteil von SiO₂ mindestens 0,01 beträgt, wobei der Anteil an Ta₂O₅ höchstens 10 Gew.-% beträgt, wobei der Anteil an ZrO₂ mindestens 0,1 Gew.-% beträgt und wobei das Verhältnis von wobei das Verhältnis des Gewichtsanteils an B₂O₃ zum Gewichtsanteil an SiO₂ höchstens 0,50 beträgt, wobei das Verfahren die folgenden Schritte umfasst:
- Schmelzen der Glasrohstoffe,
- Kühlen des erhaltenen Glases, wobei insbesondere ein Glasartikel, insbesondere ein Lichtleitelement der Erfindung erhalten wird.

In einem sechsten Aspekt betrifft die Erfindung die Verwendung eines Glases (insbesondere eines Glases der Erfindung) als Faserglas, insbesondere als Kernglas in einem Licht- und/oder Bildleiter, wobei das Glas SiO₂ sowie mindestens eine der beiden Komponenten Gd₂O₃ und Y₂O₃ umfasst, wobei das Verhältnis der Summe der Gewichtsanteile von Gd₂O₃ und Y₂O₃ zum Gewichtsanteil von SiO₂ mindestens 0,01 beträgt, wobei der Anteil an Ta₂O₅ höchstens 10 Gew.-% beträgt, wobei der Anteil an ZrO₂ mindestens 0,1 Gew.-% beträgt und wobei das Verhältnis von wobei das Verhältnis des Gewichtsanteils an B₂O₃ zum Gewichtsanteil an SiO₂ höchstens 0,50 beträgt.

In einem siebten Aspekt betrifft die Erfindung die Verwendung eines Glasartikels (insbesondere eines Glases der Erfindung) als Faserglas, insbesondere als Kernglas in einem Licht- und/oder Bildleiter, wobei der Glasartikel SiO₂ sowie mindestens eine der beiden Komponenten Gd₂O₃ und Y₂O₃ umfasst, wobei das Verhältnis der Summe der Gewichtsanteile von Gd₂O₃ und Y₂O₃ zum Gewichtsanteil von SiO₂ mindestens 0,01 beträgt, wobei der Anteil an Ta₂O₅ höchstens 10 Gew.-% beträgt, wobei der Anteil an ZrO₂ mindestens 0,1 Gew.-% beträgt und wobei das Verhältnis von wobei das Verhältnis des Gewichtsanteils an B₂O₃ zum Gewichtsanteil an SiO₂ höchstens 0,50 beträgt.

In einem achten Aspekt betrifft die Erfindung die Verwendung eines Lichtleitelements, insbesondere eines Lichtleitelements entsprechend der Erfindung, in endoskopische Anwendungen, insbesondere Endoskopen, vorteilhaft Single-Use-Endoskopen, in Projektionseinrichtungen, in der optische Nachrichtentechnik, Automotive Anwendungen, Lasertechnologie und Desinfektion, wobei das Lichtleitelement ein Glas umfasst, insbesondere als Kernglas, das SiO₂ sowie mindestens eine der beiden Komponenten Gd₂O₃ und Y₂O₃ umfasst, wobei das Verhältnis der Summe der Gewichtsanteile von Gd₂O₃ und Y₂O₃ zum Gewichtsanteil von SiO₂ mindestens 0,01 beträgt, wobei der Anteil an Ta₂O₅ höchstens 10 Gew.-% beträgt, wobei der Anteil an ZrO₂ mindestens 0,1 Gew.-% beträgt und wobei das Verhältnis von wobei das Verhältnis des Gewichtsanteils an B₂O₃ zum Gewichtsanteil an SiO₂ höchstens 0,50 beträgt.

Vorteilhaft ist ein Glas, bei dem der Anteil an Y₂O₃ und Gd₂O₃ jeweils mindestens 0,1 Gew.-% beträgt, besonders vorteilhaft jeweils mindestens 0,2 Gew.-%. Dies kann die Schmelzbarkeit verbessern. Weitere Vorteile, beispielsweise die Unterdrückung von Kristalliten, werden in dieser Beschreibung ebenfalls genannt.

Die weitere Ausgestaltung der Aspekte der Erfindung kann insbesondere anhand der hierin beschriebenen Ausführungsformen erfolgen. Die Ausführungsformen beziehen sich auf sämtliche Aspekte der Erfindung und sind insbesondere auch miteinander kombinierbar.

### Detaillierte Beschreibung der Offenbarung

Die Erfindung betrifft wiederziehbares Glas, insbesondere für Glasfasern. Die Erfindung betrifft auch ein Verfahren zu deren Herstellung und ihre Verwendungen. Die Gläser der Erfindung werden vorzugsweise als Kernglas in einem Licht- und/oder Bildleiter verwendet. Außerdem betrifft die Erfindung eine Lichtleitelement, insbesondere einen Licht- und/oder Bildleiter, der das erfindungsgemäße Glas als Kernglas und ein Mantelglas umfasst.

### Glaszusammensetzung

Die Zusammensetzung der erfindungsgemäßen Gläser wird hierin in Gewichtsprozent (Gew.-%) angegeben, sofern nichts anderes angegeben ist. Sofern nichts Abweichendes angegeben ist, beziehen sich die Angaben auf die Analysezusammensetzungen. Dem Fachmann ist bekannt, wie die Zusammensetzung eines Glases analysiert werden kann. Die Analyse kann insbesondere mittels Röntgenfluoreszenzspektroskopie (englisch: "X-ray Fluorescence Spectroscopy", XRF) erfolgen. An Stellen, an denen in der vorliegenden Offenbarung auf die Synthesezusammensetzungen Bezug genommen wird, ist dies explizit kenntlich gemacht.

In einigen Ausführungsformen liegt der Anteil an SiO₂ in einem Bereich von 10 bis 55 Gew.-%, beispielsweise in einem Bereich von 15 bis 45 Gew.-%, von 17 bis 40 Gew.-%, von 18 bis 35 Gew.-%, von 20 bis 35 Gew.-%, von 22 bis 35 Gew.-%, von 23 bis 34 Gew.-%, von 24 bis 33 Gew.-%, von 24 bis 32 Gew.-%, von 25 bis 32 Gew.-%, oder von 27 bis 31 Gew.-%. In einigen Ausführungsformen beträgt der Anteil an SiO₂ mindestens 10 Gew.-%, beispielsweise mindestens 15 Gew.-%, mindestens 17 Gew.-%, mindestens 18 Gew.-%, mindestens 20 Gew.-%, mindestens 22 Gew.-%, mindestens 23 Gew.-%, mindestens 24 Gew.-% mindestens 25 Gew.-%, oder mindestens 27 Gew.-%. In einigen Ausführungsformen beträgt der Anteil an SiO₂ höchstens 55 Gew.-%, höchstens 45 Gew.-%, höchstens 40 Gew.-%, höchstens 35 Gew.-%, höchstens 34 Gew.-%, höchstens 33 Gew.-%, höchstens 32 Gew.-%, oder höchstens 31 Gew.-%.

In einigen Ausführungsformen liegt der Anteil an Gd₂O₃ in einem Bereich von 0 bis 15 Gew.-%, beispielsweise von 0 bis 10 Gew.-%, von 0 bis 9,0 Gew.-%, von 0 bis 8,0 Gew.-%, von 0,1 bis 7,0 Gew.-%, von 0,2 bis 7,0 Gew.-%, von 0,5 bis 7,0 Gew.-%, von 1,0 bis 7,0 Gew.-%, von 1,5 bis 6,0 Gew.-%, von 2,0 bis 5,5 Gew.-%, von 2,5 bis 5,0 Gew.-%, von 3,0 bis 5,5 Gew.-%, von 3,0 bis 4,5 Gew.-%, oder von 3,5 bis 4,0 Gew.-%. In einigen Ausführungsformen beträgt der Anteil an Gd₂O₃ mindestens 0,1 Gew.-%, mindestens 0,2 Gew.-%, mindestens 0,5 Gew.-%, mindestens 1,0 Gew.-%, mindestens 1,5 Gew.-%, mindestens 2,0 Gew.-%, mindestens 2,5 Gew.-%, mindestens 3,0 Gew.-%, oder mindestens 3,5 Gew.-%. In einigen Ausführungsformen beträgt der Anteil an Gd₂O₃ höchstens 15 Gew.-%, beispielsweise höchstens 10 Gew.-%, höchstens 9,0 Gew.-%, höchstens 8,0 Gew.-%, höchstens 7,0 Gew.-%, höchstens 6,0 Gew.-%, höchstens 5,5 Gew.-%, höchstens 5,0 Gew.-%, höchstens 4,5 Gew.-%, oder höchstens 4,0 Gew.-%. In einigen Ausführungsformen beträgt der Anteil an Gd₂O₃ höchstens 3,0 Gew.-%, höchstens 2,0 Gew.-%, höchstens 1,0 Gew.-%, höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Gd₂O₃.

In einigen Ausführungsformen liegt der Anteil an Y₂O₃ in einem Bereich von 0 bis 15 Gew.-%, beispielsweise von 0 bis 10 Gew.-%, von 1,0 bis 10 Gew.-%, von 0 bis 9,0 Gew.-%, von 0 bis 8,0 Gew.-%, von 0,1 bis 7,0 Gew.-%, von 0,2 bis 5,0 Gew.-%, von 0,5 bis 2,5 Gew.-%, von 0,5 bis 2,0 Gew.-%, von 0,5 bis 1,5 Gew.-%, von 2,0 bis 8,0 Gew.-%, oder von 2,5 bis 6,0 Gew.-%. In einigen Ausführungsformen beträgt der Anteil an Y₂O₃ mindestens 0,1 Gew.-%, mindestens 0,2 Gew.-%, mindestens 0,5 Gew.-%, mindestens 1,0 Gew.-%, mindestens 1,5 Gew.-%, mindestens 2,0 Gew.-%, oder mindestens 2,5 Gew.-%. In einigen Ausführungsformen beträgt der Anteil an Y₂O₃ höchstens 15 Gew.-%, beispielsweise höchstens 10 Gew.-%, höchstens 9,0 Gew.-%, höchstens 8,0 Gew.-%, höchstens 7,0 Gew.-%, höchstens 6,0 Gew.-% höchstens 5,0 Gew.-%, höchstens 2,5 Gew.-%, höchstens 2,0 Gew.-%, höchstens 1,5 Gew.-%, oder höchstens 1,0 Gew.-%, höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Y₂O₃.

In einigen Ausführungsformen liegt die Summe der Gewichtsanteile von Gd₂O₃ und Y₂O₃ in einem Bereich von 0,2 bis 20 Gew.-%, beispielsweise von 0,5 bis 15 Gew.-%, von 1,0 bis 10 Gew.-%, von 1,5 bis 9,0 Gew.-%, von 2,0 bis 8,0 Gew.-%, von 2,5 bis 7,0 Gew.-%, von 3,0 bis 6,0 Gew.-%, von 3,5 bis 5,5 Gew.-%, oder von 4,0 bis 5,0 Gew.-%. In einigen Ausführungsformen beträgt die Summe der Gewichtsanteile von Gd₂O₃ und Y₂O₃ mindestens 0,2 Gew.-%, beispielsweise mindestens 0,5 Gew.-%, mindestens 1,0 Gew.-%, mindestens 1,5 Gew.-%, mindestens 2,0 Gew.-%, mindestens 2,5 Gew.-%, mindestens 3,0 Gew.-%, mindestens 3,5 Gew.-%, oder mindestens 4,0 Gew.-%. In einigen Ausführungsformen beträgt die Summe der Gewichtsanteile von Gd₂O₃ und Y₂O₃ höchstens 20 Gew.-%, beispielsweise höchstens 15 Gew.-%, höchstens 10 Gew.-%, höchstens 9,0 Gew.-%, höchstens 8,0 Gew.-%, höchstens 7,0 Gew.-%, höchstens 6,0 Gew.-%, höchstens 5,5 Gew.-%, oder höchstens 5,0 Gew.-%.

In einigen Ausführungsformen liegt das Verhältnis der Summe der Gewichtsanteile von Gd₂O₃ und Y₂O₃ zum Gewichtsanteil von SiO₂ in einem Bereich von 0,01 bis 0,75, beispielsweise in einem Bereich von 0,02 bis 0,60, von 0,04 bis 0,50, von 0,06 bis 0,40, von 0,08 bis 0,30, von 0,09 bis 0,25, von 0,10 bis 0,20, oder von 0,12 bis 0,18. In einigen Ausführungsformen beträgt das Verhältnis der Summe der Gewichtsanteile von Gd₂O₃ und Y₂O₃ zum Gewichtsanteil von SiO₂ mindestens 0,01, beispielsweise mindestens 0,02, mindestens 0,04, mindestens 0,06, mindestens 0,08, mindestens 0,09, mindestens 0,10, oder mindestens 0,12. In einigen Ausführungsformen beträgt das Verhältnis der Summe der Gewichtsanteile von Gd₂O₃ und Y₂O₃ zum Gewichtsanteil von SiO₂ höchstens 0,75, beispielsweise höchstens 0,60, höchstens 0,50, höchstens 0,40, höchstens 0,30, höchstens 0,25, oder höchstens 0,20.

In einigen Ausführungsformen liegt der Anteil an Ta₂O₅ in einem Bereich von 0 bis 10 Gew.-%, beispielsweise von 0 bis 9,0 Gew.-%, von 0 bis 8,0 Gew.-%, von 0 bis 7,0 Gew.-%, von 0,1 bis 7,0 Gew.-%, von 0,2 bis 7,0 Gew.-%, von 0,5 bis 7,0 Gew.-%, von 1,0 bis 7,0 Gew.-%, von 2,0 bis 7,0 Gew.-%, von 2,0 bis 5,0 Gew.-%, von 2,5 bis 6,5 Gew.-%, von 3,0 bis 6,0 Gew.-%, von 3,5 bis 5,5 Gew.-%, von 4,0 bis 5,0 Gew.-%, von 4,1 bis 4,9 Gew.-%, oder von 4,2 bis 4,8 Gew.-%. In einigen Ausführungsformen beträgt der Anteil an Ta₂O₅ mindestens 0,1 Gew.-%, beispielsweise mindestens 0,2 Gew.-%, mindestens 0,5 Gew.-%, mindestens 1,0 Gew.-%, mindestens 2,0 Gew.-%, mindestens 2,5 Gew.-%, mindestens 3,0 Gew.-%, mindestens 3,5 Gew.-%, mindestens 4,0 Gew.-%, mindestens 4,1 Gew.-%, oder mindestens 4,2 Gew.-%. In einigen Ausführungsformen beträgt der Anteil an Ta₂O₅ höchstens 10 Gew.-%, beispielsweise höchstens 9,0 Gew.-%, höchstens 8,0 Gew.-%, höchstens 7,0 Gew.-%, höchstens 6,5 Gew.-%, höchstens 6,0 Gew.-%, höchstens 5,5 Gew.-%, höchstens 5,0 Gew.-%, höchstens 4,9 Gew.-%, höchstens 4,8 Gew.-%, höchstens 4,5 Gew.-%, höchstens 4,0 Gew.-%, höchstens 3,0 Gew.-%, höchstens 2,0 Gew.-%, höchstens 1,0 Gew.-%, höchstens 0,5 Gew.-%, höchstens 0,2 Gew.-%, höchstens 0,1 Gew.-%, höchstens 0,05 Gew.-%, oder höchstens 0,01 Gew.-%. In einigen besonders vorteilhaften Ausführungsformen ist das Glas frei von Ta₂O₅.

In einigen Ausführungsformen liegt die Summe der Gewichtsanteile von Ta₂O₅ und SiO₂ in einem Bereich von 20 bis 50 Gew.-%, beispielsweise in einem Bereich von >20 bis 49 Gew.-%, von 21 bis 48 Gew.-%, von 22 bis 45 Gew.-%, von 25 bis 40 Gew.-%, von 27 bis 38 Gew.-%, von 29 bis 37 Gew.-%, von 30 bis 36 Gew.-%, oder von 31 bis 35 Gew.-%. In einigen Ausführungsformen beträgt die Summe der Gewichtsanteile von Ta₂O₅ und SiO₂ mindestens 20 Gew.-% beträgt, beispielsweise mehr als 20 Gew.-%, mindestens 21 Gew.-%, mindestens 22 Gew.-%, mindestens 25 Gew.-%, mindestens 27 Gew.-%, mindestens 29 Gew.-%, mindestens 30 Gew.-%, oder mindestens 31 Gew.-%. In einigen Ausführungsformen beträgt die Summe der Gewichtsanteile von Ta₂O₅ und SiO₂ höchstens 50 Gew.-%, beispielsweise höchstens 49 Gew.-%, höchstsens 48 Gew.-%, höchstens 45 Gew.-%, höchstens 40 Gew.-%, höchstens 38 Gew.-%, höchstens 37 Gew.-%, höchstens 36 Gew.-%, oder höchstens 35 Gew.-%.

In einigen Ausführungsformen beträgt der Anteil an Y₂O₃ und Gd₂O₃ jeweils mindestens 0,1 Gew.-%, vorteilhaft mindestens 0,2 Gew.-% oder mindestens 0,5 Gew.-%. Vorteilhafte Obergrenzen der Summe von Y₂O₃ und Gd₂O₃ wurden voranstehend genannt.In einigen Ausführungsformen liegt das Verhältnis des Gewichtsanteils von Ta₂O₅ zur Summe der Gewichtsanteile von Gd₂O₃ und Y₂O₃ in einem Bereich von 0 bis 8,0, beispielsweise von 0,10 bis 6,0, von 0,20 bis 5,0, in einem Bereich von 0,25 bis 4,0, von 0,30 bis 3,5, von 0,35 bis 3,0, von 0,40 bis 2,9, von 0,45 bis 2,8, von 0,45 bis 2,5, von 0,50 bis 2,0, von 0,60 bis 1,7, von 0,70 bis 1,5, von 0,75 bis 1,2, von 0,80 bis 1,1, oder von 0,90 bis 1,0. In einigen Ausführungsformen beträgt das Verhältnis des Gewichtsanteils von Ta₂O₅ zur Summe der Gewichtsanteile von Gd₂O₃ und Y₂O₃ mindestens 0,10, beispielsweise mindestens 0,20, mindestens 0,25, mindestens 0,30, mindestens 0,35, mindestens 0,40, mindestens 0,45, mindestens 0,50, mindestens 0,60, mindestens 0,70, mindestens 0,75, mindestens 0,80, oder mindestens 0,90. In einigen Ausführungsformen beträgt das Verhältnis des Gewichtsanteils von Ta₂O₅ zur Summe der Gewichtsanteile von Gd₂O₃ und Y₂O₃ höchstens 8,0, beispielsweise höchstens 6,0, höchstens 5,0, höchstens 4,0, höchstens 3,5, höchstens 3,0, höchstens 2,9, höchstens 2,8, höchstens 2,5, höchstens 2,0, höchstens 1,7, höchstens 1,5, höchstens 1,2, höchstens 1,1, höchstens 1,0, höchstens 0,8, höchstens 0,5, höchstens 0,2, höchstens 0,1 oder sogar 0.

In einigen Ausführungsformen liegt der Anteil von BaO in einem Bereich von 0 bis 50 Gew.-%, beispielsweise von 0,1 bis 45 Gew.-%, von 1,0 bis 35 Gew.-%, von 2,0 bis 30 Gew.-%, von 5,0 bis 30 Gew.-%, von 10 bis 30 Gew.-%, von 15 bis 30 Gew.-%, von 15 bis 28 Gew.-%, von 17 bis 27 Gew.-%, von 17 bis 26 Gew.-%, von 17 bis 21 Gew.-%, oder von 19 bis 24 Gew.-%. In einigen Ausführungsformen beträgt der Anteil von BaO mindestens 0,1 Gew.-%, beispielsweise mindestens 1,0 Gew.-%, mindestens 2,0 Gew.-%, mindestens 5,0 Gew.-%, mindestens 10 Gew.-%, mindestens 15 Gew.-%, mindestens 17 Gew.-%, oder mindestens 19 Gew.-%. In einigen Ausführungsformen beträgt der Anteil von BaO höchstens 50 Gew.-%, beispielsweise höchstens 45 Gew.-%, höchstens 35 Gew.-%, höchstens 30 Gew.-%, höchstens 28 Gew.-%, höchstens 27 Gew.-%, höchstens 26 Gew.-%, höchstens 24 Gew.-%, oder höchstens 21 Gew.-%.

In einigen Ausführungsformen liegt der Anteil an La₂O₃ in einem Bereich von 0 bis 70 Gew.-%, beispielsweise von 0,1 bis 50 Gew.-%, von 2,0 bis 45 Gew.-%, von 5,0 bis 40 Gew.-%, von 10 bis 30 Gew.-%, von 15 bis 25 Gew.-%, von 15 bis 24 Gew.-%, von 17 bis 22 Gew.-%, von 17 bis 28 Gew.-%, oder von 19 bis 26 Gew.-%. In einigen Ausführungsformen beträgt der Anteil an La₂O₃ mindestens 0,1 Gew.-%, beispielsweise mindestens 2,0 Gew.-%, mindestens 5,0 Gew.-%, mindestens 10 Gew.-%, mindestens 12 Gew.-%, mindestens 15 Gew.-%, mindestens 16 Gew.-%, mindestens 17 Gew.-%, oder mindestens 19 Gew.-%. In einigen Ausführungsformen beträgt der Anteil an La₂O₃ höchstens 70 Gew.-%, beispielsweise höchstens 50 Gew.-%, höchstens 45 Gew.-%, höchstens 40 Gew.-%, höchstens 38 Gew.-%, höchstens 37 Gew.-%, höchstens 35 Gew.-%, höchstens 30 Gew.-%, höchstens 28 Gew.-%, höchstens 26 Gew.-%, höchstens 25 Gew.-%, höchstens 24 Gew.-%, oder höchstens 22 Gew.-%.

In einigen Ausführungsformen liegt die Summe der Anteile von BaO und La₂O₃ in einem Bereich von 20 Gew.-% bis 60 Gew.-%, beispielsweise von 25 bis 55 Gew.-%, von 30 bis 50 Gew.-%, von 32 bis 48 Gew.-%, von 35 bis 45 Gew.-%, oder von 38 bis 44 Gew.-%. In einigen Ausführungsformen beträgt die Summe der Anteile von BaO und La₂O₃ mindestens 20 Gew.-%, beispielsweise mindestens 25 Gew.-%, mindestens 30 Gew.-%, mindestens 32 Gew.-%, mindestens 35 Gew.-%, oder mindestens 38 Gew.-%. In einigen Ausführungsformen beträgt die Summe der Anteile von BaO und La₂O₃ höchstens 60 Gew.-%, beispielsweise höchstens 55 Gew.-%, höchstens 50 Gew.-%, höchstens 48 Gew.-%, höchstens 45 Gew.-%, oder höchstens 44 Gew.-%.

In einigen Ausführungsformen liegt das Verhältnis des Gewichtsanteils von Gd₂O₃ zum Gewichtsanteil von La₂O₃ in einem Bereich von 0,01 bis 0,75, beispielsweise von 0,02 bis 0,50, von 0,05 bis 0,35, von 0,06 bis 0,34, von 0,07 bis 0,33, von 0,10 bis 0,30, oder von 0,15 bis 0,25. In einigen Ausführungsformen beträgt das Verhältnis des Gewichtsanteils von Gd₂O₃ zum Gewichtsanteil von La₂O₃ mindestens 0,01, beispielsweise mindestens 0,02, mindestens 0,05, mindestens 0,06, mindestens 0,07, mindestens 0,10, oder mindestens 0,15. In einigen Ausführungsformen beträgt das Verhältnis des Gewichtsanteils von Gd₂O₃ zum Gewichtsanteil von La₂O₃ höchstens 0,75, beispielsweise höchstens 0,50, höchstens 0,35, höchstens 0,34, höchstens 0,33, höchstens 0,30, oder höchstens 0,25.

Dies bedeutet, dass vorteilhaft das Verhältnis des Gewichtsanteils von La₂O₃ zur Summe der Gewichtsanteile von Gd₂O₃ und Y₂O₃ höchstens 10 beträgt.La₂O₃ trägt zum Einstellen eines hohen Brechwerts des Glases bei. Daher werden allgemein relativ hohe Gehalte dieser Komponente angestrebt. Es hat sich allerdings gezeigt, dass gerade bei hohen Anteilen von La₂O₃, insbesondere im Bereich eines Mindestanteils von 15 Gew.-% oder mehr, die Schmelzbarkeit des Glases unrationeller wird. Insbesondere steigt die Schmelztemperatur und das Glas neigt zur Entglasung. Die Erfinder haben erkannt, dass die Anwesenheit von Gd₂O₃ und/oder Y₂O₃, insbesondere mit den hierin genannten Mindestgehalten, der Entglasung entgegengewirkt werden kann. Insbesondere wenn das Verhältnis von La₂O₃ zur Summe der Gewichtsanteile von Gd₂O₃ und Y₂O₃ wie beschrieben eingestellt wird, kann die Kristallisationsneigung des Glases unterdrückt werden. Es wird vermutet, dass La₂O₃ und B₂O₃ zu Lanthan-Boraten reagieren können, welche auskristallisieren. Insbesondere Gd₂O₃, aber auch Y₂O₃, vorteilhaft die Kombination beider, stabilisieren allerdings das Glasnetzwerk, so dass die Kristallisationsneigung vermindert und die Schmelzbarkeit des Glases verbessert wird.

Die Anwesenheit von insbesondere Y₂O₃, aber auch Gd₂O₃, vorzugsweise in Kombination beider, kann auch dazu beitragen, die Absorption im blauen Spektralbereich des sichtbaren Spektrums, mithin bei einer Wellenlänge von ca. 400 nm, dahingehend zu verbessern, dass dort eine bessere Transmission und weniger Absorption auftritt.

In einigen Ausführungsformen liegt die Summe der Anteile von La₂O₃ und Gd₂O₃ in einem Bereich von 10 bis 50 Gew.-%, beispielsweise von 12 bis 40 Gew.-%, von 15 bis 30 Gew.-%, von 17 bis 29 Gew.-%, von 19 bis 28 Gew.-%, von 20 bis 27 Gew.-%, oder von 21 bis 26 Gew.-%. In einigen Ausführungsformen beträgt die Summe der Anteile von La₂O₃ und Gd₂O₃ mindestens 10 Gew.-%, beispielsweise mindestens 12 Gew.-%, mindestens 15 Gew.-%, mindestens 17 Gew.-%, mindestens 19 Gew.-%, mindestens 20 Gew.-%, oder mindestens 21 Gew.-%. In einigen Ausführungsformen beträgt die Summe der Anteile von La₂O₃ und Gd₂O₃ höchstens 50 Gew.-%, beispielsweise höchstens 40 Gew.-%, höchstens 30 Gew.-%, höchstens 29 Gew.-%, höchstens 28 Gew.-%, höchstens 27 Gew.-%, oder höchstens 26 Gew.-%.

In einigen Ausführungsformen liegt das Verhältnis des Gewichtsanteils von Y₂O₃ zum Gewichtsanteil von La₂O₃ in einem Bereich von 0,01 bis 0,15, beispielsweise von 0,02 bis 0,10, von 0,03 bis 0,08, oder von 0,04 bis 0,06. In einigen Ausführungsformen beträgt das Verhältnis des Gewichtsanteils von Y₂O₃ zum Gewichtsanteil von La₂O₃ mindestens 0,01, beispielsweise mindestens 0,02, mindestens 0,03, oder mindestens 0,04. In einigen Ausführungsformen beträgt das Verhältnis des Gewichtsanteils von Y₂O₃ zum Gewichtsanteil von La₂O₃ höchstens 0,15, beispielsweise höchstens 0,10, höchstens 0,08, oder höchstens 0,06.

In einigen Ausführungsformen liegt die Summe der Anteile von La₂O₃ und Y₂O₃ in einem Bereich von 10 bis 31 Gew.-%, beispielsweise von 12 bis 29 Gew.-%, von 14 bis 27 Gew.-%, von 16 bis 25 Gew.-%, oder von 18 bis 23 Gew.-%. In einigen Ausführungsformen beträgt die Summe der Anteile von La₂O₃ und Y₂O₃ mindestens 10 Gew.-%, beispielsweise mindestens 12 Gew.-%, mindestens 14 Gew.-%, mindestens 16 Gew.-%, oder mindestens 18 Gew.-%. In einigen Ausführungsformen beträgt die Summe der Anteile von La₂O₃ und Y₂O₃ höchstens 31 Gew.-%, beispielsweise höchstens 29 Gew.-%, höchstens 27 Gew.-%, höchstens 25 Gew.-%, oder höchstens 23 Gew.-%.

In einigen Ausführungsformen liegt das Verhältnis des Gewichtsanteils von La₂O₃ zur Summe der Gewichtsanteile von Gd₂O₃ und Y₂O₃ in einem Bereich von 1,0 bis 10, beispielsweise von 2,0 bis 8,0, von 2,5 bis 7,0, von 3,0 bis 6,0, oder von 3,5 bis 5,0. In einigen Ausführungsformen beträgt das Verhältnis des Gewichtsanteils von La₂O₃ zur Summe der Gewichtsanteile von Gd₂O₃ und Y₂O₃ mindestens 1,0, beispielsweise mindestens 2,0, mindestens 2,5, mindestens 3,0, oder mindestens 3,5. In einigen Ausführungsformen beträgt das Verhältnis des Gewichtsanteils von La₂O₃ zur Summe der Gewichtsanteile von Gd₂O₃ und Y₂O₃ höchstens 10, beispielsweise höchstens 8,0, höchstens 7,0, höchstens 6,0, oder höchstens 5,0.

In einigen Ausführungsformen liegt die Summe der Anteile von La₂O₃, Gd₂O₃ und Y₂O₃ in einem Bereich von 10 bis 50 Gew.-%, beispielsweise von 12 bis 40 Gew.-%, von 15 bis 35 Gew.-%, von 18 bis 32 Gew.-%, von 20 bis 30 Gew.-%, von 21 bis 27 Gew.-%, oder von 22 bis 26 Gew.-%. In einigen Ausführungsformen beträgt die Summe der Anteile von La₂O₃, Gd₂O₃ und Y₂O₃ mindestens 10 Gew.-%, beispielsweise mindestens 12 Gew.-%, mindestens 15 Gew.-%, mindestens 18 Gew.-%, mindestens 20 Gew.-%, mindestens 21 Gew.-%, oder mindestens 22 Gew.-%. In einigen Ausführungsformen beträgt die Summe der Anteile von La₂O₃, Gd₂O₃ und Y₂O₃ höchstens 50 Gew.-%, beispielsweise höchstens 40 Gew.-%, höchstens 35 Gew.-%, höchstens 32 Gew.-%, höchstens 30 Gew.-%, höchstens 27 Gew.-%, oder höchstens 26 Gew.-%.

In einigen Ausführungsformen liegt der Anteil von B₂O₃ in einem Bereich von 0 bis 25 Gew.-%, beispielsweise von 0,1 bis 20 Gew.-%, von 0,5 bis 15 Gew.-%, von 1,0 bis 13 Gew.-%, von 2,0 bis 10 Gew.-%, von 2,0 bis 8,0 Gew.-%, von 2,0 bis 6,0 Gew.-%, von 2,0 bis 5,0 Gew.-%, von 2,5 bis 4,5 Gew.-%, von 2,5 bis 4,0 Gew.-%, oder von 3,0 bis 4,0 Gew.-%. In einigen Ausführungsformen beträgt der Anteil von B₂O₃ mindestens 0,1 Gew.-%, beispielsweise mindestens 0,2 Gew.-%, mindestens 0,5 Gew.-%, mindestens 1,0 Gew.-%, mindestens 1,5 Gew.-%, mindestens 2,0 Gew.-%, mindestens 2,5 Gew.-%, oder mindestens 3,0 Gew.-%. In einigen Ausführungsformen beträgt der Anteil von B₂O₃ höchstens 25 Gew.-%, beispielsweise höchstens 20 Gew.-%, höchstens 15 Gew.-%, höchstens 13 Gew.-%, höchstens 10 Gew.-%, höchstens 8,0 Gew.-%, höchstens 6,0 Gew.-%, höchstens 5,0 Gew.-%, höchstens 4,5 Gew.-%, oder höchstens 4,0 Gew.-%.

Erfindungsgemäß beträgt das Verhältnis des Gewichtsanteils an B₂O₃ zum Gewichtsanteil an SiO₂ höchstens 0,50. In einigen Ausführungsformen liegt das Verhältnis des Gewichtsanteils an B₂O₃ zum Gewichtsanteil an SiO₂ in einem Bereich von 0 bis 0,50, beispielsweise von 0 bis < 0,50, von 0,01 bis 0,40, von 0,02 bis 0,30, von 0,04 bis 0,20, von 0,05 bis 0,18 von 0,06 bis 0,17, von 0,08 bis 0,16, von 0,10 bis 0,15, oder von 0,11 bis 0,14. In einigen Ausführungsformen beträgt das Verhältnis des Gewichtsanteils an B₂O₃ zum Gewichtsanteil an SiO₂ mindestens 0,01, beispielsweise mindestens 0,02, mindestens 0,04, mindestens 0,05, mindestens 0,06, mindestens 0,08, mindestens 0,10, oder mindestens 0,11. In einigen Ausführungsformen ist der Gewichtsanteil von B₂O₃ kleiner als der Gewichtsanteil von SiO₂. In einigen Ausführungsformen beträgt das Verhältnis des Gewichtsanteils an B₂O₃ zum Gewichtsanteil an SiO₂ höchstens 0,50, höchstens 0,40, höchstens 0,30, höchstens 0,20, höchstens 0,18, höchstens 0,17, höchstens 0,16, höchstens 0,15, oder höchstens 0,14.

In einigen Ausführungsformen liegt die Summe der Anteile von SiO₂ und B₂O₃ in einem Bereich von 15 bis 50 Gew.-%, beispielsweise von 20 bis 44 Gew.-%, von 25 bis 40 Gew.-%, von 27 bis 37 Gew.-%, von 29 bis 35 Gew.-%, oder von 30 bis 34 Gew.-%. In einigen Ausführungsformen beträgt die Summe der Anteile von SiO₂ und B₂O₃ mindestens 15 Gew.-%, beispielsweise mindestens 20 Gew.-%, mindestens 25 Gew.-%, mindestens 27 Gew.-%, mindestens 29 Gew.-%, oder mindestens 30 Gew.-%. In einigen Ausführungsformen beträgt die Summe der Anteile von SiO₂ und B₂O₃ höchstens 50 Gew.-%, beispielsweise höchstens 44 Gew.-%, höchstens 40 Gew.-%, höchstens 37 Gew.-%, höchstens 35 Gew.-%, oder höchstens 34 Gew.-%.

In einigen Ausführungsformen liegt der Anteil an Nb₂O₅ in einem Bereich von 0 bis 10 Gew.-%, beispielsweise von 0 bis 9,0 Gew.-%, von 0 bis 8,0 Gew.-%, von 0 bis 7,0 Gew.-%, von 0,1 bis 5,0 Gew.-%, von 0,1 bis 2,0 Gew.-%, von 0,1 bis 1,5 Gew.-%, von 0,1 bis 1,0 Gew.-%, oder von 0,1 bis 0,5 Gew.-%. In einigen Ausführungsformen beträgt der Anteil an Nb₂O₅ mindestens 0,1 Gew.-%, beispielsweise mindestens 0,2 Gew.-% oder mindestens 0,5 Gew.-%. In einigen Ausführungsformen beträgt der Anteil an Nb₂O₅ höchstens 10 Gew.-%, beispielsweise höchstens 9,0 Gew.-%, höchstens 8,0 Gew.-%, höchstens 7,0 Gew.-%, höchstens 5,0 Gew.-%, höchstens 2,0 Gew.-%, höchstens 1,5 Gew.-%, höchstens 1,0 Gew.-%, höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Nb₂O₅.

In einigen Ausführungsformen liegt die Summe der Anteile von La₂O₃, Gd₂O₃, Ta₂O₅ und Nb₂O₅ in einem Bereich von 15 bis 50 Gew.-%, beispielsweise von 20 bis 45 Gew.-%, von >20 bis 40 Gew.-%, von 21 bis 38 Gew.-%, von 22 bis 36 Gew.-%, von 23 bis 34 Gew.-%, von 24 bis 32 Gew.-%, oder von 25 bis 31 Gew.-%. In einigen Ausführungsformen beträgt die Summe der Anteile von La₂O₃, Gd₂O₃, Ta₂O₅ und Nb₂O₅ mindestens 15 Gew.-%, beispielsweise mindestens 20 Gew.-%, mehr als 20 Gew.-%, mindestens 21 Gew.-%, mindestens 22 Gew.-%, mindestens 23 Gew.-%, mindestens 24 Gew.-%, oder mindestens 25 Gew.-%. In einigen Ausführungsformen beträgt die Summe der Anteile von La₂O₃, Gd₂O₃, Ta₂O₅ und Nb₂O₅ höchstens 50 Gew.-%, höchstens 45 Gew.-%, höchstens 40 Gew.-%, höchstens 38 Gew.-%, höchstens 36 Gew.-%, höchstens 34 Gew.-%, höchstens 32 Gew.-%, oder höchstens 31 Gew.-%.

In einigen Ausführungsformen ist der Gewichtsanteil von Y₂O₃ größer als der Gewichtsanteil von Nb₂O₅. In einigen Ausführungsformen liegt das Verhältnis des Gewichtsanteils von Nb₂O₅ zum Gewichtsanteil von Y₂O₃ in einem Bereich von 0 bis <1,00, beispielsweise von 0,01 bis 0,90, von 0,02 bis 0,75, von 0,05 bis 0,50, von 0,10 bis 0,35, oder von 0,15 bis 0,25. In einigen Ausführungsformen beträgt das Verhältnis des Gewichtsanteils von Nb₂O₅ zum Gewichtsanteil von Y₂O₃ mindestens 0,01, beispielsweise mindestens 0,02, mindestens 0,05, mindestens 0,10, oder mindestens 0,15. In einigen Ausführungsformen ist das Verhältnis des Gewichtsanteils von Nb₂O₅ zum Gewichtsanteil von Y₂O₃ kleiner als 1,00. In einigen Ausführungsformen beträgt das Verhältnis des Gewichtsanteils von Nb₂O₅ zum Gewichtsanteil von Y₂O₃ höchstens 0,90, höchstens 0,75, höchstens 0,50, höchstens 0,35, oder höchstens 0,25, beispielsweise höchstens 0,15, höchstens 0,10, höchstens 0,05, höchstens 0,02, höchstens 0,01, oder sogar 0.

In einigen Ausführungsformen liegt die Summe der Anteile von Ta₂O₅ und Nb₂O₅ in einem Bereich von 0 bis 10 Gew.-%, beispielsweise von 0,1 bis 9,5 Gew.-%, von 0,2 bis 9,0 Gew.-%, von 0,5 bis 8,5 Gew.-%, von 1,0 bis 8,0 Gew.-%, von 1,5 bis 7,5 Gew.-%, von 2,0 bis 7,0 Gew.-%, von 2,5 bis 7,0 Gew.-%, von 3,0 bis 7,0 Gew.-%, von 3,0 bis 6,5 Gew.-%, von 3,5 bis 5,5 Gew.-%, von 4,0 bis 5,0 Gew.-%, von 4,1 bis 4,9 Gew.-%, oder von 4,2 bis 4,8 Gew.-%. In einigen Ausführungsformen beträgt die Summe der Anteile von Ta₂O₅ und Nb₂O₅ mindestens 0,1 Gew.-%, beispielsweise mindestens 0,2 Gew.-%, mindestens 0,5 Gew.-%, mindestens 1,0 Gew.-%, mindestens 2,0 Gew.-%, mindestens 2,5 Gew.-%, mindestens 3,0 Gew.-%, mindestens 3,5 Gew.-%, mindestens 4,0 Gew.-%, mindestens 4,1 Gew.-%, oder mindestens 4,2 Gew.-%. In einigen Ausführungsformen beträgt die Summe der Anteile von Ta₂O₅ und Nb₂O₅ höchstens 10 Gew.-%, beispielsweise höchstens 9,5 Gew.-%, höchstens 9,0 Gew.-%, höchstens 8,5 Gew.-%, höchstens 8,0 Gew.-%, höchstens 7,5 Gew.-%, höchstens 7,0 Gew.-%, höchstens 6,5 Gew.-%, höchstens 6,0 Gew.-%, höchstens 5,5 Gew.-%, höchstens 5,0 Gew.-%, höchstens 4,9 Gew.-%, höchstens 4,8 Gew.-%, höchstens 4,5 Gew.-%, höchstens 4,0 Gew.-%, höchstens 3,0 Gew.-%, höchstens 2,5 Gew.-%, höchstens 2,0 Gew.-%, höchstens 1,5 Gew.-%, oder höchstens 1,0 Gew.-%.

In einigen Ausführungsformen liegt das Verhältnis des Gewichtsanteils von Nb₂O₅ zum Gewichtsanteil von Ta₂O₅ in einem Bereich von 0 bis 0,30, beispielsweise von 0 bis 0,25, von 0,01 bis 0,20, von 0,02 bis 0,10, oder von 0,03 bis 0,05. In einigen Ausführungsformen beträgt das Verhältnis des Gewichtsanteils von Nb₂O₅ zum Gewichtsanteil von Ta₂O₅ mindestens 0,01, mindestens 0,02, oder mindestens 0,03. In einigen Ausführungsformen beträgt das Verhältnis des Gewichtsanteils von Nb₂O₅ zum Gewichtsanteil von Ta₂O₅ höchstens 0,30, beispielsweise höchstens 0,25, höchstens 0,20, höchstens 0,15, höchstens 0,10, höchstens 0,08, höchstens 0,05, höchstens 0,02, höchstens 0,01, oder sogar 0.

In einigen Ausführungsformen liegt das Verhältnis des Gewichtsanteils von Nb₂O₅ zur Summe der Gewichtsanteile von Gd₂O₃ und Y₂O₃ in einem Bereich von 0 bis 0,30, beispielsweise von 0 bis 0,25, von 0,01 bis 0,20, von 0,02 bis 0,10, oder von 0,03 bis 0,05. In einigen Ausführungsformen beträgt das Verhältnis des Gewichtsanteils von Nb₂O₅ zur Summe der Gewichtsanteile von Gd₂O₃ und Y₂O₃ mindestens 0,01, mindestens 0,02, oder mindestens 0,03. In einigen Ausführungsformen beträgt das Verhältnis des Gewichtsanteils von Nb₂O₅ zur Summe der Gewichtsanteile von Gd₂O₃ und Y₂O₃ höchstens 0,30, beispielsweise höchstens 0,25, höchstens 0,20, höchstens 0,15, höchstens 0,10, höchstens 0,08, höchstens 0,05, höchstens 0,02, höchstens 0,01, oder sogar 0.

Erfindungsgemäß enthält das Glas ZrO₂. Die Untergrenze beträgt 0,1 Gew.-%. In einigen Ausführungsformen liegt der Anteil an ZrO₂ in einem Bereich von 0,1 bis 10 Gew.-%, beispielsweise von 0,1 bis 9,0 Gew.-%, von 0,5 bis 8,0 Gew.-%, von 1,0 bis 7,0 Gew.-%, von 1,0 bis 5,0 Gew.-%, von 1,5 bis 4,5 Gew.-%, von 2,0 bis 4,5 Gew.-%, von 2,0 bis 3,5 Gew.-%, oder von 2,5 bis 4,0 Gew.-%. In einigen Ausführungsformen beträgt der Anteil an ZrO₂ mindestens 0,1 Gew.-%, mindestens 0,2 Gew.-%, mindestens 0,5 Gew.-%, mindestens 1,0 Gew.-%, mindestens 1,5 Gew.-%, mindestens 2,0 Gew.-%, oder mindestens 2,5 Gew.-%. In einigen Ausführungsformen beträgt der Anteil an ZrO₂ höchstens 10 Gew.-%, beispielsweise höchstens 9,0 Gew.-%, höchstens 8,0 Gew.-%, höchstens 7,0 Gew.-%, höchstens 5,0 Gew.-%, höchstens 4,5 Gew.-%, höchstens 4,0 Gew.-%, oder höchstens 3,5 Gew.-%.

In einigen Ausführungsformen liegt die Summe der Gewichtsanteile von Nb₂O₅ und ZrO₂ in einem Bereich von 0 bis 15 Gew.-%, beispielsweise von 0,1 bis 12 Gew.-%, von 0,2 bis 10 Gew.-%, von 0,5 bis 8,0 Gew.-%, von 1,0 bis 7,0 Gew.-%, von 1,5 bis 6,0 Gew.-%, von 2,0 bis 5,0 Gew.-%, oder von 2,5 bis 4,0 Gew.-%. In einigen Ausführungsformen beträgt die Summe der Gewichtsanteile von Nb₂O₅ und ZrO₂ mindestens 0,1 Gew.-%, beispielsweise mindestens 0,2 Gew.-%, mindestens 0,5 Gew.-%, mindestens 1,0 Gew.-%, mindestens 1,5 Gew.-%, mindestens 2,0 Gew.-%, oder mindestens 2,5 Gew.-%. In einigen Ausführungsformen beträgt die Summe der Gewichtsanteile von Nb₂O₅ und ZrO₂ höchstens 15 Gew.-%, beispielsweise höchstens 12 Gew.-%, höchstens 10 Gew.-%, höchstens 8,0 Gew.-%, höchstens 7,0 Gew.-%, höchstens 6,0 Gew.-%, höchstens 5,0 Gew.-%, oder höchstens 4,0 Gew.-%.

In einigen Ausführungsformen liegt das Verhältnis des Gewichtsanteils von Nb₂O₅ zum Gewichtsanteil von ZrO₂ in einem Bereich von 0,01 bis 0,50, beispielsweise von 0,02 bis 0,30, von 0,03 bis 0,20, oder von 0,04 bis 0,10. In einigen Ausführungsformen beträgt das Verhältnis des Gewichtsanteils von Nb₂O₅ zum Gewichtsanteil von ZrO₂ mindestens 0,01, beispielsweise mindestens 0,02, mindestens 0,03, oder mindestens 0,04. In einigen Ausführungsformen beträgt das Verhältnis des Gewichtsanteils von Nb₂O₅ zum Gewichtsanteil von ZrO₂ höchstens 0,50, beispielsweise höchstens 0,30, höchstens 0,20, höchstens 0,10, höchstens 0,07, höchstens 0,05, höchstens 0,02, höchstens 0,01, oder sogar 0.

In einigen Ausführungsformen liegt der Anteil an Li₂O in einem Bereich von 0 bis 5,0 Gew.-%, beispielsweise von 0 bis 2,0 Gew.-%, von 0 bis 1,5 Gew.-%, von 0 bis 1,0 Gew.-%, von 0,1 bis 1,0, von 0,2 bis 0,9 Gew.-%, von 0,3 bis 0,9 Gew.-%, von 0,4 bis 0,9 Gew.-%, von 0,5 bis 0,9 Gew.-%, von 0,5 bis 1,5 Gew.-%, von 0,5 bis 1,1 Gew.-%, von 0,6 bis 0,9 Gew.-%, oder von 0,6 bis 0,8 Gew.-%. In einigen Ausführungsformen beträgt der Anteil an Li₂O mindestens 0,1 Gew.-%, beispielsweise mindestens 0,2 Gew.-%, mindestens 0,3 Gew.-%, mindestens 0,4 Gew.-%, mindestens 0,5 Gew.-%, oder mindestens 0,6 Gew.-%. In einigen Ausführungsformen beträgt der Anteil an Li₂O höchstens 5,0 Gew.-%, beispielsweise höchstens 2,0 Gew.-%, höchstens 1,5 Gew.-%, höchstens 1,1 Gew.-%, höchstens 1,0 Gew.-%, höchstens 0,9 Gew.-%, höchstens 0,8 Gew.-%, höchstens 0,5 Gew.-%, höchstens 0,3 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Li₂O.

In einigen Ausführungsformen liegt der Anteil an Na₂O in einem Bereich von 0 bis 5,0 Gew.-%, beispielsweise von 0 bis 2,0 Gew.-%, von 0,1 bis 2,0 Gew.-%, von 0 bis 1,5 Gew.-%, von 0,2 bis 1,5 Gew.-%, von 0 bis 1,0 Gew.-%, von 0,3 bis 1,0 Gew.-%, von 0 bis 0,5, von 0 bis 0,2 Gew.-%, oder von 0 bis 0,1 Gew.-%. In einigen Ausführungsformen beträgt der Anteil an Na₂O mindestens 0,1 Gew.-%, beispielsweise mindestens 0,2 Gew.-%, mindestens 0,3 Gew.-%, mindestens 0,4 Gew.-%, mindestens 0,5 Gew.-%, oder mindestens 0,6 Gew.-%. In einigen Ausführungsformen beträgt der Anteil an Na₂O höchstens 5,0 Gew.-%, beispielsweise höchstens 2,0 Gew.-%, höchstens 1,5 Gew.-%, höchstens 1,0 Gew.-%, höchstens 0,5 Gew.-%, höchstens 0,3 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Na₂O.

In einigen Ausführungsformen liegt die Summe der Anteile von Li₂O und Na₂O in einem Bereich von 0 bis 10 Gew.-%, beispielsweise von 0 bis 5,0 Gew.-%, von 0 bis 4,0 Gew.-%, von 0 bis 3,0 Gew.-%, von 0 bis 2,0 Gew.-%, von 0,1 bis 1,5 Gew.-%, von 0,1 bis 1,2 Gew.-%, von 0,1 bis 1,1 Gew.-%, oder von 0,2 bis 1,0 Gew.-%. In einigen Ausführungsformen beträgt die Summe der Anteile von Li₂O und Na₂O mindestens 0,1 Gew.-%, beispielsweise mindestens 0,2 Gew.-%, mindestens 0,3 Gew.-%, mindestens 0,4 Gew.-%, mindestens 0,5 Gew.-%, oder mindestens 0,6 Gew.-%. In einigen Ausführungsformen beträgt die Summe der Anteile von Li₂O und Na₂O höchstens 10 Gew.-%, beispielsweise höchstens 5,0 Gew.-%, höchstens 4,0 Gew.-%, höchstens 3,0 Gew.-%, höchstens 2,0 Gew.-%, höchstens 1,5 Gew.-%, höchstens 1,2 Gew.-%, höchstens 1,1 Gew.-%, höchstens 1,0 Gew.-%, höchstens 0,5 Gew.-%, höchstens 0,3 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Li₂O und Na₂O.

In einigen Ausführungsformen liegt der Anteil an K₂O in einem Bereich von 0 bis 5,0 Gew.-%, beispielsweise von 0 bis 2,0 Gew.-%, von 0,1 bis 2,0 Gew.-%, von 0 bis 1,5 Gew.-%, von 0,2 bis 1,5 Gew.-%, von 0 bis 1,0 Gew.-%, von 0,3 bis 1,0 Gew.-%, von 0 bis 0,5, von 0 bis 0,2 Gew.-%, oder von 0 bis 0,1 Gew.-%. In einigen Ausführungsformen beträgt der Anteil an K₂O mindestens 0,1 Gew.-%, beispielsweise mindestens 0,2 Gew.-%, mindestens 0,3 Gew.-%, mindestens 0,4 Gew.-%, mindestens 0,5 Gew.-%, oder mindestens 0,6 Gew.-%. In einigen Ausführungsformen beträgt der Anteil an K₂O höchstens 5,0 Gew.-%, beispielsweise höchstens 2,0 Gew.-%, höchstens 1,5 Gew.-%, höchstens 1,0 Gew.-%, höchstens 0,5 Gew.-%, höchstens 0,3 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von K₂O.

In einigen Ausführungsformen liegt die Summe der Anteile von Na₂O und K₂O in einem Bereich von 0 bis 10 Gew.-%, beispielsweise von 0 bis 5,0 Gew.-%, von 0 bis 4,0 Gew.-%, von 0 bis 3,0 Gew.-%, von 0 bis 2,0 Gew.-%, von 0,1 bis 1,5 Gew.-%, von 0,1 bis 1,2 Gew.-%, von 0,1 bis 1,1 Gew.-%, oder von 0,2 bis 1,0 Gew.-%. In einigen Ausführungsformen beträgt die Summe der Anteile von Na₂O und K₂O mindestens 0,1 Gew.-%, beispielsweise mindestens 0,2 Gew.-%, mindestens 0,3 Gew.-%, mindestens 0,4 Gew.-%, mindestens 0,5 Gew.-%, oder mindestens 0,6 Gew.-%. In einigen Ausführungsformen beträgt die Summe der Anteile von Na₂O und K₂O höchstens 10 Gew.-%, beispielsweise höchstens 5,0 Gew.-%, höchstens 4,0 Gew.-%, höchstens 3,0 Gew.-%, höchstens 2,0 Gew.-%, höchstens 1,5 Gew.-%, höchstens 1,2 Gew.-%, höchstens 1,1 Gew.-%, höchstens 1,0 Gew.-%, höchstens 0,5 Gew.-%, höchstens 0,3 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Na₂O und K₂O.

In einigen Ausführungsformen liegt die Summe der Anteile von Li₂O, Na₂O und K₂O in einem Bereich von 0 bis 10 Gew.-%, beispielsweise von 0 bis 5,0 Gew.-%, von 0 bis 4,0 Gew.-%, von 0 bis 3,0 Gew.-%, von 0 bis 2,0 Gew.-%, von 0,1 bis 1,5 Gew.-%, von 0,1 bis 1,2 Gew.-%, von 0,1 bis 1,1 Gew.-%, oder von 0,2 bis 1,0 Gew.-%. In einigen Ausführungsformen beträgt die Summe der Anteile von Li₂O, Na₂O und K₂O mindestens 0,1 Gew.-%, beispielsweise mindestens 0,2 Gew.-%, mindestens 0,3 Gew.-%, mindestens 0,4 Gew.-%, mindestens 0,5 Gew.-%, oder mindestens 0,6 Gew.-%. In einigen Ausführungsformen beträgt die Summe der Anteile von Li₂O, Na₂O und K₂O höchstens 10 Gew.-%, beispielsweise höchstens 5,0 Gew.-%, höchstens 4,0 Gew.-%, höchstens 3,0 Gew.-%, höchstens 2,0 Gew.-%, höchstens 1,5 Gew.-%, höchstens 1,2 Gew.-%, höchstens 1,1 Gew.-%, höchstens 1,0 Gew.-%, höchstens 0,5 Gew.-%, höchstens 0,3 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Li₂O, Na₂O und K₂O.

In einigen Ausführungsformen liegt der Anteil an Rb₂O in einem Bereich von 0 bis 5,0 Gew.-%, beispielsweise von 0 bis 2,0 Gew.-%, von 0,1 bis 2,0 Gew.-%, von 0 bis 1,5 Gew.-%, von 0,2 bis 1,5 Gew.-%, von 0 bis 1,0 Gew.-%, von 0,3 bis 1,0 Gew.-%, von 0 bis 0,5, von 0 bis 0,2 Gew.-%, oder von 0 bis 0,1 Gew.-%. In einigen Ausführungsformen beträgt der Anteil an Rb₂O mindestens 0,1 Gew.-%, beispielsweise mindestens 0,2 Gew.-%, mindestens 0,3 Gew.-%, mindestens 0,4 Gew.-%, mindestens 0,5 Gew.-%, oder mindestens 0,6 Gew.-%. In einigen Ausführungsformen beträgt der Anteil an Rb₂O höchstens 5,0 Gew.-%, beispielsweise höchstens 2,0 Gew.-%, höchstens 1,5 Gew.-%, höchstens 1,0 Gew.-%, höchstens 0,5 Gew.-%, höchstens 0,3 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Rb₂O.

In einigen Ausführungsformen liegt die Summe der Anteile von Li₂O, Na₂O, K₂O und Rb₂O in einem Bereich von 0 bis 10 Gew.-%, beispielsweise von 0 bis 5,0 Gew.-%, von 0 bis 4,0 Gew.-%, von 0 bis 3,0 Gew.-%, von 0 bis 2,0 Gew.-%, von 0,1 bis 1,5 Gew.-%, von 0,1 bis 1,2 Gew.-%, von 0,1 bis 1,1 Gew.-%, oder von 0,2 bis 1,0 Gew.-%. In einigen Ausführungsformen beträgt die Summe der Anteile von Li₂O, Na₂O, K₂O und Rb₂O mindestens 0,1 Gew.-%, beispielsweise mindestens 0,2 Gew.-%, mindestens 0,3 Gew.-%, mindestens 0,4 Gew.-%, mindestens 0,5 Gew.-%, oder mindestens 0,6 Gew.-%. In einigen Ausführungsformen beträgt die Summe der Anteile von Li₂O, Na₂O, K₂O und Rb₂O höchstens 10 Gew.-%, beispielsweise höchstens 5,0 Gew.-%, höchstens 4,0 Gew.-%, höchstens 3,0 Gew.-%, höchstens 2,0 Gew.-%, höchstens 1,5 Gew.-%, höchstens 1,2 Gew.-%, höchstens 1,1 Gew.-%, höchstens 1,0 Gew.-%, höchstens 0,5 Gew.-%, höchstens 0,3 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Li₂O, Na₂O, K₂O und Rb₂O.

In einigen Ausführungsformen liegt der Anteil an Cs₂O in einem Bereich von 0 bis 5,0 Gew.-%, beispielsweise von 0 bis 2,0 Gew.-%, von 0,1 bis 2,0 Gew.-%, von 0 bis 1,5 Gew.-%, von 0,2 bis 1,5 Gew.-%, von 0 bis 1,0 Gew.-%, von 0,3 bis 1,0 Gew.-%, von 0 bis 0,5, von 0 bis 0,2 Gew.-%, oder von 0 bis 0,1 Gew.-%. In einigen Ausführungsformen beträgt der Anteil an Cs₂O mindestens 0,1 Gew.-%, beispielsweise mindestens 0,2 Gew.-%, mindestens 0,3 Gew.-%, mindestens 0,4 Gew.-%, mindestens 0,5 Gew.-%, oder mindestens 0,6 Gew.-%. In einigen Ausführungsformen beträgt der Anteil an Cs₂O höchstens 5,0 Gew.-%, beispielsweise höchstens 2,0 Gew.-%, höchstens 1,5 Gew.-%, höchstens 1,0 Gew.-%, höchstens 0,5 Gew.-%, höchstens 0,3 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Cs₂O.

In einigen Ausführungsformen liegt die Summe der Anteile von Li₂O, Na₂O, K₂O und Cs₂O in einem Bereich von 0 bis 10 Gew.-%, beispielsweise von 0 bis 5,0 Gew.-%, von 0 bis 4,0 Gew.-%, von 0 bis 3,0 Gew.-%, von 0 bis 2,0 Gew.-%, von 0,1 bis 1,5 Gew.-%, von 0,1 bis 1,2 Gew.-%, von 0,1 bis 1,1 Gew.-%, oder von 0,2 bis 1,0 Gew.-%. In einigen Ausführungsformen beträgt die Summe der Anteile von Li₂O, Na₂O, K₂O und Cs₂O mindestens 0,1 Gew.-%, beispielsweise mindestens 0,2 Gew.-%, mindestens 0,3 Gew.-%, mindestens 0,4 Gew.-%, mindestens 0,5 Gew.-%, oder mindestens 0,6 Gew.-%. In einigen Ausführungsformen beträgt die Summe der Anteile von Li₂O, Na₂O, K₂O und Cs₂O höchstens 10 Gew.-%, beispielsweise höchstens 5,0 Gew.-%, höchstens 4,0 Gew.-%, höchstens 3,0 Gew.-%, höchstens 2,0 Gew.-%, höchstens 1,5 Gew.-%, höchstens 1,2 Gew.-%, höchstens 1,1 Gew.-%, höchstens 1,0 Gew.-%, höchstens 0,5 Gew.-%, höchstens 0,3 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Li₂O, Na₂O, K₂O und Cs₂O.

In einigen Ausführungsformen liegt die Summe der Anteile von Na₂O, K₂O und Cs₂O in einem Bereich von 0 bis 10 Gew.-%, beispielsweise von 0 bis 5,0 Gew.-%, von 0 bis 4,0 Gew.-%, von 0 bis 3,0 Gew.-%, von 0 bis 2,0 Gew.-%, von 0,1 bis 1,5 Gew.-%, von 0,1 bis 1,2 Gew.-%, von 0,1 bis 1,1 Gew.-%, oder von 0,2 bis 1,0 Gew.-%. In einigen Ausführungsformen beträgt die Summe der Anteile von Na₂O, K₂O und Cs₂O mindestens 0,1 Gew.-%, beispielsweise mindestens 0,2 Gew.-%, mindestens 0,3 Gew.-%, mindestens 0,4 Gew.-%, mindestens 0,5 Gew.-%, oder mindestens 0,6 Gew.-%. In einigen Ausführungsformen beträgt die Summe der Anteile von Na₂O, K₂O und Cs₂O höchstens 10 Gew.-%, beispielsweise höchstens 5,0 Gew.-%, höchstens 4,0 Gew.-%, höchstens 3,0 Gew.-%, höchstens 2,0 Gew.-%, höchstens 1,5 Gew.-%, höchstens 1,2 Gew.-%, höchstens 1,1 Gew.-%, höchstens 1,0 Gew.-%, höchstens 0,5 Gew.-%, höchstens 0,3 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Na₂O, K₂O und Cs₂O.

In einigen Ausführungsformen liegt die Summe der Anteile von Na₂O, K₂O, Rb₂O und Cs₂O in einem Bereich von 0 bis 10 Gew.-%, beispielsweise von 0 bis 5,0 Gew.-%, von 0 bis 4,0 Gew.-%, von 0 bis 3,0 Gew.-%, von 0 bis 2,0 Gew.-%, von 0,1 bis 1,5 Gew.-%, von 0,1 bis 1,2 Gew.-%, von 0,1 bis 1,1 Gew.-%, oder von 0,2 bis 1,0 Gew.-%. In einigen Ausführungsformen beträgt die Summe der Anteile von Na₂O, K₂O, Rb₂O und Cs₂O mindestens 0,1 Gew.-%, beispielsweise mindestens 0,2 Gew.-%, mindestens 0,3 Gew.-%, mindestens 0,4 Gew.-%, mindestens 0,5 Gew.-%, oder mindestens 0,6 Gew.-%. In einigen Ausführungsformen beträgt die Summe der Anteile von Na₂O, K₂O, Rb₂O und Cs₂O höchstens 10 Gew.-%, beispielsweise höchstens 5,0 Gew.-%, höchstens 4,0 Gew.-%, höchstens 3,0 Gew.-%, höchstens 2,0 Gew.-%, höchstens 1,5 Gew.-%, höchstens 1,2 Gew.-%, höchstens 1,1 Gew.-%, höchstens 1,0 Gew.-%, höchstens 0,5 Gew.-%, höchstens 0,3 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Na₂O, K₂O, Rb₂O und Cs₂O.

Die Ausdrücke "R₂O" oder "Σ R₂O" bezeichnen hierin die Summe der Alkalimetalloxide, also die Summe von Li₂O, Na₂O, K₂O, Rb₂O und Cs₂O. Der Anteil von R₂O ist also die Summe der Anteile von Li₂O, Na₂O, K₂O, Rb₂O und Cs₂O.

In einigen Ausführungsformen liegt der Anteil von R₂O in einem Bereich von 0 bis 10 Gew.-%, beispielsweise von 0 bis 5,0 Gew.-%, von 0 bis 4,0 Gew.-%, von 0 bis 3,0 Gew.-%, von 0 bis 2,0 Gew.-%, von 0,1 bis 1,5 Gew.-%, von 0,1 bis 1,2 Gew.-%, von 0,1 bis 1,1 Gew.-%, oder von 0,2 bis 1,0 Gew.-%. In einigen Ausführungsformen beträgt der Anteil von R₂O mindestens 0,1 Gew.-%, beispielsweise mindestens 0,2 Gew.-%, mindestens 0,3 Gew.-%, mindestens 0,4 Gew.-%, mindestens 0,5 Gew.-%, oder mindestens 0,6 Gew.-%. In einigen Ausführungsformen beträgt der Anteil von R₂O höchstens 10 Gew.-%, beispielsweise höchstens 5,0 Gew.-%, höchstens 4,0 Gew.-%, höchstens 3,0 Gew.-%, höchstens 2,0 Gew.-%, höchstens 1,5 Gew.-%, höchstens 1,2 Gew.-%, höchstens 1,1 Gew.-%, höchstens 1,0 Gew.-%, höchstens 0,5 Gew.-%, höchstens 0,3 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von R₂O.

In einigen Ausführungsformen liegt das Verhältnis des Gewichtsanteils von Li₂O zur Summe der Gewichtsanteile von Li₂O, Na₂O, K₂O, Rb₂O und Cs₂O in einem Bereich von 0,1 bis 1,0, beispielsweise von 0,3 bis 0,9, von 0,5 bis 0,8 oder von 0,6 bis 0,7. In einigen Ausführungsformen beträgt das Verhältnis des Gewichtsanteils von Li₂O zur Summe der Gewichtsanteile von Li₂O, Na₂O, K₂O, Rb₂O und Cs₂O mindestens 0,1, beispielsweise mindestens 0,1, mindestens 0,3, mindestens 0,5, mindestens 0,6, mindestens 0,7, mindestens 0,8, mindestens 0,9, oder sogar 1,0. In einigen Ausführungsformen beträgt das Verhältnis des Gewichtsanteils von Li₂O zur Summe der Gewichtsanteile von Li₂O, Na₂O, K₂O, Rb₂O und Cs₂O höchstens 0,9, beispielsweise höchstens 0,8, höchstens 0,7, höchstens 0,6, höchstens 0,5, höchstens 0,3, höchstens 0,2, höchstens 0,1, oder sogar 0.

In einigen Ausführungsformen liegt das Verhältnis des Gewichtsanteils von Na₂O zur Summe der Gewichtsanteile von Li₂O, Na₂O, K₂O, Rb₂O und Cs₂O in einem Bereich von 0 bis 0,7, beispielsweise von 0 bis 0,5, von 0 bis 0,4, oder von 0,1 bis 0,3. In einigen Ausführungsformen beträgt das Verhältnis des Gewichtsanteils von Na₂O zur Summe der Gewichtsanteile von Li₂O, Na₂O, K₂O, Rb₂O und Cs₂O mindestens 0,1, beispielsweise mindestens 0,2 oder mindestens 0,3. In einigen Ausführungsformen beträgt das Verhältnis des Gewichtsanteils von Na₂O zur Summe der Gewichtsanteile von Li₂O, Na₂O, K₂O, Rb₂O und Cs₂O höchstens 0,7, höchstens 0,5, höchstens 0,4, höchstens 0,3, höchstens 0,2, höchstens 0,1, oder sogar 0.

In einigen Ausführungsformen liegt das Verhältnis des Gewichtsanteils von K₂O zur Summe der Gewichtsanteile von Li₂O, Na₂O, K₂O, Rb₂O und Cs₂O in einem Bereich von 0 bis 0,7, beispielsweise von 0 bis 0,5, von 0 bis 0,4, oder von 0,1 bis 0,3. In einigen Ausführungsformen beträgt das Verhältnis des Gewichtsanteils von K₂O zur Summe der Gewichtsanteile von Li₂O, Na₂O, K₂O, Rb₂O und Cs₂O mindestens 0,1, beispielsweise mindestens 0,2 oder mindestens 0,3. In einigen Ausführungsformen beträgt das Verhältnis des Gewichtsanteils von K₂O zur Summe der Gewichtsanteile von Li₂O, Na₂O, K₂O, Rb₂O und Cs₂O höchstens 0,7, höchstens 0,5, höchstens 0,4, höchstens 0,3, höchstens 0,2, höchstens 0,1, oder sogar 0.

In einigen Ausführungsformen liegt das Verhältnis des Gewichtsanteils von Rb₂O zur Summe der Gewichtsanteile von Li₂O, Na₂O, K₂O, Rb₂O und Cs₂O in einem Bereich von 0 bis 0,7, beispielsweise von 0 bis 0,5, von 0 bis 0,4, oder von 0,1 bis 0,3. In einigen Ausführungsformen beträgt das Verhältnis des Gewichtsanteils von Rb₂O zur Summe der Gewichtsanteile von Li₂O, Na₂O, K₂O, Rb₂O und Cs₂O mindestens 0,1, beispielsweise mindestens 0,2 oder mindestens 0,3. In einigen Ausführungsformen beträgt das Verhältnis des Gewichtsanteils von Rb₂O zur Summe der Gewichtsanteile von Li₂O, Na₂O, K₂O, Rb₂O und Cs₂O höchstens 0,7, höchstens 0,5, höchstens 0,4, höchstens 0,3, höchstens 0,2, höchstens 0,1, oder sogar 0.

In einigen Ausführungsformen liegt das Verhältnis des Gewichtsanteils von Cs₂O zur Summe der Gewichtsanteile von Li₂O, Na₂O, K₂O, Rb₂O und Cs₂O in einem Bereich von 0 bis 0,7, beispielsweise von 0 bis 0,5, von 0 bis 0,4, oder von 0,1 bis 0,3. In einigen Ausführungsformen beträgt das Verhältnis des Gewichtsanteils von Cs₂O zur Summe der Gewichtsanteile von Li₂O, Na₂O, K₂O, Rb₂O und Cs₂O mindestens 0,1, beispielsweise mindestens 0,2 oder mindestens 0,3. In einigen Ausführungsformen beträgt das Verhältnis des Gewichtsanteils von Cs₂O zur Summe der Gewichtsanteile von Li₂O, Na₂O, K₂O, Rb₂O und Cs₂O höchstens 0,7, höchstens 0,5, höchstens 0,4, höchstens 0,3, höchstens 0,2, höchstens 0,1, oder sogar 0.

In einigen Ausführungsformen liegt das Verhältnis der Summe der Gewichtsanteile von Na₂O und K₂O zum Gewichtsanteil von Li₂O in einem Bereich von 0 bis 2,0, beispielsweise von 0 bis 1,5, von 0 bis 1,0, von 0,1 bis 0,7, oder von 0,2 bis 0,5. In einigen Ausführungsformen beträgt das Verhältnis der Summe der Gewichtsanteile von Na₂O und K₂O zum Gewichtsanteil von Li₂O mindestens 0,1, beispielsweise mindestens 0,2, mindestens 0,3, oder mindestens 0,5. In einigen Ausführungsformen beträgt das Verhältnis der Summe der Gewichtsanteile von Na₂O und K₂O zum Gewichtsanteil von Li₂O höchstens 2,0, beispielsweise höchstens 1,5, höchstens 1,0, höchstens 0,7, höchstens 0,5, höchstens 0,3, höchstens 0,2, höchstens 0,1, oder sogar 0.

In einigen Ausführungsformen liegt das Verhältnis des Gewichtsanteils von Na₂O zum Gewichtsanteil von Li₂O in einem Bereich von 0 bis 2,0, beispielsweise von 0 bis 1,5, von 0 bis 1,0, von 0,1 bis 0,7, oder von 0,2 bis 0,5. In einigen Ausführungsformen beträgt das Verhältnis des Gewichtsanteils von Na₂O zum Gewichtsanteil von Li₂O mindestens 0,1, beispielsweise mindestens 0,2, mindestens 0,3, oder mindestens 0,5. In einigen Ausführungsformen beträgt das Verhältnis des Gewichtsanteils von Na₂O zum Gewichtsanteil von Li₂O höchstens 2,0, beispielsweise höchstens 1,5, höchstens 1,0, höchstens 0,7, höchstens 0,5, höchstens 0,3, höchstens 0,2, höchstens 0,1, oder sogar 0.

In einigen Ausführungsformen liegt das Verhältnis des Gewichtsanteils von K₂O zum Gewichtsanteil von Li₂O in einem Bereich von 0 bis 2,0, beispielsweise von 0 bis 1,5, von 0 bis 1,0, von 0,1 bis 0,7, oder von 0,2 bis 0,5. In einigen Ausführungsformen beträgt das Verhältnis des Gewichtsanteils von K₂O zum Gewichtsanteil von Li₂O mindestens 0,1, beispielsweise mindestens 0,2, mindestens 0,3, oder mindestens 0,5. In einigen Ausführungsformen beträgt das Verhältnis des Gewichtsanteils von K₂O zum Gewichtsanteil von Li₂O höchstens 2,0, beispielsweise höchstens 1,5, höchstens 1,0, höchstens 0,7, höchstens 0,5, höchstens 0,3, höchstens 0,2, höchstens 0,1, oder sogar 0.

In einigen Ausführungsformen liegt der Anteil an MgO in einem Bereich von 0 bis 5,0 Gew.-%, beispielsweise von 0 bis 2,0 Gew.-%, von 0,1 bis 2,0 Gew.-%, von 0 bis 1,5 Gew.-%, von 0,2 bis 1,5 Gew.-%, von 0 bis 1,0 Gew.-%, von 0,3 bis 1,0 Gew.-%, von 0 bis 0,5, von 0 bis 0,2 Gew.-%, oder von 0 bis 0,1 Gew.-%. In einigen Ausführungsformen beträgt der Anteil an MgO mindestens 0,1 Gew.-%, beispielsweise mindestens 0,2 Gew.-%, oder mindestens 0,3 Gew.-%. In einigen Ausführungsformen beträgt der Anteil an MgO höchstens 5,0 Gew.-%, beispielsweise höchstens 2,0 Gew.-%, höchstens 1,0 Gew.-%, höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von MgO.

In einigen Ausführungsformen liegt der Anteil an CaO in einem Bereich von 0 bis 5,0 Gew.-%, beispielsweise von 0 bis 2,0 Gew.-%, von 0,1 bis 2,0 Gew.-%, von 0 bis 1,5 Gew.-%, von 0,2 bis 1,5 Gew.-%, von 0 bis 1,0 Gew.-%, von 0,3 bis 1,0 Gew.-%, von 0 bis 0,5, von 0 bis 0,2 Gew.-%, oder von 0 bis 0,1 Gew.-%. In einigen Ausführungsformen beträgt der Anteil an CaO mindestens 0,1 Gew.-%, beispielsweise mindestens 0,2 Gew.-%, oder mindestens 0,3 Gew.-%. In einigen Ausführungsformen beträgt der Anteil an CaO höchstens 5,0 Gew.-%, beispielsweise höchstens 2,0 Gew.-%, höchstens 1,0 Gew.-%, höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von CaO.

In einigen Ausführungsformen liegt der Anteil an SrO in einem Bereich von 0 bis 5,0 Gew.-%, beispielsweise von 0 bis 2,0 Gew.-%, von 0,1 bis 2,0 Gew.-%, von 0 bis 1,5 Gew.-%, von 0,2 bis 1,5 Gew.-%, von 0 bis 1,0 Gew.-%, von 0,3 bis 1,0 Gew.-%, von 0 bis 0,5, von 0 bis 0,2 Gew.-%, oder von 0 bis 0,1 Gew.-%. In einigen Ausführungsformen beträgt der Anteil an SrO mindestens 0,1 Gew.-%, beispielsweise mindestens 0,2 Gew.-%, oder mindestens 0,3 Gew.-%. In einigen Ausführungsformen beträgt der Anteil an SrO höchstens 5,0 Gew.-%, beispielsweise höchstens 2,0 Gew.-%, höchstens 1,0 Gew.-%, höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von SrO.

In einigen Ausführungsformen liegt die Summe der Anteile an MgO, CaO und SrO in einem Bereich von 0 bis 5,0 Gew.-%, beispielsweise von 0 bis 2,0 Gew.-%, von 0,1 bis 2,0 Gew.-%, von 0 bis 1,5 Gew.-%, von 0,2 bis 1,5 Gew.-%, von 0 bis 1,0 Gew.-%, von 0,3 bis 1,0 Gew.-%, von 0 bis 0,5, von 0 bis 0,2 Gew.-%, oder von 0 bis 0,1 Gew.-%. In einigen Ausführungsformen beträgt die Summe der Anteile an MgO, CaO und SrO mindestens 0,1 Gew.-%, beispielsweise mindestens 0,2 Gew.-%, oder mindestens 0,3 Gew.-%. In einigen Ausführungsformen beträgt die Summe der Anteile an MgO, CaO und SrO höchstens 5,0 Gew.-%, beispielsweise höchstens 2,0 Gew.-%, höchstens 1,0 Gew.-%, höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von MgO, CaO und SrO.

In einigen Ausführungsformen liegt das Verhältnis der Summe der Gewichtsanteile von CaO und SrO zum Gewichtsanteil von BaO in einem Bereich von 0 bis 0,20, beispielsweise von 0 bis 0,15, von 0 bis 0,10, oder von 0,01 bis 0,05. In einigen Ausführungsformen beträgt das Verhältnis der Summe der Gewichtsanteile von CaO und SrO zum Gewichtsanteil von BaO mindestens 0,01, beispielsweise mindestens 0,02, oder mindestens 0,05. In einigen Ausführungsformen beträgt das Verhältnis der Summe der Gewichtsanteile von CaO und SrO zum Gewichtsanteil von BaO höchstens 0,20, beispielsweise höchstens 0,15, höchstens 0,10, höchstens 0,05, höchstens 0,02, höchstens 0,01, oder sogar 0.

In einigen Ausführungsformen liegt das Verhältnis der Summe der Gewichtsanteile von MgO, CaO und SrO zum Gewichtsanteil von BaO in einem Bereich von 0 bis 0,20, beispielsweise von 0 bis 0,15, von 0 bis 0,10, oder von 0,01 bis 0,05. In einigen Ausführungsformen beträgt das Verhältnis der Summe der Gewichtsanteile von MgO, CaO und SrO zum Gewichtsanteil von BaO mindestens 0,01, beispielsweise mindestens 0,02, oder mindestens 0,05. In einigen Ausführungsformen beträgt das Verhältnis der Summe der Gewichtsanteile von MgO, CaO und SrO zum Gewichtsanteil von BaO höchstens 0,20, beispielsweise höchstens 0,15, höchstens 0,10, höchstens 0,05, höchstens 0,02, höchstens 0,01, oder sogar 0.

In einigen Ausführungsformen liegt die Summe der Anteile von MgO, CaO, SrO und BaO in einem Bereich von 0 bis 50 Gew.-%, beispielsweise von 0,1 bis 45 Gew.-%, von 1,0 bis 35 Gew.-%, von 2,0 bis 30 Gew.-%, von 5,0 bis 30 Gew.-%, von 10 bis 30 Gew.-%, von 15 bis 28 Gew.-%, von >15 bis 27 Gew.-%, von 17 bis 27 Gew.-%, von 17 bis 26 Gew.-%, oder von 19 bis 24 Gew.-%. In einigen Ausführungsformen beträgt die Summe der Anteile von MgO, CaO, SrO und BaO mindestens 0,1 Gew.-%, beispielsweise mindestens 1,0 Gew.-%, mindestens 2,0 Gew.-%, mindestens 5,0 Gew.-%, mindestens 10 Gew.-%, mindestens 15 Gew.-%, mehr als 15 Gew.-%, mindestens 16 Gew.-%, mindestens 17 Gew.-%, oder mindestens 19 Gew.-%. In einigen Ausführungsformen beträgt die Summe der Anteile von MgO, CaO, SrO und BaO höchstens 50 Gew.-%, beispielsweise höchstens 45 Gew.-%, höchstens 35 Gew.-%, höchstens 30 Gew.-%, höchstens 28 Gew.-%, höchstens 27 Gew.-%, höchstens 26 Gew.-%, oder höchstens 24 Gew.-%.

In einigen Ausführungsformen liegt die Summe der Anteile von CaO, SrO und BaO in einem Bereich von 0 bis 50 Gew.-%, beispielsweise von 0,1 bis 45 Gew.-%, von 1,0 bis 35 Gew.-%, von 2,0 bis 30 Gew.-%, von 5,0 bis 30 Gew.-%, von 10 bis 30 Gew.-%, von 15 bis 28 Gew.-%, von >15 bis 27 Gew.-%, von 17 bis 27 Gew.-%, von 17 bis 26 Gew.-%, oder von 19 bis 24 Gew.-%. In einigen Ausführungsformen beträgt die Summe der Anteile von CaO, SrO und BaO mindestens 0,1 Gew.-%, beispielsweise mindestens 1,0 Gew.-%, mindestens 2,0 Gew.-%, mindestens 5,0 Gew.-%, mindestens 10 Gew.-%, mindestens 15 Gew.-%, mehr als 15 Gew.-%, mindestens 16 Gew.-%, mindestens 17 Gew.-%, oder mindestens 19 Gew.-%. In einigen Ausführungsformen beträgt die Summe der Anteile von CaO, SrO und BaO höchstens 50 Gew.-%, beispielsweise höchstens 45 Gew.-%, höchstens 35 Gew.-%, höchstens 30 Gew.-%, höchstens 28 Gew.-%, höchstens 27 Gew.-%, höchstens 26 Gew.-%, oder höchstens 24 Gew.-%.

In einigen Ausführungsformen liegt der Anteil an ZnO in einem Bereich von 0 bis 30 Gew.-%, beispielsweise von 0,1 bis 25 Gew.-%, von 0,5 bis 20 Gew.-%, von 1,0 bis 18 Gew.-%, von 2,0 bis 18 Gew.-%, von 5,0 bis 18 Gew.-%, von 5,0 bis 15 Gew.-%, von 7,0 bis 14 Gew.-%, von 10 bis 18 Gew.-%, von 11 bis 17 Gew.-%, von 11 bis 16 Gew.-%, von 12 bis 15 Gew.-%, von 12 bis 15,0 Gew.-%, von 12 bis <15,0 Gew.-%, oder von 12 bis 14,5 Gew.-%. In einigen Ausführungsformen beträgt der Anteil an ZnO mindestens 0,1 Gew.-%, beispielsweise mindestens 0,5 Gew.-%, mindestens 1,0 Gew.-%, mindestens 2,0 Gew.-%, mindestens 5,0 Gew.-%, mindestens 7,0 Gew.-%, mindestens 8,0 Gew.-%, mindestens 10 Gew.-%, mindestens 11 Gew.-%, oder mindestens 12 Gew.-%. In einigen Ausführungsformen beträgt der Anteil an ZnO höchstens 30 Gew.-%, beispielsweise höchstens 25 Gew.-%, höchstens 20 Gew.-%, höchstens 18 Gew.-%, höchstens 17 Gew.-%, höchstens 16 Gew.-%, höchstens 15 Gew.-%, höchstens 15,0 Gew.-%, weniger als 15,0 Gew.-%, höchstens 14,5 Gew.-%, oder höchstens 14,0 Gew.-%.

In einigen Ausführungsformen liegt die Summe der Anteile von MgO, CaO, SrO, BaO und ZnO in einem Bereich von 0 bis 70 Gew.-%, beispielsweise von 0,1 bis 65 Gew.-%, von 1,0 bis 60 Gew.-%, von 2,0 bis 55 Gew.-%, von 5,0 bis 50 Gew.-%, von 10 bis 45 Gew.-%, von 15 bis 42 Gew.-%, von 20 bis 41 Gew.-%, von 25 bis 40 Gew.-%, von 30 bis 39 Gew.-%, von 31 bis 38 Gew.-%, oder von 32 bis 37 Gew.-%. In einigen Ausführungsformen beträgt die Summe der Anteile von MgO, CaO, SrO, BaO und ZnO mindestens 0,1 Gew.-%, beispielsweise mindestens 1,0 Gew.-%, mindestens 2,0 Gew.-%, mindestens 5,0 Gew.-%, mindestens 10 Gew.-%, mindestens 15 Gew.-%, mindestens 20 Gew.-%, mindestens 25 Gew.-%, mindestens 30 Gew.-%, mindestens 31 Gew.-%, oder mindestens 32 Gew.-%. In einigen Ausführungsformen beträgt die Summe der Anteile von MgO, CaO, SrO, BaO und ZnO höchstens 70 Gew.-%, beispielsweise höchstens 65 Gew.-%, höchstens 60 Gew.-%, höchstens 55 Gew.-%, höchstens 50 Gew.-%, höchstens 45 Gew.-%, höchstens 42 Gew.-%, höchstens 41 Gew.-%, höchstens 40 Gew.-%, höchstens 39 Gew.-%, höchstens 38 Gew.-%, oder höchstens 37 Gew.-%.

In einigen Ausführungsformen liegt die Summe der Anteile von BaO und ZnO in einem Bereich von 0 bis 70 Gew.-%, beispielsweise von 0,1 bis 65 Gew.-%, von 1,0 bis 60 Gew.-%, von 2,0 bis 55 Gew.-%, von 5,0 bis 50 Gew.-%, von 10 bis 45 Gew.-%, von 15 bis 42 Gew.-%, von 20 bis 41 Gew.-%, von 25 bis 40 Gew.-%, von 30 bis 39 Gew.-%, von 31 bis 38 Gew.-%, oder von 32 bis 37 Gew.-%. In einigen Ausführungsformen beträgt die Summe der Anteile von BaO und ZnO mindestens 0,1 Gew.-%, beispielsweise mindestens 1,0 Gew.-%, mindestens 2,0 Gew.-%, mindestens 5,0 Gew.-%, mindestens 10 Gew.-%, mindestens 15 Gew.-%, mindestens 20 Gew.-%, mindestens 25 Gew.-%, mindestens 30 Gew.-%, mindestens 31 Gew.-%, oder mindestens 32 Gew.-%. In einigen Ausführungsformen beträgt die Summe der Anteile von BaO und ZnO höchstens 70 Gew.-%, beispielsweise höchstens 65 Gew.-%, höchstens 60 Gew.-%, höchstens 55 Gew.-%, höchstens 50 Gew.-%, höchstens 45 Gew.-%, höchstens 42 Gew.-%, höchstens 41 Gew.-%, höchstens 40 Gew.-%, höchstens 39 Gew.-%, höchstens 38 Gew.-%, oder höchstens 37 Gew.-%.

In einigen Ausführungsformen liegt der Anteil an TiO₂ in einem Bereich von 0 bis 5,0 Gew.-%, beispielsweise von 0 bis 2,0 Gew.-%, von 0,1 bis 2,0 Gew.-%, von 0 bis 1,5 Gew.-%, von 0,2 bis 1,5 Gew.-%, von 0 bis 1,0 Gew.-%, von 0,3 bis 1,0 Gew.-%, von 0 bis 0,5, von 0 bis 0,2 Gew.-%, oder von 0 bis 0,1 Gew.-%. In einigen Ausführungsformen beträgt der Anteil an TiO₂ mindestens 0,1 Gew.-%, beispielsweise mindestens 0,2 Gew.-%, oder mindestens 0,3 Gew.-%. In einigen Ausführungsformen beträgt der Anteil an TiO₂ höchstens 5,0 Gew.-%, beispielsweise höchstens 2,0 Gew.-%, höchstens 1,0 Gew.-%, höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von TiO₂.

In einigen Ausführungsformen liegt die Summe der Gewichtsanteile von TiO₂, Nb₂O₅ und ZrO₂ in einem Bereich von 0 bis 15 Gew.-%, beispielsweise von 0,1 bis 12 Gew.-%, von 0,2 bis 10 Gew.-%, von 0,5 bis 8,0 Gew.-%, von 1,0 bis 7,0 Gew.-%, von 1,5 bis 6,0 Gew.-%, von 2,0 bis 5,0 Gew.-%, oder von 2,5 bis 4,0 Gew.-%. In einigen Ausführungsformen beträgt die Summe der Gewichtsanteile von TiO₂, Nb₂O₅ und ZrO₂ mindestens 0,1 Gew.-%, beispielsweise mindestens 0,2 Gew.-%, mindestens 0,5 Gew.-%, mindestens 1,0 Gew.-%, mindestens 1,5 Gew.-%, mindestens 2,0 Gew.-%, oder mindestens 2,5 Gew.-%. In einigen Ausführungsformen beträgt die Summe der Gewichtsanteile von TiO₂, Nb₂O₅ und ZrO₂ höchstens 15 Gew.-%, beispielsweise höchstens 12 Gew.-%, höchstens 10 Gew.-%, höchstens 8,0 Gew.-%, höchstens 7,0 Gew.-%, höchstens 6,0 Gew.-%, höchstens 5,0 Gew.-%, oder höchstens 4,0 Gew.-%.

In einigen Ausführungsformen liegt das Verhältnis des Gewichtsanteils von Nb₂O₅ zur Summe der Gewichtsanteile von Nb₂O₅, TiO₂ und ZrO₂ in einem Bereich von 0,01 bis 0,40, beispielsweise von 0,02 bis 0,30, von 0,03 bis 0,20, oder von 0,04 bis 0,10. In einigen Ausführungsformen beträgt das Verhältnis des Gewichtsanteils von Nb₂O₅ zur Summe der Gewichtsanteile von Nb₂O₅, TiO₂ und ZrO₂ mindestens 0,01, beispielsweise mindestens 0,02, mindestens 0,03, oder mindestens 0,04. In einigen Ausführungsformen beträgt das Verhältnis des Gewichtsanteils von Nb₂O₅ zur Summe der Gewichtsanteile von Nb₂O₅, TiO₂ und ZrO₂ höchstens 0,40, beispielsweise höchstens 0,30, höchstens 0,20, höchstens 0,10, höchstens 0,07, höchstens 0,05, höchstens 0,02, höchstens 0,01, oder sogar 0.

In einigen Ausführungsformen liegt das Verhältnis des Gewichtsanteils von TiO₂ zum Gewichtsanteil von Nb₂O₅ in einem Bereich von 0 bis 0,20, beispielsweise von 0 bis 0,15, von 0 bis 0,10, oder von 0,01 bis 0,05. In einigen Ausführungsformen beträgt das Verhältnis des Gewichtsanteils von TiO₂ zum Gewichtsanteil von Nb₂O₅ mindestens 0,01, beispielsweise mindestens 0,02, oder mindestens 0,05. In einigen Ausführungsformen beträgt das Verhältnis des Gewichtsanteils von TiO₂ zum Gewichtsanteil von Nb₂O₅ höchstens 0,20, beispielsweise höchstens 0,15, höchstens 0,10, höchstens 0,05, höchstens 0,02, höchstens 0,01, oder sogar 0.

In einigen Ausführungsformen liegt die Summe der Anteile von TiO₂ und Nb₂O₅ in einem Bereich von 0 bis 10 Gew.-%, beispielsweise von 0 bis 9,0 Gew.-%, von 0 bis 8,0 Gew.-%, von 0 bis 7,0 Gew.-%, von 0,1 bis 5,0 Gew.-%, von 0,1 bis 2,0 Gew.-%, von 0,1 bis 1,5 Gew.-%, von 0,1 bis 1,0 Gew.-%, oder von 0,1 bis 0,5 Gew.-%. In einigen Ausführungsformen beträgt die Summe der Anteile von TiO₂ und Nb₂O₅ mindestens 0,1 Gew.-%, beispielsweise mindestens 0,2 Gew.-% oder mindestens 0,5 Gew.-%. In einigen Ausführungsformen beträgt die Summe der Anteile von TiO₂ und Nb₂O₅ höchstens 10 Gew.-%, beispielsweise höchstens 9,0 Gew.-%, höchstens 8,0 Gew.-%, höchstens 7,0 Gew.-%, höchstens 5,0 Gew.-%, höchstens 2,0 Gew.-%, höchstens 1,5 Gew.-%, höchstens 1,0 Gew.-%, höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von TiO₂ und Nb₂O₅.

In einigen Ausführungsformen liegt das Verhältnis der Summe der Gewichtsanteile von TiO₂ und Nb₂O₅ zum Gewichtsanteil von SiO₂ in einem Bereich von 0 bis 0,20, beispielsweise von 0 bis 0,15, von 0 bis 0,10, oder von 0,01 bis 0,05. In einigen Ausführungsformen beträgt das Verhältnis der Summe der Gewichtsanteile von TiO₂ und Nb₂O₅ zum Gewichtsanteil von SiO₂ mindestens 0,01, beispielsweise mindestens 0,02, oder mindestens 0,05. In einigen Ausführungsformen beträgt das Verhältnis der Summe der Gewichtsanteile von TiO₂ und Nb₂O₅ zum Gewichtsanteil von SiO₂ höchstens 0,20, beispielsweise höchstens 0,15, höchstens 0,10, höchstens 0,05, höchstens 0,02, höchstens 0,01, oder sogar 0.

In einigen Ausführungsformen liegt der Anteil an WO₃ in einem Bereich von 0 bis 5,0 Gew.-%, beispielsweise von 0 bis 2,0 Gew.-%, von 0,1 bis 2,0 Gew.-%, von 0 bis 1,5 Gew.-%, von 0,2 bis 1,5 Gew.-%, von 0 bis 1,0 Gew.-%, von 0,3 bis 1,0 Gew.-%, von 0 bis 0,5, von 0 bis 0,2 Gew.-%, oder von 0 bis 0,1 Gew.-%. In einigen Ausführungsformen beträgt der Anteil an WO₃ mindestens 0,1 Gew.-%, beispielsweise mindestens 0,2 Gew.-%, oder mindestens 0,3 Gew.-%. In einigen Ausführungsformen beträgt der Anteil an WO₃ höchstens 5,0 Gew.-%, beispielsweise höchstens 2,0 Gew.-%, höchstens 1,0 Gew.-%, höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von WO₃.

In einigen Ausführungsformen liegt das Verhältnis der Summe der Gewichtsanteile von Nb₂O₅ und TiO₂ zur Summe der Gewichtsanteile von Nb₂O₅, TiO₂, Ta₂O₅ und WO₃ in einem Bereich von 0 bis 0,30, beispielsweise von 0 bis 0,25, von 0,01 bis 0,20, von 0,02 bis 0,10, oder von 0,03 bis 0,05. In einigen Ausführungsformen beträgt das Verhältnis der Summe der Gewichtsanteile von Nb₂O₅ und TiO₂ zur Summe der Gewichtsanteile von Nb₂O₅, TiO₂, Ta₂O₅ und WO₃ mindestens 0,01, mindestens 0,02, oder mindestens 0,03. In einigen Ausführungsformen beträgt das Verhältnis der Summe der Gewichtsanteile von Nb₂O₅ und TiO₂ zur Summe der Gewichtsanteile von Nb₂O₅, TiO₂, Ta₂O₅ und WO₃ höchstens 0,30, beispielsweise höchstens 0,25, höchstens 0,20, höchstens 0,15, höchstens 0,10, höchstens 0,08, höchstens 0,05, höchstens 0,02, höchstens 0,01, oder sogar 0.

In einigen Ausführungsformen liegt die Summe der Anteile von TiO₂, Nb₂O₅ und WO₃ in einem Bereich von 0 bis 10 Gew.-%, beispielsweise von 0 bis 9,0 Gew.-%, von 0 bis 8,0 Gew.-%, von 0 bis 7,0 Gew.-%, von 0,1 bis 5,0 Gew.-%, von 0,1 bis 2,0 Gew.-%, von 0,1 bis 1,5 Gew.-%, von 0,1 bis 1,0 Gew.-%, oder von 0,1 bis 0,5 Gew.-%. In einigen Ausführungsformen beträgt die Summe der Anteile von TiO₂, Nb₂O₅ und WO₃ mindestens 0,1 Gew.-%, beispielsweise mindestens 0,2 Gew.-% oder mindestens 0,5 Gew.-%. In einigen Ausführungsformen beträgt die Summe der Anteile von TiO₂, Nb₂O₅ und WO₃ höchstens 10 Gew.-%, beispielsweise höchstens 9,0 Gew.-%, höchstens 8,0 Gew.-%, höchstens 7,0 Gew.-%, höchstens 5,0 Gew.-%, höchstens 2,0 Gew.-%, höchstens 1,5 Gew.-%, höchstens 1,0 Gew.-%, höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von TiO₂, Nb₂O₅ und WO₃.

In einigen Ausführungsformen liegt die Summe der Anteile von Nb₂O₅ und WO₃ in einem Bereich von 0 bis 10 Gew.-%, beispielsweise von 0 bis 9,0 Gew.-%, von 0 bis 8,0 Gew.-%, von 0 bis 7,0 Gew.-%, von 0,1 bis 5,0 Gew.-%, von 0,1 bis 2,0 Gew.-%, von 0,1 bis 1,5 Gew.-%, von 0,1 bis 1,0 Gew.-%, oder von 0,1 bis 0,5 Gew.-%. In einigen Ausführungsformen beträgt die Summe der Anteile von Nb₂O₅ und WO₃ mindestens 0,1 Gew.-%, beispielsweise mindestens 0,2 Gew.-% oder mindestens 0,5 Gew.-%. In einigen Ausführungsformen beträgt die Summe der Anteile von Nb₂O₅ und WO₃ höchstens 10 Gew.-%, beispielsweise höchstens 9,0 Gew.-%, höchstens 8,0 Gew.-%, höchstens 7,0 Gew.-%, höchstens 5,0 Gew.-%, höchstens 2,0 Gew.-%, höchstens 1,5 Gew.-%, höchstens 1,0 Gew.-%, höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Nb₂O₅ und WO₃.

In einigen Ausführungsformen liegt das Verhältnis des Gewichtsanteils an WO₃ zum Gewichtsanteil an ZnO in einem Bereich von 0 bis 0,20, beispielsweise von 0 bis 0,15, von 0 bis 0,10, oder von 0,01 bis 0,05. In einigen Ausführungsformen liegt das Verhältnis des Gewichtsanteils an WO₃ zum Gewichtsanteil an ZnO mindestens 0,01, beispielsweise mindestens 0,02, oder mindestens 0,05. In einigen Ausführungsformen liegt das Verhältnis des Gewichtsanteils an WO₃ zum Gewichtsanteil an ZnO höchstens 0,20, beispielsweise höchstens 0,15, höchstens 0,10, höchstens 0,05, höchstens 0,02, höchstens 0,01, oder sogar 0.

In einigen Ausführungsformen liegt das Verhältnis des Gewichtsanteils an WO₃ zum Gewichtsanteil an Ta₂O₅ in einem Bereich von 0 bis 0,20, beispielsweise von 0 bis 0,15, von 0 bis 0,10, oder von 0,01 bis 0,05. In einigen Ausführungsformen liegt das Verhältnis des Gewichtsanteils an WO₃ zum Gewichtsanteil an Ta₂O₅ mindestens 0,01, beispielsweise mindestens 0,02, oder mindestens 0,05. In einigen Ausführungsformen liegt das Verhältnis des Gewichtsanteils an WO₃ zum Gewichtsanteil an Ta₂O₅ höchstens 0,20, beispielsweise höchstens 0,15, höchstens 0,10, höchstens 0,05, höchstens 0,02, höchstens 0,01, oder sogar 0.

In einigen Ausführungsformen liegt das Verhältnis der Summe der Gewichtsanteile von WO₃ und TiO₂ zur Summe der Gewichtsanteile von Nb₂O₅ und SiO₂ in einem Bereich von 0 bis 0,20, beispielsweise von 0 bis 0,15, von 0 bis 0,10, oder von 0,01 bis 0,05. In einigen Ausführungsformen beträgt das Verhältnis der Summe der Gewichtsanteile von WO₃ und TiO₂ zur Summe der Gewichtsanteile von Nb₂O₅ und SiO₂ mindestens 0,01, beispielsweise mindestens 0,02, oder mindestens 0,05. In einigen Ausführungsformen beträgt das Verhältnis der Summe der Gewichtsanteile von WO₃ und TiO₂ zur Summe der Gewichtsanteile von Nb₂O₅ und SiO₂ höchstens 0,20, beispielsweise höchstens 0,15, höchstens 0,10, höchstens 0,05, höchstens 0,02, höchstens 0,01, oder sogar 0.

In einigen Ausführungsformen liegt der Anteil an Al₂O₃ in einem Bereich von 0 bis 5,0 Gew.-%, beispielsweise von 0 bis 2,0 Gew.-%, von 0,1 bis 2,0 Gew.-%, von 0 bis 1,5 Gew.-%, von 0,2 bis 1,5 Gew.-%, von 0 bis 1,0 Gew.-%, von 0,3 bis 1,0 Gew.-%, von 0 bis 0,5, von 0 bis 0,2 Gew.-%, oder von 0 bis 0,1 Gew.-%. In einigen Ausführungsformen beträgt der Anteil an Al₂O₃ mindestens 0,1 Gew.-%, beispielsweise mindestens 0,2 Gew.-%, oder mindestens 0,3 Gew.-%. In einigen Ausführungsformen beträgt der Anteil an Al₂O₃ höchstens 5,0 Gew.-%, beispielsweise höchstens 2,0 Gew.-%, höchstens 1,0 Gew.-%, höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Al₂O₃.

In einigen Ausführungsformen liegt der Anteil an Ga₂O₃ in einem Bereich von 0 bis 5,0 Gew.-%, beispielsweise von 0 bis 2,0 Gew.-%, von 0,1 bis 2,0 Gew.-%, von 0 bis 1,5 Gew.-%, von 0,2 bis 1,5 Gew.-%, von 0 bis 1,0 Gew.-%, von 0,3 bis 1,0 Gew.-%, von 0 bis 0,5, von 0 bis 0,2 Gew.-%, oder von 0 bis 0,1 Gew.-%. In einigen Ausführungsformen beträgt der Anteil an Ga₂O₃ mindestens 0,1 Gew.-%, beispielsweise mindestens 0,2 Gew.-%, oder mindestens 0,3 Gew.-%. In einigen Ausführungsformen beträgt der Anteil an Ga₂O₃ höchstens 5,0 Gew.-%, beispielsweise höchstens 2,0 Gew.-%, höchstens 1,0 Gew.-%, höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Ga₂O₃.

In einigen Ausführungsformen liegt die Summe der Anteile von Al₂O₃ und Ga₂O₃ in einem Bereich von 0 bis 5,0 Gew.-%, beispielsweise von 0 bis 2,0 Gew.-%, von 0,1 bis 2,0 Gew.-%, von 0 bis 1,5 Gew.-%, von 0,2 bis 1,5 Gew.-%, von 0 bis 1,0 Gew.-%, von 0,3 bis 1,0 Gew.-%, von 0 bis 0,5, von 0 bis 0,2 Gew.-%, oder von 0 bis 0,1 Gew.-%. In einigen Ausführungsformen beträgt die Summe der Anteile von Al₂O₃ und Ga₂O₃ mindestens 0,1 Gew.-%, beispielsweise mindestens 0,2 Gew.-%, oder mindestens 0,3 Gew.-%. In einigen Ausführungsformen beträgt die Summe der Anteile von Al₂O₃ und Ga₂O₃ höchstens 5,0 Gew.-%, beispielsweise höchstens 2,0 Gew.-%, höchstens 1,0 Gew.-%, höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Al₂O₃ und Ga₂O₃.

In einigen Ausführungsformen liegt die Summe der Anteile von B₂O₃, Al₂O₃ und Ga₂O₃ in einem Bereich von 0 bis 25 Gew.-%, beispielsweise von 0,1 bis 20 Gew.-%, von 0,5 bis 15 Gew.-%, von 1,0 bis 13 Gew.-%, von 2,0 bis 10 Gew.-%, von 2,0 bis 8,0 Gew.-%, von 2,0 bis 6,0 Gew.-%, von 2,0 bis 5,0 Gew.-%, von 2,5 bis 4,5 Gew.-%, oder von 2,5 bis 4,0 Gew.-%. In einigen Ausführungsformen beträgt die Summe der Anteile von B₂O₃, Al₂O₃ und Ga₂O₃ mindestens 0,1 Gew.-%, beispielsweise mindestens 0,2 Gew.-%, mindestens 0,5 Gew.-%, mindestens 1,0 Gew.-%, mindestens 1,5 Gew.-%, mindestens 2,0 Gew.-%, oder mindestens 2,5 Gew.-%. In einigen Ausführungsformen beträgt die Summe der Anteile von B₂O₃, Al₂O₃ und Ga₂O₃ höchstens 25 Gew.-%, beispielsweise höchstens 20 Gew.-%, höchstens 15 Gew.-%, höchstens 13 Gew.-%, höchstens 10 Gew.-%, höchstens 8,0 Gew.-%, höchstens 6,0 Gew.-%, höchstens 5,0 Gew.-%, höchstens 4,5 Gew.-%, oder höchstens 4,0 Gew.-%.

In einigen Ausführungsformen liegt die Summe der Gewichtsanteile von SiO₂ und Al₂O₃ in einem Bereich von 10 bis 55 Gew.-%, beispielsweise in einem Bereich von 15 bis 45 Gew.-%, von 17 bis 40 Gew.-%, von 18 bis 35 Gew.-%, von 20 bis 35 Gew.-%, von 22 bis 35 Gew.-%, von 23 bis 34 Gew.-%, von 24 bis 33 Gew.-%, oder von 25 bis 32 Gew.-%. In einigen Ausführungsformen beträgt die Summe der Gewichtsanteile von SiO₂ und Al₂O₃ mindestens 10 Gew.-%, beispielsweise mindestens 15 Gew.-%, mindestens 17 Gew.-%, mindestens 18 Gew.-%, mindestens 20 Gew.-%, mindestens 22 Gew.-%, mindestens 23 Gew.-%, mindestens 24 Gew.-% oder mindestens 25 Gew.-%. In einigen Ausführungsformen beträgt die Summe der Gewichtsanteile von SiO₂ und Al₂O₃ höchstens 55 Gew.-%, höchstens 45 Gew.-%, höchstens 40 Gew.-%, höchstens 35 Gew.-%, höchstens 34 Gew.-%, höchstens 33 Gew.-%, oder höchstens 32 Gew.-%.

In einigen Ausführungsformen liegt die Summe der Anteile von Bi₂O₃, La₂O₃, Gd₂O₃, Ta₂O₅, TiO₂, Nb₂O₅ und WO₃ in einem Bereich von 15 bis 50 Gew.-%, beispielsweise von 20 bis 45 Gew.-%, von >20 bis 40 Gew.-%, von 21 bis 38 Gew.-%, von 22 bis 36 Gew.-%, von 23 bis 34 Gew.-%, von 24 bis 32 Gew.-%, oder von 25 bis 31 Gew.-%. In einigen Ausführungsformen beträgt die Summe der Anteile von Bi₂O₃, La₂O₃, Gd₂O₃, Ta₂O₅, TiO₂, Nb₂O₅ und WO₃ mindestens 15 Gew.-%, beispielsweise mindestens 20 Gew.-%, mehr als 20 Gew.-%, mindestens 21 Gew.-%, mindestens 22 Gew.-%, mindestens 23 Gew.-%, mindestens 24 Gew.-%, oder mindestens 25 Gew.-%. In einigen Ausführungsformen beträgt die Summe der Anteile von Bi₂O₃, La₂O₃, Gd₂O₃, Ta₂O₅, TiO₂, Nb₂O₅ und WO₃ höchstens 50 Gew.-%, höchstens 45 Gew.-%, höchstens 40 Gew.-%, höchstens 38 Gew.-%, höchstens 36 Gew.-%, höchstens 34 Gew.-%, höchstens 32 Gew.-%, oder höchstens 31 Gew.-%.

In einigen Ausführungsformen liegt die Summe der Gewichtsanteile von F, Bi₂O₃, TiO₂, WO₃, Nb₂O₅ und K₂O in einem Bereich von 0 bis 10 Gew.-%, beispielsweise von 0 bis 9,0 Gew.-%, von 0 bis 8,0 Gew.-%, von 0 bis 7,0 Gew.-%, von 0,1 bis 5,0 Gew.-%, von 0,1 bis 2,0 Gew.-%, von 0,1 bis 1,5 Gew.-%, von 0,1 bis 1,0 Gew.-%, oder von 0,1 bis 0,5 Gew.-%. In einigen Ausführungsformen beträgt die Summe der Gewichtsanteile von F, Bi₂O₃, TiO₂, WO₃, Nb₂O₅ und K₂O mindestens 0,1 Gew.-%, beispielsweise mindestens 0,2 Gew.-% oder mindestens 0,5 Gew.-%. In einigen Ausführungsformen beträgt die Summe der Gewichtsanteile von F, Bi₂O₃, TiO₂, WO₃, Nb₂O₅ und K₂O höchstens 10 Gew.-%, beispielsweise höchstens 9,0 Gew.-%, höchstens 8,0 Gew.-%, höchstens 7,0 Gew.-%, höchstens 5,0 Gew.-%, höchstens 2,0 Gew.-%, höchstens 1,5 Gew.-%, höchstens 1,0 Gew.-%, weniger als 1,0 Gew.-%, höchstens 0,9 Gew.-%, höchstens 0,8 Gew.-%, höchstens 0,7 Gew.-%, höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von F, Bi₂O₃, TiO₂, WO₃, Nb₂O₅ und K₂O.

In einigen Ausführungsformen enthält das Glas Läutermittel ausgewählt aus Sb₂O₃, As₂O₃, SO₃, SnO₂, CI und Kombinationen von zwei oder mehr davon, insbesondere in einem Gesamtanteil von 0,01 bis 2,00 Gew.-%, beispielsweise von 0,01 bis 1,50 Gew.-%, von 0,01 bis 1,00 Gew.-%, von 0,01 bis 0,75 Gew.-%, von 0,01 bis 0,50 Gew.-%, von 0,01 bis 0,25 Gew.-%, von 0,01 bis 0,20 Gew.-%, von 0,01 bis 0,15 Gew.-%, von 0,02 bis 0,10 Gew.-%, von 0,02 bis 0,08 Gew.-%, von 0,02 bis 0,06 Gew.-%, oder von 0,03 bis 0,05 Gew.-%. In einigen Ausführungsformen enthält das Glas Läutermittel ausgewählt aus Sb₂O₃, As₂O₃, SO₃, SnO₂, CI und Kombinationen von zwei oder mehr davon, insbesondere in einem Gesamtanteil von mindestens 0,01 Gew.-%, mindestens 0,02 Gew.-%, oder mindestens 0,03 Gew.-%. In einigen Ausführungsformen enthält das Glas Läutermittel ausgewählt aus Sb₂O₃, As₂O₃, SO₃, SnO₂, CI und Kombinationen von zwei oder mehr davon, insbesondere in einem Gesamtanteil von höchstens 2,00 Gew.-%, höchstens 1,50 Gew.-%, höchstens 1,00 Gew.-%, höchstens 0,75 Gew.-%, höchstens 0,50 Gew.-%, höchstens 0,25 Gew.-%, höchstens 0,20 Gew.-%, höchstens 0,15 Gew.-%, höchstens 0,10 Gew.-%, höchstens 0,08 Gew.-%, höchstens 0,06 Gew.-%, oder höchstens 0,05 Gew.-%.

In einigen Ausführungsformen liegt der Anteil von Sb₂O₃ in einem Bereich von 0 bis 2,00 Gew.-%, beispielsweise von 0 bis 1,50 Gew.-%, von 0 bis 1,00 Gew.-%, von 0 bis 0,75 Gew.-%, von 0 bis 0,50 Gew.-%, von 0,01 bis 0,25 Gew.-%, von 0,01 bis 0,20 Gew.-%, von 0,01 bis 0,15 Gew.-%, von 0,02 bis 0,10 Gew.-%, von 0,02 bis 0,08 Gew.-%, von 0,02 bis 0,06 Gew.-%, oder von 0,03 bis 0,05 Gew.-%. In einigen Ausführungsformen beträgt der Anteil von Sb₂O₃ mindestens 0,01 Gew.-%, mindestens 0,02 Gew.-%, oder mindestens 0,03 Gew.-%. In einigen Ausführungsformen beträgt der Anteil von Sb₂O₃ höchstens 2,00 Gew.-%, höchstens 1,50 Gew.-%, höchstens 1,00 Gew.-%, höchstens 0,75 Gew.-%, höchstens 0,50 Gew.-%, höchstens 0,25 Gew.-%, höchstens 0,20 Gew.-%, höchstens 0,15 Gew.-%, höchstens 0,10 Gew.-%, höchstens 0,08 Gew.-%, höchstens 0,06 Gew.-%, höchstens 0,05 Gew.-%, höchstens 0,04 Gew.-%, höchstens 0,03 Gew.-%, höchstens 0,02 Gew.-%, oder höchstens 0,01 Gew.-%. In einigen Ausführungsformen ist das Glas frei von Sb₂O₃.

In einigen Ausführungsformen beträgt der Anteil von As₂O₃ höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei As₂O₃.

In einigen Ausführungsformen beträgt der Anteil von SO₃ höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei SO₃.

In einigen Ausführungsformen beträgt der Gesamtanteil an Oxiden von Sn, insbesondere der Gesamtanteil von SnO₂ und SnO höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Oxiden von Sn, insbesondere frei von SnO₂ und SnO.

In einigen Ausführungsformen beträgt der Anteil von Cl höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei Cl.

In einigen Ausführungsformen beträgt der Anteil von Ag₂O höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Ag₂O.

In einigen Ausführungsformen beträgt der Anteil von mindestens einem von Cr₂O₃, NiO, Fe₂O₃ und Pt höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen beträgt der Anteil von Cr₂O₃, NiO, Fe₂O₃ und Pt jeweils höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen beträgt die Summe der Anteile von Cr₂O₃, NiO, Fe₂O₃ und Pt höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von mindestens einem von Cr₂O₃, NiO, Fe₂O₃ und Pt. In einigen Ausführungsformen ist das Glas frei von Cr₂O₃, NiO, Fe₂O₃ und Pt.

In einigen Ausführungsformen beträgt der Anteil von Bi₂O₃ höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Bi₂O₃.

In einigen Ausführungsformen beträgt der Anteil an Tb₂O₃ höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Tb₂O₃.

In einigen Ausführungsformen beträgt der Anteil an Eu₂O₃ höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Eu₂O₃.

In einigen Ausführungsformen beträgt der Anteil von mindestens einem von F, Cl und I höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen beträgt der Anteil von F, CI und I jeweils höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen beträgt die Summe der Anteile von F, Cl und I höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von mindestens einem von F, Cl und I. In einigen Ausführungsformen ist das Glas frei von F, Cl und I.

In einigen Ausführungsformen beträgt der Anteil an GeO₂ höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von GeO₂.

In einigen Ausführungsformen beträgt der Anteil an P₂O₅ höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von P₂O₅.

In einigen Ausführungsformen beträgt die Summe der Anteile von Al₂O₃, GeO₂, Ga₂O₃ und P₂O₅ höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Al₂O₃, GeO₂, Ga₂O₃ und P₂O₅.

In einigen Ausführungsformen beträgt die Summe der Anteile von GeO₂ und Ga₂O₃ höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von GeO₂ und Ga₂O₃.

In einigen Ausführungsformen beträgt der Anteil an CuO höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von CuO.

In einigen Ausführungsformen beträgt die Summe der Anteile an Oxiden von V, Cr, Mn, Fe, Co, Ni, Cu, Mo, Ru, Ce, Pr und Er höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von Oxiden von V, Cr, Mn, Fe, Co, Ni, Cu, Mo, Ru, Ce, Pr und Er.

In einigen Ausführungsformen beträgt die Summe der Anteile von CeO₂ und Tb₂O₃ höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von CeO₂ und Tb₂O₃.

In einigen Ausführungsformen beträgt der Anteil an CeO₂ höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von CeO₂.

In einigen Ausführungsformen beträgt der Gesamtanteil an Schwefeloxiden, insbesondere der Gesamtanteil an SO₂ und SO₃ höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas schwefelfrei, insbesondere frei ist von SO₂ und SO₃.

In einigen Ausführungsformen beträgt der Anteil von mindestens einem von As₂O₃ und PbO höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen beträgt der Anteil von As₂O₃ und PbO jeweils höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen beträgt die Summe der Anteile von As₂O₃ und PbO höchstens 1,0 Gew.-%, beispielsweise höchstens 0,5 Gew.-%, höchstens 0,2 Gew.-%, höchstens 0,1 Gew.-%, höchstens 0,05 Gew.-%, oder höchstens 0,01 Gew.-%. In einigen Ausführungsformen ist das Glas frei von mindestens einem von As₂O₃ und PbO. In einigen Ausführungsformen ist das Glas frei von As₂O₃ und PbO.

Wenn es in dieser Offenbarung heißt, dass das Glas "frei von" einem Bestandteil ist oder dass es einen bestimmten Bestandteil nicht enthält, dann bedeutet dies, dass dieser Bestandteil allenfalls als Verunreinigung in dem Glas vorhanden sein darf. Das bedeutet, dass er nicht in wesentlichen Mengen zugesetzt ist. Als nicht wesentliche Mengen gelten Mengen von weniger als 100 ppm (Gewichtsanteil), weniger als 75 ppm (Gewichtsanteil), weniger als 50 ppm (Gewichtsanteil), weniger als 25 ppm (Gewichtsanteil), insbesondere weniger als 10 ppm (Gewichtsanteil).

In einigen bevorzugten Ausführungsformen umfasst das Glas die folgenden Komponenten in den angegebenen Anteilen (in Gew.-):

| Komponente | Min (Gew.-%) | Max (Gew.-%) |
|---|---|---|
| SiO₂ | 10 | 55 |
| B₂O₃ | 0 | 25 |
| CaO | 0 | 5,0 |
| BaO | 0 | 50 |
| SrO | 0 | 5,0 |
| ZnO | 0 | 30 |
| La₂O₃ | 0 | 70 |
| Gd₂O₃ | 0 | 15 |
| Y₂O₃ | 0 | 15 |
| ZrO₂ | 0,1 | 10 |
| Ta₂O₅ | 0 | 10 |
| Nb₂O₅ | 0 | 10 |
| Σ R₂O | 0 | 10 |

In weiter bevorzugten Ausführungsformen umfasst das Glas die folgenden Komponenten in den angegebenen Anteilen (in Gew.-):

| **Komponente** | **Min (Gew.-%)** | **Max (Gew.-%)** |
|---|---|---|
| SiO₂ | 15 | 45 |
| B₂O₃ | 0,1 | 20 |
| CaO | 0 | 2,0 |
| BaO | 0,1 | 45 |
| SrO | 0 | 3,0 |
| ZnO | 0,1 | 25 |
| La₂O₃ | 0,1 | 50 |
| Gd₂O₃ | 0 | 10 |
| Y₂O₃ | 0 | 10 |
| ZrO₂ | 0,2 | 9,0 |
| Ta₂O₅ | 0 | 9,0 |
| Nb₂O₅ | 0 | 9,0 |
| Σ R₂O | 0 | 4,0 |

In weiter bevorzugten Ausführungsformen umfasst das Glas die folgenden Komponenten in den angegebenen Anteilen (in Gew.-):

| **Komponente** | **Min (Gew.-%)** | **Max (Gew.-%)** |
|---|---|---|
| SiO₂ | 17 | 40 |
| B₂O₃ | 0,5 | 15 |
| CaO | 0 | 1,0 |
| BaO | 1,0 | 35 |
| SrO | 0 | 2,5 |
| ZnO | 0,5 | 20 |
| La₂O₃ | 2,0 | 45 |
| Gd₂O₃ | 0 | 9,0 |
| Y₂O₃ | 0 | 9,0 |
| ZrO₂ | 0,5 | 8,0 |
| Ta₂O₅ | 0 | 8,0 |
| Nb₂O₅ | 0 | 8,0 |
| Σ R₂O | 0 | 3,0 |

In weiter bevorzugten Ausführungsformen umfasst das Glas die folgenden Komponenten in den angegebenen Anteilen (in Gew.-):

| **Komponente** | **Min (Gew.-%)** | **Max (Gew.-%)** |
|---|---|---|
| SiO₂ | 18 | 35 |
| B₂O₃ | 1,0 | 13 |
| CaO | 0 | 0,5 |
| BaO | 2,0 | 30 |
| SrO | 0 | 2,0 |
| ZnO | 1,0 | 18 |
| La₂O₃ | 5,0 | 40 |
| Gd₂O₃ | 0 | 8,0 |
| Y₂O₃ | 0 | 8,0 |
| ZrO₂ | 1,0 | 7,0 |
| Ta₂O₅ | 0 | 7,0 |
| Nb₂O₅ | 0 | 7,0 |
| Σ R₂O | 0 | 2,0 |

In weiter bevorzugten Ausführungsformen umfasst das Glas die folgenden Komponenten in den angegebenen Anteilen (in Gew.-):

| **Komponente** | **Min (Gew.-%)** | **Max (Gew.-%)** |
|---|---|---|
| SiO₂ | 20 | 35 |
| B₂O₃ | 2,0 | 10 |
| CaO | 0 | 0,2 |
| BaO | 5,0 | 30 |
| SrO | 0 | 1,0 |
| ZnO | 2,0 | 18 |
| La₂O₃ | 10 | 30 |
| Gd₂O₃ | 0,1 | 7,0 |
| Y₂O₃ | 0,1 | 7,0 |
| ZrO₂ | 1,0 | 5,0 |
| Ta₂O₅ | 0,1 | 7,0 |
| Nb₂O₅ | 0,1 | 5,0 |
| Σ R₂O | 0,1 | 1,5 |

In weiter bevorzugten Ausführungsformen umfasst das Glas die folgenden Komponenten in den angegebenen Anteilen (in Gew.-):

| **Komponente** | **Min (Gew.-%)** | **Max (Gew.-%)** |
|---|---|---|
| SiO₂ | 22 | 35 |
| B₂O₃ | 2,0 | 8,0 |
| CaO | 0 | 0,1 |
| BaO | 10 | 30 |
| SrO | 0 | 0,5 |
| ZnO | 5,0 | 18 |
| La₂O₃ | 15 | 25 |
| Gd₂O₃ | 0,2 | 7,0 |
| Y₂O₃ | 0,2 | 5,0 |
| ZrO₂ | 1,5 | 4,5 |
| Ta₂O₅ | 0,2 | 7,0 |
| Nb₂O₅ | 0,1 | 2,0 |
| Σ R₂O | 0,1 | 1,2 |

In weiter bevorzugten Ausführungsformen umfasst das Glas die folgenden Komponenten in den angegebenen Anteilen (in Gew.-):

| **Komponente** | **Min (Gew.-%)** | **Max (Gew.-%)** |
|---|---|---|
| SiO₂ | 22 | 35 |
| B₂O₃ | 2,0 | 8,0 |
| CaO | 0 | 0,1 |
| BaO | 15 | 30 |
| SrO | 0 | 0 |
| ZnO | 2,0 | 18 |
| La₂O₃ | 10 | 30 |
| Gd₂O₃ | 0,5 | 7,0 |
| Y₂O₃ | 1,0 | 10 |
| ZrO₂ | 1,0 | 5,0 |
| Ta₂O₅ | 1,0 | 7,0 |
| Nb₂O₅ | 0 | 0 |
| Σ R₂O | 0 | 2,0 |

In weiter bevorzugten Ausführungsformen umfasst das Glas die folgenden Komponenten in den angegebenen Anteilen (in Gew.-):

| **Komponente** | **Min (Gew.-%)** | **Max (Gew.-%)** |
|---|---|---|
| SiO₂ | 23 | 34 |
| B₂O₃ | 2,0 | 6,0 |
| CaO | 0 | 0,1 |
| BaO | 15 | 28 |
| SrO | 0 | 0,5 |
| ZnO | 10 | 18 |
| La₂O₃ | 15 | 25 |
| Gd₂O₃ | 0,5 | 7,0 |
| Y₂O₃ | 0,5 | 2,5 |
| ZrO₂ | 1,5 | 4,5 |
| Ta₂O₅ | 0,5 | 7,0 |
| Nb₂O₅ | 0,1 | 1,5 |
| Σ R₂O | 0,1 | 1,1 |

In weiter bevorzugten Ausführungsformen umfasst das Glas die folgenden Komponenten in den angegebenen Anteilen (in Gew.-):

| **Komponente** | **Min (Gew.-%)** | **Max (Gew.-%)** |
|---|---|---|
| SiO₂ | 24 | 33 |
| B₂O₃ | 2,0 | 5,0 |
| CaO | 0 | 0,1 |
| BaO | 17 | 27 |
| SrO | 0 | 0,5 |
| ZnO | 11 | 17 |
| La₂O₃ | 15 | 25 |
| Gd₂O₃ | 1,0 | 7,0 |
| Y₂O₃ | 0,5 | 2,0 |
| ZrO₂ | 2,0 | 4,5 |
| Ta₂O₅ | 1,0 | 7,0 |
| Nb₂O₅ | 0,1 | 1,0 |
| Σ R₂O | 0,1 | 1,1 |

In weiter bevorzugten Ausführungsformen umfasst das Glas die folgenden Komponenten in den angegebenen Anteilen (in Gew.-):

| **Komponente** | **Min (Gew.-%)** | **Max (Gew.-%)** |
|---|---|---|
| SiO₂ | 24 | 32 |
| B₂O₃ | 2,0 | 5,0 |
| CaO | 0 | 0,1 |
| BaO | 17 | 26 |
| SrO | 0 | 0 |
| ZnO | 5,0 | 15 |
| La₂O₃ | 17 | 28 |
| Gd₂O₃ | 1,5 | 6,0 |
| Y₂O₃ | 2,0 | 8,0 |
| ZrO₂ | 2,0 | 4,5 |
| Ta₂O₅ | 2,0 | 7,0 |
| Nb₂O₅ | 0 | 0 |
| Σ R₂O | 0 | 2,0 |

In weiter bevorzugten Ausführungsformen umfasst das Glas die folgenden Komponenten in den angegebenen Anteilen (in Gew.-):

| **Komponente** | **Min (Gew.-%)** | **Max (Gew.-%)** |
|---|---|---|
| SiO₂ | 25 | 32 |
| B₂O₃ | 2,5 | 4,5 |
| CaO | 0 | 0,1 |
| BaO | 17 | 26 |
| SrO | 0 | 0,5 |
| ZnO | 11 | 16 |
| La₂O₃ | 15 | 24 |
| Gd₂O₃ | 1,5 | 6,0 |
| Y₂O₃ | 0,5 | 1,5 |
| ZrO₂ | 2,0 | 4,5 |
| Ta₂O₅ | 2,0 | 7,0 |
| Nb₂O₅ | 0,1 | 0,5 |
| Σ R₂O | 0,2 | 1,1 |

In weiter bevorzugten Ausführungsformen umfasst das Glas die folgenden Komponenten in den angegebenen Anteilen (in Gew.-):

| **Komponente** | **Min (Gew.-%)** | **Max (Gew.-%)** |
|---|---|---|
| SiO₂ | 27 | 31 |
| B₂O₃ | 3,0 | 4,0 |
| CaO | 0 | 0 |
| BaO | 17 | 21 |
| SrO | 0 | 0 |
| ZnO | 7,0 | 14 |
| La₂O₃ | 19 | 26 |
| Gd₂O₃ | 3,0 | 5,5 |
| Y₂O₃ | 2,5 | 6,0 |
| ZrO₂ | 2,0 | 3,5 |
| Ta₂O₅ | 2,0 | 5,0 |
| Nb₂O₅ | 0 | 0 |
| Σ R₂O | 0 | 2,0 |

### Optische Eigenschaften

In einigen Ausführungsformen weist das Glas bei einer Probendicke von 25 mm und einer Wellenlänge von 380 nm eine Reintransmission von mindestens 0,900 auf, beispielsweise eine Reintransmission von mindestens 0,910, mindestens 0,920, mindestens 0,930, mindestens 0,940, mindestens 0,950, mindestens 0,960, mindestens 0,965, mindestens 0,970, mindestens 0,975, oder mindestens 0,980. In einigen Ausführungsformen weist das Glas bei einer Probendicke von 25 mm und einer Wellenlänge von 380 nm eine Reintransmission von höchstens 0,999, höchstens 0,998 höchstens 0,995, höchstens 0,990 oder höchstens 0,985 auf. In einigen Ausführungsformen weist das Glas bei einer Probendicke von 25 mm und einer Wellenlänge von 380 nm eine Reintransmission in einem Bereich von 0,900 bis 0,999 auf, beispielsweise in einem Bereich von 0,910 bis 0,999, von 0,920 bis 0,998, von 0,930 bis 0,998, von 0,940 bis 0,995, von 0,950 bis 0,995, von 0,960 bis 0,995, von 0,965 bis 0,990, von 0,970 bis 0,990, von 0,975 bis 0,985, oder von 0,980 bis 0,985.

In einigen Ausführungsformen weist das Glas bei einer Probendicke von 25 mm im gesamten Wellenlängenbereich von 380 nm bis 700 nm eine Reintransmission von mindestens 0,900 auf, beispielsweise eine Reintransmission von mindestens 0,910, mindestens 0,920, mindestens 0,930, mindestens 0,940, mindestens 0,950, mindestens 0,960, mindestens 0,965, mindestens 0,970, mindestens 0,975, oder mindestens 0,980. In einigen Ausführungsformen weist das Glas bei einer Probendicke von 25 mm im gesamten Wellenlängenbereich von 380 nm bis 700 nm eine Reintransmission von höchstens 0,999, höchstens 0,998 höchstens 0,995, höchstens 0,990 oder höchstens 0,985 auf. In einigen Ausführungsformen weist das Glas bei einer Probendicke von 25 mm im gesamten Wellenlängenbereich von 380 nm bis 700 nm eine Reintransmission in einem Bereich von 0,900 bis 0,999 auf, beispielsweise in einem Bereich von 0,910 bis 0,999, von 0,920 bis 0,998, von 0,930 bis 0,998, von 0,940 bis 0,995, von 0,950 bis 0,995, von 0,960 bis 0,995, von 0,965 bis 0,990, von 0,970 bis 0,990, von 0,975 bis 0,985, oder von 0,980 bis 0,985.

In einigen Ausführungsformen weist das Glas bei einer Wellenlänge von 380 nm eine Dämpfung in einem Bereich von 0,5 bis <10 dB/m auf, beispielsweise von 1,0 bis <5,0 dB/m, von 1,5 bis 4,0 dB/m, oder von 2,0 bis 3,5 dB/m. In einigen Ausführungsformen weist das Glas bei einer Wellenlänge von 380 nm eine Dämpfung von mindestens 0,5 dB/m auf, beispielsweise mindestens 1,0 dB/m, von mindestens 1,5 dB/m, oder von mindestens 2,0 dB/m. In einigen Ausführungsformen weist das Glas bei einer Wellenlänge von 380 nm eine Dämpfung von weniger als 10 dB/m auf, beispielsweise weniger als 5,0 dB/m, höchstens 4,0 dB/m, oder höchstens 3,5 dB/m.

In einigen Ausführungsformen beträgt bei einer Probendicke von jeweils 25 mm das Verhältnis der Reintransmission bei einer Wellenlänge von 380 nm und der Reintransmission bei einer Wellenlänge von 600 nm mindestens 0,900, beispielsweise mindestens 0,910, mindestens 0,920, mindestens 0,930, mindestens 0,940, mindestens 0,950, mindestens 0,960, mindestens 0,965, mindestens 0,970, mindestens 0,975, oder mindestens 0,980. In einigen Ausführungsformen beträgt bei einer Probendicke von jeweils 25 mm das Verhältnis der Reintransmission bei einer Wellenlänge von 380 nm und der Reintransmission bei einer Wellenlänge von 600 nm höchstens 0,999, höchstens 0,998 höchstens 0,995, höchstens 0,990 oder höchstens 0,985. In einigen Ausführungsformen liegt bei einer Probendicke von jeweils 25 mm das Verhältnis der Reintransmission bei einer Wellenlänge von 380 nm und der Reintransmission bei einer Wellenlänge von 600 nm in einem Bereich von 0,900 bis 0,999, beispielsweise in einem Bereich von 0,910 bis 0,999, von 0,920 bis 0,998, von 0,930 bis 0,998, von 0,940 bis 0,995, von 0,950 bis 0,995, von 0,960 bis 0,995, von 0,965 bis 0,990, von 0,970 bis 0,990, von 0,975 bis 0,985, oder von 0,980 bis 0,985.

In einigen Ausführungsformen liegt der Brechungsindex n_{d} in einem Bereich von 1,60 bis 1,85, beispielsweise in einem Bereich von 1,65 bis 1,80, von 1,67 bis 1,77, von 1,68 bis 1,75, von 1,69 bis 1,74, oder 1,70 bis 1,73. In einigen Ausführungsformen beträgt der Brechungsindex n_{d} mindestens 1,60, beispielsweise mindestens 1,65, mindestens 1,67, mindestens 1,68, mindestens 1,69, oder mindestens 1,70. In einigen Ausführungsformen beträgt der Brechungsindex nd höchstens 1,85, beispielsweise höchstens 1,80, höchstens 1,77, höchstens 1,75, höchstens 1,74, oder höchstens 1,73.

In einigen Ausführungsformen liegt die Abbe-Zahl v_{d} in einem Bereich von 35 bis 60, beispielweise von 40 bis 55, von 42 bis 54, von 43 bis 53, von 44 bis 52, oder von 45 bis 51. In einigen Ausführungsformen beträgt die Abbe-Zahl vd mindestens 35, beispielsweise mindestens 40, mindestens 42, mindestens 43, mindestens 44, oder mindestens 45. In einigen Ausführungsformen beträgt die Abbe-Zahl vd höchstens 60, beispielsweise höchstens 55, höchstens 54, höchstens 53, höchstens 52, oder höchstens 51.

### Kristallisationsbeständigkeit

Die Gläser der Erfindung weisen nicht nur hervorragende optische Eigenschaften auf. Die Gläser der Erfindung zeichnen sich auch dadurch aus, dass sie eine besonders hohe Kristallisationsbeständigkeit aufweisen. Dies ist von großer Bedeutung für die Herstellung im Faserzug. Andernfalls können Entglasungskristalle entstehen, insbesondere an der Glasoberfläche, wodurch beispielsweise die Formgebung des Glases beeinträchtigen wird. Daher weisen die Gläser eine gute Entglasungsstabilität auf. Dies ist besonders wichtig für große Abmessungen und dicke Wandungen.

Ein Maß für die Kristallisationsbeständigkeit ist die maximale Kristallisationsgeschwindigkeit KGₘₐₓ. Je geringer KGₘₐₓ ist, desto größer ist die Kristallisationsbeständigkeit. In der vorliegenden Offenbarung werden die Begriffe "Kristallisationsbeständigkeit" und "Entglasungsstabilität" synonym verwendet. KGₘₐₓ beschreibt die maximale Kristallisationsgeschwindigkeit (in der Regel in µm/min). Die Messung der Kristallisationsgeschwindigkeit ist bekannt. Bevorzugt wird die Kristallisationsgeschwindigkeit entlang gebildeter Kristalle gemessen, d.h. entlang ihrer größten Ausdehnung.

Unter "UEG" wird erfindungsgemäß die sogenannte untere Entglasungsgrenze verstanden. Dies ist diejenige Temperatur, bei welcher, unter aufsteigender Temperaturführung, die Entglasung des Materials beginnt. Oberhalb einer bestimmten Temperatur, die als obere Entglasungsgrenze (OEG) oder als Liquidustemperatur bezeichnet wird, entstehen auch nach längerer Zeit keine Kristalle. Die Zahlenwerte von UEG und OEG unterscheiden sich in der Regel bei verschiedenen Gläsern.

Wenn es zu Kristallisation kommt, tritt diese bei Temperaturen oberhalb der unteren Entglasungsgrenze (UEG) und unterhalb der oberen Entglasungsgrenze (OEG) auf, also in einem Bereich zwischen UEG und OEG. Die Temperatur, bei der die maximale Kristallisationsgeschwindigkeit erreicht wird, liegt folglich ebenfalls zwischen UEG und OEG. Um die maximale Kristallisationsgeschwindigkeit KGₘₐₓ zu bestimmen, muss das Glas somit auf eine Temperatur zwischen UEG und OEG erwärmt werden. Da für ein gegebenes Glas in der Regel nicht bekannt ist, wo genau im Bereich zwischen UEG und OEG die maximale Kristallisation auftritt, werden häufig verschiedene Temperaturen innerhalb des Bereichs getestet, um KGₘₐₓ zu bestimmen. Auf diese Weise können auch UEG und OEG selbst als die Unter- bzw. Obergrenze des Bereichs bestimmt werden, in dem Kristallisation auftritt.

Wenn in der vorliegenden Offenbarung auf die untere Entglasungsgrenze UEG Bezug genommen wird, so ist damit die UEG gemeint, die ermittelt wird, indem das Glas für eine Haltezeit von 5 Minuten in einem Gradientenofen mit aufsteigender Temperaturführung thermisch behandelt wird, sofern nichts anderes angegeben ist.

Insbesondere wird die Kristallisationsgeschwindigkeit bestimmt, indem das Glas für eine Haltezeit von 5 Minuten oder einer Stunde in einem Gradientenofen mit aufsteigender Temperaturführung thermisch behandelt wird. Ein Gradientenofen ist ein Ofen mit unterschiedlichen Heizzonen, also ein Ofen mit unterschiedliche Temperaturbereichen. Aufsteigende Temperaturführung bedeutet, dass die Temperatur des Glases, bevor es in den Gradientenofen eingeführt wird, geringer ist, als die Temperaturen in sämtlichen Bereichen des Ofens. Durch das Einführen in den Ofen steigt also die Temperatur des Glases an, unabhängig davon, in welchen Bereich des Ofens das Glas eingeführt wird. Die Entglasungsmessung wird also insbesondere durch eine fünfminütige oder einstündige thermische Behandlung in einem (bereits heißen) Gradientenofen durchgeführt, der in verschiedene Temperaturzonen unterteilt ist. Es handelt sich um einen ortsaufgelösten Temperaturgradienten im Gradientenofen und nicht um einen zeitlich aufgelösten Gradienten, da der Gradientenofen ortsaufgelöst in verschiedene Temperaturzonen eingeteilt ist.

Dadurch, dass der Gradientenofen in mehrere Heizzonen aufgeteilt ist, können verschiedene Temperaturen gleichzeitig getestet werden. Dies ist ein besonderer Vorteil eines Gradientenofens. Beispielsweise kann die unterste Temperatur bei 950°C liegen und die oberste Temperatur bei 1250°C, oder die unterste Temperatur kann bei 700°C liegen und die oberste Temperatur bei 1000 °C. Die Temperaturen sollten so gewählt werden, dass die Kristallisationsgeschwindigkeit, bei verschiedenen Temperaturen im Bereich zwischen UEG und OEG bestimmt werden kann, so dass aus einem Vergleich der potentiell verschiedenen Kristallisationsgeschwindigkeiten innerhalb des Bereichs zwischen UEG und OEG die größte der Kristallisationsgeschwindigkeiten als maximale Kristallisationsgeschwindigkeit KGₘₐₓ bestimmt werden kann. Wenn UEG und OEG unbekannt sind, werden vorteilhafterweise Temperaturen in einem relativ großen Bereich getestet, um eine Bestimmung von UEG und OEG zu ermöglichen.

Dass ein Glas beispielsweise eine maximalen Kristallisationsgeschwindigkeit (KGₘₐₓ) von höchstens 15 µm/min in einem Temperaturbereich von 700°C bis 1250°C aufweist, wenn das Glas für eine Haltezeit von einer Stunde in einem Gradientenofen mit aufsteigender Temperaturführung thermisch behandelt wird, bedeutet also nicht, dass in dem Gradientenofen Temperaturen im gesamten Bereich von 700°C bis 1250°C vorliegen müssen. Wenn für ein bestimmtes Glas beispielsweise bekannt ist, dass OEG 1000°C beträgt, müssen im Gradientenofen keine Temperaturen von mehr als 1000°C getestet werden, da bei diesen Temperaturen dann ohnehin keine Kristallisation auftritt, so dass die maximale Kristallisationsgeschwindigkeit KGₘₐₓ unterhalb von 1000°C vorliegen muss. Ebenso gilt beispielsweise, dass im Gradientenofen keine Temperaturen von weniger als 950°C getestet werden müssen, wenn für ein Glas bekannt ist, dass UEG 950°C beträgt, da bei diesen Temperaturen dann ohnehin keine Kristallisation auftritt, so dass die maximale Kristallisationsgeschwindigkeit KGₘₐₓ oberhalb von 950°C vorliegen muss.

Wenn keine Entglasung auftritt, gibt es keine Kristallisation, so dass KGₘₐₓ nicht bestimmt werden kann. In diesem Fall kann für KGₘₐₓ ein Wert von 0 µm/min angenommen werden.

Die Kristallisationsgeschwindigkeit wird bevorzugt unter Verwendung von Glasgries bestimmt, insbesondere von Glasgries mit einem Durchmesser von 1,6 mm bis 4 mm. Glasgries wird für die thermische Behandlung im Gradientenofen bevorzugt auf einem Träger platziert, beispielsweise einem Platinträger. Der Träger kann Vertiefungen aufweisen, insbesondere jeweils eine zur Aufnahme eines Glaskorns, und ein Loch an der Unterseite jeder Vertiefung, so dass die Kristallisationsgeschwindigkeit im Anschluss an die thermische Behandlung mikroskopisch bestimmt werden kann. Im Hinblick auf die bevorzugte Größe der Glaskörner haben die Vertiefungen bevorzugt jeweils einen Durchmesser von 4 mm und die Löcher jeweils einen Durchmesser von 1 mm. Im Anschluss an die thermische Behandlung kann mikroskopisch bestimmt werden, in welchem Temperaturbereich welche Kristallisationsgeschwindigkeit vorlag. Die größte Kristallisationsgeschwindigkeit, die festgestellt wird, ist die maximale Kristallisationsgeschwindigkeit KGₘₐₓ. UEG und OEG können als Unter- bzw. Obergrenze des Temperaturbereichs bestimmt werden, in dem Kristallisation auftrat. Die Zuordnung der einzelnen Glaskörner zu den verschiedenen Temperaturzonen im Gradientenofen ist unproblematisch, da bekannt ist, an welcher Position im Ofen, welche Temperatur vorliegt, und an welcher Position im Ofen, sich welches Glaskorn befunden hat.

Die Gläser der Erfindung weisen eine so hohe Entglasungsstabilität auf, dass die maximale Kristallisationsgeschwindigkeit (KGₘₐₓ) in einigen Ausführungsformen höchstens 7,5 µm/min in einem Temperaturbereich von 700°C bis 1250°C (insbesondere 800°C bis 1200°C, 850°C bis 1150°C, 900°C bis 1100°C, oder 950°C bis 1050°C) beträgt, wenn das Glas für eine Haltezeit von einer Stunde in einem Gradientenofen mit aufsteigender Temperaturführung thermisch behandelt wird. In einigen Ausführungsformen beträgt KGₘₐₓ höchstens 6,0 µm/min in einem Temperaturbereich von 700°C bis 1250°C (insbesondere 800°C bis 1200°C, 850°C bis 1150°C, 900°C bis 1100°C, oder 950°C bis 1050°C), beispielsweise höchstens 5,0 µm/min höchstens 4,0 µm/min, höchstens 3,0 µm/min, höchstens 2,5 µm/min, höchstens 2,0 µm/min, höchstens 1,5 µm/min, höchstens 1,0 µm/min, höchstens 0,5 µm/min, höchsten 0,2 µm/min, höchstens 0,1 µm/min, oder sogar 0 µm/min, wenn das Glas für eine Haltezeit von einer Stunde in einem Gradientenofen mit aufsteigender Temperaturführung thermisch behandelt wird. In einigen Ausführungsformen beträgt KGₘₐₓ mindestens 0,1 µm/min in einem Temperaturbereich von 700°C bis 1250°C (insbesondere 800°C bis 1200°C, 850°C bis 1150°C, 900°C bis 1100°C, oder 950°C bis 1050°C), beispielsweise mindestens 0,2 µm/min, mindestens 0,5 µm/min, mindestens 1,0 µm/min, mindestens 1,5 µm/min, mindestens 2,0 µm/min, mindestens 2,5 µm/min, oder mindestens 3,0 µm/min, wenn das Glas für eine Haltezeit von einer Stunde in einem Gradientenofen mit aufsteigender Temperaturführung thermisch behandelt wird. In einigen Ausführungsformen liegt KGₘₐₓ in einem Temperaturbereich von 700°C bis 1250°C (insbesondere 800°C bis 1200°C, 850°C bis 1150°C, 900°C bis 1100°C, oder 950°C bis 1050°C) in einem Bereich von 0 bis 7,5 µm/min, beispielsweise in einem Bereich von 0 bis 6,0 µm/min, von 0,1 bis 5,0 µm, von 0,2 bis 4,0 µm/min, von 0,5 bis 3,0 µm/min, von 1,0 bis 2,5 µm/min, von 1,5 bis 2,0 µm/min, von 1,0 bis 1,5 µm/min, von 0,5 bis 1,0 µm/min, von 0,2 bis 0,5 µm/min, von 0,1 bis 0,2 µm/min, oder von 0 bis 0,1 µm/min, wenn das Glas für eine Haltezeit von einer Stunde in einem Gradientenofen mit aufsteigender Temperaturführung thermisch behandelt wird.

In einigen Ausführungsformen liegt OEG in einem Bereich von 900°C bis 1400°C, beispielsweise von 950°C bis 1350°C, von 1000°C bis 1300°C, von 1050°C bis 1250°C, oder von 1100°C bis 1200°C, wenn das Glas für eine Haltezeit von einer Stunde in einem Gradientenofen mit aufsteigender Temperaturführung thermisch behandelt wird. In einigen Ausführungsformen beträgt OEG mindestens 900°C, beispielsweise mindestens 950°C, mindestens 1000°C, mindestens 1050°C, oder mindestens 1100°C, wenn das Glas für eine Haltezeit von einer Stunde in einem Gradientenofen mit aufsteigender Temperaturführung thermisch behandelt wird. In einigen Ausführungsformen beträgt OEG höchstens 1400°C, beispielsweise höchstens 1350°C, höchstens 1300°C, höchstens 1250°C, oder höchstens 1200°C, wenn das Glas für eine Haltezeit von einer Stunde in einem Gradientenofen mit aufsteigender Temperaturführung thermisch behandelt wird.

Die Gläser der Erfindung weisen eine so hohe Entglasungsstabilität auf, dass die maximale Kristallisationsgeschwindigkeit (KGₘₐₓ) in einigen Ausführungsformen höchstens 25 µm/min in einem Temperaturbereich von 700°C bis 1250°C (insbesondere 850°C bis 1200°C, 900°C bis 1150°C, 950°C bis 1100°C, oder 1000°C bis 1050°C) beträgt, wenn das Glas für eine Haltezeit von 5 Minuten in einem Gradientenofen mit aufsteigender Temperaturführung thermisch behandelt wird. In einigen Ausführungsformen beträgt KGₘₐₓ höchstens 20 µm/min in einem Temperaturbereich von 700°C bis 1250°C (insbesondere 850°C bis 1200°C, 900°C bis 1150°C, 950°C bis 1100°C, oder 1000°C bis 1050°C), beispielsweise höchstens 15 µm/min höchstens 12 µm/min, höchstens 10 µm/min, höchstens 7,5 µm/min, höchstens 5,0 µm/min, höchstens 4,0 µm/min, höchstens 3,0 µm/min, höchstens 2,0 µm/min, höchstens 1,0 µm/min, höchstens 0,5 µm/min, höchsten 0,2 µm/min, höchstens 0,1 µm/min, oder sogar 0 µm/min, wenn das Glas für eine Haltezeit von 5 Minuten in einem Gradientenofen mit aufsteigender Temperaturführung thermisch behandelt wird. In einigen Ausführungsformen beträgt KGₘₐₓ mindestens 0,1 µm/min in einem Temperaturbereich von 700°C bis 1250°C (insbesondere 850°C bis 1200°C, 900°C bis 1150°C, 950°C bis 1100°C, oder 1000°C bis 1050°C), beispielsweise mindestens 0,2 µm/min, mindestens 0,5 µm/min, mindestens 1,0 µm/min, mindestens 2,0 µm/min, mindestens 3,0 µm/min, mindestens 5,0 µm/min, oder mindestens 7,5 µm/min, wenn das Glas für eine Haltezeit von 5 Minuten in einem Gradientenofen mit aufsteigender Temperaturführung thermisch behandelt wird. In einigen Ausführungsformen liegt KGₘₐₓ in einem Temperaturbereich von 700°C bis 1250°C (insbesondere 850°C bis 1200°C, 900°C bis 1150°C, 950°C bis 1100°C, oder 1000°C bis 1050°C) in einem Bereich von 0 bis 25 µm/min, beispielsweise in einem Bereich von 0,1 bis 20 µm/min, von 0,5 bis 15 µm, von 1,0 bis 12 µm/min, von 2,0 bis 10 µm/min, von 3,0 bis 7,5 µm/min, von 2,0 bis 5,0 µm/min, von 1,0 bis 4,0 µm/min, von 0,5 bis 3,0 µm/min, von 0,2 bis 2,0 µm/min, von 0,1 bis 1,0 µm/min, oder von 0 bis 0,1 µm/min, wenn das Glas für eine Haltezeit von 5 Minuten in einem Gradientenofen mit aufsteigender Temperaturführung thermisch behandelt wird.

In einigen Ausführungsformen liegt UEG in einem Bereich von 650°C bis 1100°C, beispielsweise von 700°C bis 1050°C, von 750°C bis 1000°C, von 800°C bis 950°C, oder von 850°C bis 900°C, wenn das Glas für eine Haltezeit von 5 Minuten in einem Gradientenofen mit aufsteigender Temperaturführung thermisch behandelt wird. In einigen Ausführungsformen beträgt UEG mindestens 650°C, beispielsweise mindestens 700°C, mindestens 750°C, mindestens 800°C, oder mindestens 850°C, wenn das Glas für eine Haltezeit von 5 Minuten in einem Gradientenofen mit aufsteigender Temperaturführung thermisch behandelt wird. In einigen Ausführungsformen beträgt UEG höchstens 1100°C, beispielsweise höchstens 1050°C, höchstens 1000°C, höchstens 950°C, oder höchstens 900°C, wenn das Glas für eine Haltezeit von 5 Minuten in einem Gradientenofen mit aufsteigender Temperaturführung thermisch behandelt wird.

In einigen Ausführungsformen liegt OEG in einem Bereich von 850°C bis 1350°C, beispielsweise von 900°C bis 1300°C, von 950°C bis 1250°C, von 1000°C bis 1200°C, oder von 1050°C bis 1150°C, wenn das Glas für eine Haltezeit von 5 Minuten in einem Gradientenofen mit aufsteigender Temperaturführung thermisch behandelt wird. In einigen Ausführungsformen beträgt OEG mindestens 850°C, beispielsweise mindestens 900°C, mindestens 950°C, mindestens 1000°C, oder mindestens 1050°C, wenn das Glas für eine Haltezeit von 5 Minuten in einem Gradientenofen mit aufsteigender Temperaturführung thermisch behandelt wird. In einigen Ausführungsformen beträgt OEG höchstens 1350°C, beispielsweise höchstens 1300°C, höchstens 1250°C, höchstens 1200°C, oder höchstens 1150°C, wenn das Glas für eine Haltezeit von 5 Minuten in einem Gradientenofen mit aufsteigender Temperaturführung thermisch behandelt wird.

In einigen Ausführungsformen liegt die Differenz von OEG und UEG in einem Bereich von 100 bis 300 K, beispielsweise in einem Bereich von 125 bis 275 K, von 150 bis 250 K, oder von 175 bis 225 K, wenn das Glas für eine Haltezeit von 5 Minuten in einem Gradientenofen mit aufsteigender Temperaturführung thermisch behandelt wird. In einigen Ausführungsformen beträgt die Differenz von OEG und UEG mindestens 100 K, beispielsweise mindestens 125 K, mindestens 150 K, oder mindestens 175 K, wenn das Glas für eine Haltezeit von 5 Minuten in einem Gradientenofen mit aufsteigender Temperaturführung thermisch behandelt wird. In einigen Ausführungsformen beträgt die Differenz von OEG und UEG höchstens 300 K, beispielsweise höchstens 275 K, höchstens 250 K, oder höchstens 225 K, wenn das Glas für eine Haltezeit von 5 Minuten in einem Gradientenofen mit aufsteigender Temperaturführung thermisch behandelt wird.

Aufgrund seiner hervorragenden Entglasungsstabilität kann das Glas der Erfindung in jedem bekannten Glasformgebungsverfahren hergestellt werden, insbesondere in Rohrzugverfahren, beispielsweise im Danner-Verfahren, im Vello-Verfahren oder im A-Zug-Verfahren (Vertikalzug-Verfahren).

### Weitere Eigenschaften

Der obere Kühlpunkt T13 (englisch: "annealing point") ist die Temperatur, bei der die Viskosität 10¹³ dPas beträgt. In einigen Ausführungsformen liegt der obere Kühlpunkt T13 in einem Bereich von 550°C bis 750°C, beispielsweise von 575°C bis 725°C, von 600°C bis 700°C, oder von 625°C bis 675°C. In einigen Ausführungsformen beträgt der obere Kühlpunkt T13 mindestens 550°C, beispielsweise mindestens 575°C, mindestens 600°C, oder mindestens 625°C. In einigen Ausführungsformen beträgt der obere Kühlpunkt T13 höchstens 750°C, beispielsweise höchstens 725°C, höchstens 700°C, oder höchstens 675°C.

Der Erweichungspunkt T7,6 (englisch: "softening point") ist die Temperatur, bei der die Viskosität 10^{7.6} dPas beträgt. In einigen Ausführungsformen liegt der Erweichungspunkt T7,6 in einem Bereich von 700°C bis 900°C, beispielsweise von 725°C bis 875°C, von 750°C bis 850°C, oder von 775°C bis 825°C. In einigen Ausführungsformen beträgt der Erweichungspunkt T7,6 mindestens 700°C, beispielsweise mindestens 725°C, mindestens 750°C, oder mindestens 775°C. In einigen Ausführungsformen beträgt der Erweichungspunkt T7,6 höchstens 900°C, beispielsweise höchstens 875°C, höchstens 850°C, oder höchstens 825°C.

In einigen Ausführungsformen liegt die Differenz zwischen der unteren Entglasungsgrenze UEG und dem Erweichungspunkt T7,6 in einem Bereich von 25 bis 175 K, beispielsweise von 40 bis 160 K, von 50 bis 150 K, von 60 bis 140 K, von 70 bis 130 K, oder von 75 bis 125 K. In einigen Ausführungsformen beträgt die Differenz zwischen der unteren Entglasungsgrenze UEG und dem Erweichungspunkt T7,6 mindestens 25 K, beispielsweise mindestens 40 K, mindestens 50 K, mindestens 60 K, mindestens 70 K, oder mindestens 75 K. In einigen Ausführungsformen beträgt die Differenz zwischen der unteren Entglasungsgrenze UEG und dem Erweichungspunkt T7,6 höchstens 175 K, beispielsweise höchstens 160 K, höchstens 150 K, höchstens 140 K, höchstens 130 K, oder höchstens 125 K.

Der Verarbeitungspunkt T4 (englisch: "working point") ist die Temperatur, bei der die Viskosität 10⁴ dPas beträgt. In einigen Ausführungsformen liegt der Verarbeitungspunkt T4 in einem Bereich von 800°C bis 1100°C, beispielsweise von 850°C bis 1050°C, von 875°C bis 1025°C, oder von 900 bis 1000°C. In einigen Ausführungsformen beträgt der Verarbeitungspunkt T4 mindestens 800°C, mindestens 850°C, mindestens 875°C, oder mindestens 900°C. In einigen Ausführungsformen beträgt der Verarbeitungspunkt T4 höchstens 1100°C, beispielsweise höchstens 1050°C, höchstens 1025°C, oder höchstens 1000°C.

In einigen Ausführungsformen liegt die Glasübergangstemperatur Tg in einem Bereich von 525°C bis 725°C, beispielsweise von 550°C bis 700°C, von 575°C bis 675°C, oder von 600°C bis 650°C. In einigen Ausführungsformen beträgt die Glasübergangstemperatur Tg mindestens 525°C, beispielsweise mindestens 550°C, mindestens 575°C, oder mindestens 600°C. In einigen Ausführungsformen beträgt die Glasübergangstemperatur Tg höchstens 725°C, beispielsweise höchstens 700°C, höchstens 675°C, oder höchstens 650°C.

Wenn in der vorliegenden Offenbarung auf den mittleren thermischen Längenausdehnungskoeffizienten (englisch: "Coefficient of Thermal Expansion", CTE) Bezug genommen wird, so ist damit der mittlere thermische Längenausdehnungskoeffizient in einem Temperaturbereich von 20°C bis 300°C gemeint, sofern nichts anderes angegeben ist.

In einigen Ausführungsformen liegt der mittlere thermische Längenausdehnungskoeffizient in einem Bereich von 5,0 bis 9,0 ppm/K, beispielsweise von 5,5 bis 8,5 ppm/K, von 6,0 bis 8,0 ppm/K, zwischen 6,0 und 8,0 ppm/K, oder von 6,5 bis 7,5 ppm/K. In einigen Ausführungsformen beträgt der mittlere thermische Längenausdehnungskoeffizient mindestens 5,0 ppm/K, beispielsweise mindestens 5,5 ppm/K, mindestens 6,0 ppm/K, mehr als 6,0 ppm/K, oder mindestens 6,5 ppm/K. In einigen Ausführungsformen beträgt der mittlere thermische Längenausdehnungskoeffizient höchstens 9,0 ppm/K, beispielsweise höchstens 8,5 ppm/K, höchstens 8,0 ppm/K, weniger als 8,0 ppm/K, oder höchstens 7,5 ppm/K.

In einigen Ausführungsformen liegt die Dichte des Glases in einem Bereich von 4,00 bis 4,60 g/cm³, beispielsweise von 4,05 bis 4,55 g/cm³, von 4,10 bis 4,50 g/cm³, von 4,15 bis 4,45 g/cm³, von 4,20 bis 4,40 g/cm³, oder von 4,25 bis 4,35 g/cm³. In einigen Ausführungsformen beträgt die Dichte des Glases mindestens 4,00 g/cm³, beispielsweise mindestens 4,05 g/cm³, mindestens 4,10 g/cm³, mindestens 4,15 g/cm³, mindestens 4,20 g/cm³, oder mindestens 4,25 g/cm³. In einigen Ausführungsformen beträgt die Dichte des Glases höchstens 4,60 g/cm³, höchstens 4,55 g/cm³, höchstens 4,50 g/cm³, höchstens 4,45 g/cm³, höchstens 4,40 g/cm³, oder höchstens 4,35 g/cm³.

### Glasartikel und/oder Lichtleitelement

Die Erfindung betrifft auch einen Glasartikel, insbesondere ein Lichtleitelement, beispielsweise eine Glasfaser und/oder einen Lichtleitstab, umfassend oder bestehend aus einem Glas der Erfindung. Das Lichtleitelement kann insbesondere eine Glasfaser mit einem Kernglas und einem Mantelglas sein. Das erfindungsgemäße Glas kommt insbesondere als Kernglas zum Einsatz.

In einigen Ausführungsformen umfasst das Lichtleitelement mindestens ein Kernglas und mindestens ein Mantelglas, beispielsweise genau ein Kernglas und mindestens ein Mantelglas, mindestens ein Kernglas und genau ein Mantelglas, oder genau ein Kernglas und genau ein Mantelglas. In einigen Ausführungsformen besteht das Lichtleitelement aus mindestens einem Kernglas und mindestens einem Mantelglas, beispielsweise aus genau einem Kernglas und mindestens einem Mantelglas, aus mindestens einem Kernglas und genau einem Mantelglas, oder aus genau einem Kernglas und genau einem Mantelglas. Das Kernglas umfasst oder besteht insbesondere aus dem Glas der vorliegenden Erfindung.

In einigen Ausführungsformen umfasst das Lichtleitelement das erfindungsgemäße Glas als Kernglas und außerdem ein Mantelglas, das das Kernglas ummantelt.

In einigen Ausführungsformen ist das Lichtleitelement ein Licht- und/oder Bildleiter, der das erfindungsgemäße Glas als Kernglas umfasst, welches mit einem Mantelglas ummantelt ist. Bei dem Lichtleiter handelt es sich beispielsweise um eine Stufenindexfaser.

Das Lichtleitelement kann entweder flexibel oder starr sein. Ob ein flexibles oder starres Lichtleitelement vorliegt, hängt hauptsächlich von dem Durchmesser des Lichtleitelements ab. Ebenfalls kann das Lichtleitelement in Form einer Glasfaser in einem Faserbündel umfassend eine Vielzahl von Glasfasern vorkommen. Solche Glasfasern und/oder Faserbündel sind üblicherweise flexibel, der Durchmesser der einzelnen Glasfasern beträgt üblicherweise einige dutzend Micrometer bis wenige hundert Micrometer. Glasfasern bestehen in der Regel aus Kern-Mantel-Systemen mit einem Kernglas und einem das Kernglas an seiner Außenumfangsfläche umgebenden Mantelglas. Die Lichtleitung erfolgt durch Totalreflektion an der Grenzfläche zwischen Kern und Mantel. Um die Totalreflektion erreichen zu können, weist das Mantelglas üblicherweise einen niedrigeren Brechungsindex auf als das Kernglas.

Lichtleitstäbe sind in der Regel starr, da sie in der Regel über einen größeren Durchmesser von etwas unter 1 Millimeter bis einige Zentimeter verfügen. Sie können als Kern-Mantel-System wie zuvor beschrieben ausgeführt sein, aber auch als Glasstab ohne Mantelglas, wobei die Totalreflektion an der Grenzfläche der Außenumfangsfläche zum umgebenden Medium, d.h. im Allgemeinen Luft erfolgt. Eine Spezialform des Lichtleitstabs, der ebenfalls von dem Begriff und/oder der Erfindung umfass ist, ist der Faserstab, bei dem eine Vielzahl von Glaselementen aus Kern-Mantelsystemen zusammengesintert oder zusammengeschmolzen sind.

Glasfasern und/oder Faserstäbe können Licht übertragen und damit die Funktion eines Lichtleiters ausüben. Ebenfalls möglich ist allerdings auch die Funktion als Bildleiter, wenn zwischen der Lage der einzelnen Faserkerne an der Eingangsfläche und der Lage der einzelnen Faserkerne an der Ausgangfläche des Bildleiters eine 1:1 Zuordnung besteht. Die Begriffe sind dem Fachmann bekannt und werden im Folgenden nicht weiter ausgeführt.

Eine relevante Kenngröße zum Ausbilden eines Lichtleitelements ist die Differenz des mittleren thermischen Längenausdehnungskoeffizienten des Kernglases und des mittleren thermischen Längenausdehnungskoeffizienten des Mantelglases, die auch als "ΔCTE(Kern-Mantel)" bezeichnet wird. Je nach Anwendung der optischen Faser ergeben sich positive ΔCTE(Kern-Mantel), aber auch eine Differenz = 0, sowie auch negative Differenzen. Für Lichtleiter ist ΔCTE(Kern-Mantel) ≥ 0 ppm/K bevorzugt. Für Bildleiter ist ΔCTE(Kern-Mantel) ≥ -0,5 ppm/K bevorzugt."

In einigen Ausführungsformen (insbesondere bei Lichtleitern (LLF)) weist das Kernglas einen mittleren thermischen Längenausdehnungskoeffizienten auf, der größer ist als der des verwendeten Mantelglases. Dadurch kann die Festigkeit, insbesondere die Faserfestigkeit verbessert werden. In einigen Ausführungsformen liegt die Differenz des mittleren thermischen Längenausdehnungskoeffizienten des Kernglases und des mittleren thermischen Längenausdehnungskoeffizienten des Mantelglases in einem Bereich von 0 bis 5,5 ppm/K, beispielsweise von 0,1 bis 5,0 ppm/K, von 0,2 bis 4,5 ppm/K, von 0,5 bis 4,0 ppm/K, von 1,0 bis 3,5 ppm/K, von 1,2 bis 3,0 ppm/K, von 1,5 bis 2,6 ppm/K, oder von >2,0 ppm/K bis 2,4 ppm/K. In einigen Ausführungsformen beträgt die Differenz des mittleren thermischen Längenausdehnungskoeffizienten des Kernglases und des mittleren thermischen Längenausdehnungskoeffizienten des Mantelglases mindestens 0,1 ppm/K, beispielsweise mindestens 0,2 ppm/K, mindestens 0,5 ppm/K, mindestens 1,0 ppm/K, mindestens 1,2 ppm/K, mindestens 1,5 ppm/K, oder mehr als 2,0 ppm/K. In einigen Ausführungsformen beträgt die Differenz des mittleren thermischen Längenausdehnungskoeffizienten des Kernglases und des mittleren thermischen Längenausdehnungskoeffizienten des Mantelglases höchstens 5,0 ppm/K, beispielsweise höchstens 4,5 ppm/K, höchstens 4,0 ppm/K, höchstens 3,5 ppm/K, höchstens 3,0 ppm/K, höchstens 2,6 ppm/K, oder höchstens 2,4 ppm/K.

In einigen Ausführungsformen (insbesondere bei Bildleitern (LFB)) liegt die Differenz des mittleren thermischen Längenausdehnungskoeffizienten des Kernglases und des mittleren thermischen Längenausdehnungskoeffizienten des Mantelglases in einem Bereich von -0,5 bis 3,5 ppm/K, beispielsweise von -0,4 bis 3,0 ppm/K, von -0,3 bis 2,5 ppm/K, von 0 bis 2,0 ppm/K, oder von 0,5 bis 1,5 ppm/K. In einigen Ausführungsformen beträgt die Differenz des mittleren thermischen Längenausdehnungskoeffizienten des Kernglases und des mittleren thermischen Längenausdehnungskoeffizienten des Mantelglases mindestens -0,5 ppm/K, beispielsweise mindestens -0,4 ppm/K, mindestens -0,3 ppm/K, mindestens -0,2 ppm/K, mindestens -0,1 ppm/K, mindestens 0 ppm/K, oder mindestens 0,5 ppm/K. In einigen Ausführungsformen beträgt die Differenz des mittleren thermischen Längenausdehnungskoeffizienten des Kernglases und des mittleren thermischen Längenausdehnungskoeffizienten des Mantelglases höchstens 3,5 ppm/K, beispielsweise höchstens 3,0 ppm/K, höchstens 2,5 ppm/K, höchstens 2,0 ppm/K, oder höchstens 1,5 ppm/K.In einigen Ausführungsformen weist das Mantelglas einen mittleren thermischen Längenausdehnungskoeffizienten in einem Bereich von 3,5 bis 7,0 ppm/K auf, beispielsweise in einem Bereich 4,0 bis 6,5 ppm/K, oder von 4,5 bis 6,0 ppm/K. In einigen Ausführungsformen beträgt der mittlere thermische Längenausdehnungskoeffizient des Mantelglases mindestens 3,5 ppm/K, beispielsweise mindestens 4,0 ppm/K, oder mindestens 4,5 ppm/K. In einigen Ausführungsformen beträgt der mittlere thermische Längenausdehnungskoeffizient des Mantelglases höchstens 7,0 ppm/K, beispielsweise höchstens 6,5 ppm/K, oder höchstens 6,0 ppm/K.

Der Brechungsindex des Mantelglases ist niedriger als der des Kernglases, damit das Licht an der Grenzfläche zwischen Kern- und Mantelglas reflektiert wird. In einigen Ausführungsformen liegt die Differenz des Brechungsindex n_{d} des Kernglases und des Brechungsindex n_{d} des Mantelglases in einem Bereich von 0,05 bis 0,40, beispielsweise von 0,08 bis 0,35, von 0,10 bis 0,30, von 0,15 bis 0,27, oder von 0,20 bis 0,25. In einigen Ausführungsformen beträgt die Differenz des Brechungsindex n_{d} des Kernglases und des Brechungsindex n_{d} des Mantelglases mindestens 0,05, beispielsweise mindestens 0,08, mindestens 0,10, mindestens 0,15, oder mindestens 0,20. In einigen Ausführungsformen beträgt die Differenz des Brechungsindex n_{d} des Kernglases und des Brechungsindex n_{d} des Mantelglases höchstens 0,40, beispielsweise höchstens 0,35, höchstens 0,30, höchstens 0,27, höchstens 0,27, oder höchstens 0,25.

In einigen Ausführungsformen liegt der Brechungsindex n_{d} des Mantelglases in einem Bereich von 1,45 bis 1,60, beispielsweise von 1,46 bis 1,55, von 1,47 bis 1,54, oder von 1,48 bis 1,52. In einigen Ausführungsformen beträgt der Brechungsindex n_{d} des Mantelglases mindestens 1,45, beispielsweise mindestens 1,46, mindestens 1,47, oder mindestens 1,48. In einigen Ausführungsformen beträgt der Brechungsindex n_{d} des Mantelglases höchstens 1,60, beispielsweise höchstens 1,55, höchstens 1,54, oder höchstens 1,52.

In einigen Ausführungsformen weist das Mantelglas einen Gehalt an SiO₂ von >60 Gew.-% auf, beispielsweise >65 Gew.-% oder mindestens 69 Gew.-% auf. Der SiO₂-Gehalt beträgt in einigen Ausführungsformen höchstens 75 Gew.-%, beispielsweise bis zu 73 Gew.-%. Das Mantelglas umgibt das erfindungsgemäße Glas in dem Licht- und/oder Bildleiter. Das erfindungsgemäße Glas bildet das sogenannte Kernglas. Daher ist das Mantelglas tendenziell stärkeren Umwelteinflüssen ausgesetzt als das Kernglas. Ein hoher SiO₂-Gehalt verleiht bessere chemische Resistenz. Folglich ist der Gehalt an dieser Komponente im Mantelglas bevorzugt größer als im Kernglas.

Das Mantelglas umfasst in einigen Ausführungsformen ferner mindestens 5,5 Gew.-% an Alkalioxiden. In einigen Ausführungsformen beträgt der Gehalt des Mantelglases an Alkalioxiden mindestens 7 Gew.-%, beispielsweise mindestens 8 Gew.-%. Alkalioxide umfassen dabei insbesondere Na₂O, K₂O, Li₂O.

Der Gehalt an Na₂O beträgt in einigen Ausführungsformen mindestens 0,5 Gew.-%, beispielsweise mindestens 2 Gew.-%. In einigen Ausführungsform des Mantelglases sind mindestens 6 Gew.-% an Na₂O im Mantelglas enthalten. Der Gehalt an Na₂O beträgt in einigen Ausführungsformen höchstens 15,5 Gew.-%, beispielsweise höchstens 15 Gew.-%.

Li₂O liegt in einigen Ausführungsformen mit einem Anteil von bis zu 0,7 Gew.-%, beispielsweise bis zu 0,6 Gew.-% im Mantelglas vor. In einigen Ausführungsformen ist das Mantelglas frei von Li₂O.

Der Gehalt an K₂O am Mantelglas beträgt in einigen Ausführungsformen mindestens 2 Gew.-%, beispielsweise mindestens 2,5 Gew.-%. Der Gehalt an K₂O beträgt in einigen Ausführungsformen höchstens 8 Gew.-%, beispielsweise bis zu 7,5 Gew.-%. In einigen Ausführungsformen ist das Glas frei von K₂O. Das Mantelglas enthält in einigen Ausführungsformen neben Na₂O und K₂O keine weiteren Alkalioxide.

Das Mantelglas umfasst in einigen Ausführungsformen mindestens 0,5 Gew.-% an Oxiden ausgewählt aus der Gruppe bestehend aus CaO, MgO, BaO und ZnO und Mischungen davon. In einigen Ausführungsformen beträgt der Gesamtgehalt dieser Oxide mindestens 0,6 Gew.-%. Der Gesamtgehalt an solchen Oxiden beträgt in einigen Ausführungsformen höchstens 12 Gew.-%, beispielsweise höchstens 11 Gew.-%, höchstens 5 Gew.-%, oder höchstens 2,5 Gew.-%. In einigen Ausführungsformen umfasst das Mantelglas genau zwei Oxide ausgewählt aus CaO, MgO, BaO und ZnO. In einigen Ausführungsformen umfasst das Mantelglas nur ein Oxid ausgewählt aus der Gruppe bestehend aus CaO, MgO, BaO und ZnO.

Das Mantelglas umfasst in einigen Ausführungsformen Al₂O₃ mit einem Gehalt von mindestens 0,5 Gew.-%, beispielsweise mindestens 1 Gew.-%, oder mindestens 2 Gew.-%. Das Mantelgas umfasst in einigen Ausführungsformen höchstens 7,5 Gew.-%, beispielsweise bis zu 7 Gew.-%, höchstens 3 Gew.-%, oder höchstens 1 Gew.-% an Al₂O₃.

Das Mantelglas kann B₂O₃ umfassen, wobei in einigen Ausführungsformen mindestens 9 Gew.-%, oder mindestens 9,5 Gew.-% an B₂O₃ im Mantelglas enthalten sind. In einigen Ausführungsformen enthält das Mantelglas höchstens 19 Gew.-%, beispielsweise bis zu 18,5 Gew.-% B₂O₃.

In einigen Ausführungsformen umfasst das Mantelglas einen höheren Gehalt der Summe der Komponenten B₂O₃ und Al₂O₃ als das Kernglas.

In einigen Ausführungsformen ist der Gehalt an SiO₂ im Mantelglas größer als der SiO₂-Gehalt im Kernglas. Außerdem ist in einigen Ausführungsformen der La₂O₃-Gehalt im Mantelglas viel kleiner als im Kernglas. Zu hohe SiO₂-Anteile im Kern erlauben nicht mehr die Einstellung der relativ hohen Brechungsindices, wenn diese mit La₂O₃ eingestellt werden. Außerdem ist der ZnO-Gehalt im Mantelglas in einigen Ausführungsformen viel geringer als im Kernglas. Der Grund ist, dass die Viskosität des Kernglases vorzugsweise geringer ist als die des Mantelglases. Das verbessert die Faserzugeigenschaften. In einigen Ausführungsformen ist die Summe der Gehalte von ZnO und BaO im Mantelglas kleiner als diese Summe im Kernglas.

Das Mantelglas kann ZrO₂ enthalten, wobei in einigen Ausführungsformen höchstens 0,04 Gew.-%, oder bis zu 0,03 Gew.-% ZrO₂ im Mantelglas vorliegen.

As₂O₃ kann beispielsweise mit einem Gehalt von bis zu 0,05 Gew.-%, oder bis zu 0,01 Gew.-% im Mantelglas enthalten sein. Arsenoxid ist verantwortlich für Solarisation. In einigen Ausführungsformen ist das Mantelglas frei von As₂O₃.

Sb₂O₃ kann beispielsweise mit einem Gehalt von bis zu 0,05 Gew.-%, oder bis zu 0,01 Gew.-% im Mantelglas enthalten sein. In einigen Ausführungsformen ist das Mantelglas frei von Sb₂O₃.

Das Mantelglas kann ferner Fluor bzw. Fluorid und/oder Chlor bzw. Chlorid umfassen. Der Gehalt an Fluorid beträgt in einigen Ausführungsformen bis zu 0,6 Gew.-%, oder bis zu 0,55 Gew.-%. Chlorid kann in einem Gehalt von beispielsweise höchstens 0,2, oder bis zu 0,15 Gew.-% am Mantelglas enthalten sein. Einige Ausführungsformen des Mantelglases sind frei von Fluor bzw. Fluorid und/oder Chlor bzw. Chlorid.

Die folgende Tabelle zeigt einige bevorzugte Zusammensetzungen von Mantelgläsern, die zusammen mit den erfindungsgemäßen Gläsern verwendet werden können. Die Mantelgläser enthalten (in Gew.-%):

| Komponente | Gruppe 1 | Gruppe 2 | Gruppe 3 | Gruppe 4 |
|---|---|---|---|---|
| SiO₂ | 70 - 78 | 63 - 75 | 75 - 85 | 62 - 70 |
| Al₂O₃ | 5 - 10 | 1 - 7 | 1 - 5 | 1 - 10 |
| B₂O₃ | 5 - 14 | 0 - 3 | 10 - 14 | > 15 |
| Li₂O | frei | 0 - 1 | 0 - 3 | < 0,1 |
| Na₂O | 0 - 10 | 8 - 20 | 2 - 8 | 0 - 10 |
| K₂O | 0 - 10 | 0 - 6 | 0 - 1 | 0 - 10 |
| MgO | 0 - 1 | 0 - 5 | frei | 0 - 5 |
| CaO | 0 - 2 | 1 - 9 | frei | 0 - 5 |
| SrO | 0 - 1 | frei | frei | 0 - 5 |
| BaO | 0 - 1 | 0 - 5 | frei | 0 - 5 |
| F | 0 - 1 | 0 - 1 | frei | 0 - 1 |

Ein Fachmann ist in der Lage, anhand seines Fachwissens auch noch weitere Mantelgläser einzusetzen.

Als besonders vorteilhaft hat sich ein Borsilikatglas als Mantelglas herausgestellt, welches mit dem zuvor beschriebenen Varianten zum Kernglas eine robuste, d.h. zugfeste Weitwinkelfaser mit einer vorteilhafenNumerischen Apertur NA von 0,86, entsprechend eines 2α-Öffnungswinkelns von 120°, ergibt.

In einigen Ausführungsformen liegt die numerische Apertur NA der Faser in einem Bereich von 0,40 bis 1,30, beispielsweise von 0,45 bis 1,20, von 0,50 bis 1,10, von 0,60 bis 1,05, von 0,70 bis 1,00, von 0,75 bis 0,95, von 0,77 bis 0,93, oder von 0,80 bis 0,90. In einigen Ausführungsformen beträgt die numerische Apertur der Faser mindestens 0,40, beispielsweise mindestens 0,45, mindestens 0,50, mindestens 0,60, mindestens 0,70, mindestens 0,75, mindestens 0,77, oder mindestens 0,80. In einigen Ausführungsformen beträgt die numerische Apertur der Faser höchstens 1,30, beispielsweise höchstens 1,20, höchstens 1,10, höchstens 1,05, höchstens 1,00, höchstens 0,95, beispielsweise höchstens 0,93, oder höchstens 0,90.

In einigen Ausführungsformen liegt der Öffnungswinkel 2α der Faser in einem Bereich von 95° bis 140°, beispielsweise von 100° bis 135°, von 105° bis 130°, von 110° bis 125°, oder von 115° bis 120°. In einigen Ausführungsformen beträgt der Öffnungswinkel der Faser mindestens 95°, beispielsweise mindestens 100°, mindestens 105°, mindestens 110°, oder mindestens 115°. In einigen Ausführungsformen beträgt der Öffnungswinkel der Faser höchstens 140°, beispielsweise höchstens 135°, höchstens 130°, höchstens 125°, oder höchstens 120°.

Für insbesondere endoskopische Anwendungen mit Kamera-Chips, deren diagonaler Sichtwinkel in etwa 120° entspricht, ist ein 2α-Öffnungswinkel von mindestens 100°, bevorzugt von 120°, besonders vorteilhaft, was einer NA von 0,86 entspricht.

In einigen Ausführungsformen weist die Faser eine Länge in einem Bereich von 0,1 bis 50 m auf, beispielsweise von 0,2 bis 25 m, von 0,5 bis 10 m, von 1,0 bis 5,0 m, von 1,0 bis 3,0 m, oder von 1,5 bis 2,5 m. In einigen Ausführungsformen weist die Faser eine Länge von mindestens 0,1 m auf, beispielsweise mindestens 0,2 m, mindestens 0,5 m, mindestens 1,0 m, oder mindestens 1,5 m. In einigen Ausführungsformen weist die Faser eine Länge von höchstens 50 m auf, beispielsweise höchstens 25 m, höchstens 10 m, höchstens 5,0 m, höchstens 3,0 m, oder höchstens 2,5 m. Natürlich können derartige Fasern auch in deutlich größeren Längen in einem Faserzugverfahren bereitgestellt werden. So ist es durchaus üblich zunächst auch Längen von größer 50 m, einigen 100 m bis hin zu wenigen Kilometern herzustellen und diese anschließend in bspw. oben genannte Längen zu konfektionieren.

In einigen Ausführungsformen weist die Faser einen Durchmesser in einem Bereich von 2,0 bis 1000 µm auf, beispielsweise in einem Bereich von 3,0 bis 750 µm, von 4,0 bis 500 µm, von 10 bis 425 µm, von 20 bis 350 µm, von 25 bis 150 µm, oder von 35 bis 100 µm. In einigen Ausführungsformen weist die Faser einen Durchmesser von mindestens 2,0 µm auf, beispielsweise mindestens 3,0 µm, mindestens 4,0 µm, mindestens 10 µm, mindestens 20 µm, mindestens 25 µm, oder mindestens 35 µm. In einigen Ausführungsformen weist die Faser einen Durchmesser von höchstens 1000 µm auf, beispielsweise höchstens 750 µm, höchstens 500 µm, höchstens 425 µm, höchstens 350 µm, höchstens 150 µm, oder höchstens 100 µm.

In einigen Ausführungsformen (insbesondere bei Lichtleitern (LLF)) weist die Faser einen Durchmesser in einem Bereich von 4,0 bis 1000 µm auf, beispielsweise von 10 bis 350 µm, von 15 bis 150 µm, von 20 bis 100 µm, oder von 25 bis 75 µm. In einigen Ausführungsformen (insbesondere bei Lichtleitern (LLF)) weist die Faser einen Durchmesser von mindestens 4,0 µm, beispielsweise mindestens 10 µm, mindestens 15 µm, mindestens 20 µm, oder mindestens 25 µm. In einigen Ausführungsformen (insbesondere bei Lichtleitern (LLF)) weist die Faser einen Durchmesser von höchstens 1000 µm auf, beispielsweise, höchstens 350 µm, höchstens 150 µm, höchstens 100 µm, oder höchstens 75 µm.

In einigen Ausführungsformen (insbesondere bei Bildleitern (LFB)) weist die Faser einen Durchmesser in einem Bereich von 2,0 bis 10 µm auf, beispielsweise von 3,0 bis 7,0 µm oder von 4,0 bis 6,0 µm. In einigen Ausführungsformen (insbesondere bei Bildleitern (LFB)) weist die Faser einen Durchmesser von mindestens 2,0 µm auf, beispielsweise mindestens 3,0 µm oder mindestens 4,0 µm. In einigen Ausführungsformen (insbesondere bei Bildleitern (LFB)) weist die Faser einen Durchmesser von höchstens 10 µm auf, beispielsweise höchstens 7,0 µm oder höchstens 6,0 µm.

Die Erfindung betrifft auch ein Faserbündel umfassend eine oder mehrere erfindungsgemäße Fasern, oder ein Faserbündel bestehend aus zwei oder mehr erfindungsgemäßen Fasern.

In einigen Ausführungsformen weisen solche Faserbündel eine Länge in einem Bereich von 0,1 bis 50 m auf, beispielsweise von 0,2 bis 25 m, von 0,5 bis 10 m, von 1,0 bis 5,0 m, von 1,0 bis 3,0 m, oder von 1,5 bis 2,5 m. In einigen Ausführungsformen weisen die Faserbündel eine Länge von mindestens 0,1 m auf, beispielsweise mindestens 0,2 m, mindestens 0,5 m, mindestens 1,0 m, oder mindestens 1,5 m. In einigen Ausführungsformen weisen die Faserbündel eine Länge von höchstens 50 m auf, beispielsweise höchstens 25 m, höchstens 10 m, höchstens 5,0 m, höchstens 3,0 m, oder höchstens 2,5 m.

In einigen Ausführungsformen (insbesondere bei Bildleitern, beispielsweise LFB) weist das Faserbündel 100 bis 50.000 Fasern auf, beispielsweise 200 bis 40.000, 500 bis 25.000, 1.000 bis 15.000, oder 3.000 bis 10.000 Fasern. In einigen Ausführungsformen (insbesondere bei Bildleitern, beispielsweise LFB) weist das Faserbündel mindestens 100 Fasern auf, beispielsweise mindestens 200, mindestens 500, mindestens 1.000, mindestens 3.000, mindestens 5.000, mindestens 7.500, oder mindestens 10.000 Fasern. In einigen Ausführungsformen (insbesondere bei Bildleitern, beispielsweise LFB) weist das Faserbündel höchstens 100.000 Fasern auf, beispielsweise höchstens 50.000, höchstens 40.000, höchstens 25.000, höchstens 15.000, oder höchstens 10.000 Fasern.

In einigen Ausführungsformen (insbesondere bei Lichtleitern (LLF)) weist das Faserbündel 3 bis 1.000 Fasern auf, beispielsweise 5 bis 750, 10 bis 500, 20 bis 200, oder 50 bis 100 Fasern. In einigen Ausführungsformen (insbesondere bei Lichtleitern (LLF)) weist das Faserbündel mindestens 3 Fasern auf, beispielsweise mindestens 5, mindestens 10, mindestens 20, oder mindestens 50 Fasern. In einigen Ausführungsformen (insbesondere bei Lichtleitern (LLF)) weist das Faserbündel höchstens 1.000 Fasern auf, beispielsweise höchstens 750, höchstens 500, höchstens 200, oder höchstens 100 Fasern.

### Herstellungsverfahren

Die Erfindung betrifft auch ein Verfahren zur Herstellung eines erfindungsgemäßen Glases oder Lichtleitelements. Das Verfahren umfasst insbesondere die folgenden Schritte:
- Schmelzen der Glasrohstoffe,
- Kühlen des erhaltenen Glases, wobei ein Glas oder Lichtleitelement der Erfindung erhalten wird.

In einigen Ausführungsformen enthält das Verfahren den Schritt des Läuterns der Glasschmelze. In einigen Ausführungsformen liegt die Läutertemperatur in einem Bereich von 1150°C bis 1650°C, beispielsweise von 1200°C bis 1600°C, von 1225°C bis 1550°C, von 1250°C bis 1500°C, von 1275°C bis 1450°C, von 1300°C bis 1400°C, oder von 1320°C bis 1360°C. In einigen Ausführungsformen beträgt die Läutertemperatur mindestens 1150°C, beispielsweise mindestens 1200°C, mindestens 1225°C, mindestens 1250°C, mindestens 1275°C, mindestens 1300°C, oder mindestens 1320°C. In einigen Ausführungsformen beträgt die Läutertemperatur höchstens 1650°C, beispielsweise höchstens 1600°C, höchstens 1550°C, höchstens 1500°C, höchstens 1450°C, höchstens 1400°C, oder höchstens 1360°C.

Das Glasgemenge kann mit Hochfrequenzheizung (HF) aufgeschmolzen und/oder geläutert werden. Es ist aber ebenfalls möglich, das Gemenge ohne Hochfrequenzheizung aufzuschmelzen und zu läutern. Es ist ein besonderer Vorteil des Glases der vorliegenden Erfindung, dass bei dessen Herstellung auf die aufwendige Hochfrequenzheizung verzichtet werden kann. Ein Verzicht auf Hochfrequenzheizung ist auch aus dem Grund vorteilhaft, dass in Anwesenheit zunehmender Gehalte an Nb und Ti ein HF-geläutertes Glas braun beziehungsweise gelb/grün wird. Enthält das Glas Fe als Verunreinigung, führt HF-Läuterung zu einem vergrößerten Gehalt an Fe(II), welches im Nahinfrarot (NIR) absorbiert.

Unter "Hochfrequenzheizung" wird ein Verfahren verstanden, bei dem das zu erhitzende Gemenge durch induktive Einkopplung eines elektromagnetischen Wechselfeldes erwärmt wird. Das elektromagnetische Wechselfeld weist beispielsweise Frequenzen von wenigstens 50 kHz, oder wenigstens 100 kHz auf. Die Frequenz beträgt beispielsweise höchstens 5 MHz, höchstens 3 MHz, oder höchstens 1 MHz. Das elektromagnetische Feld erzeugt in der elektrisch leitfähigen Glasschmelze Wechselströme, die aufgrund der Joule'schen Wärme zu einem direkten Aufheizen der Schmelze führen.

Der Schritt des Kühlens kann aktives Kühlen, passives Kühlen oder eine Mischung von beiden umfassen.

In einigen Ausführungsformen umfasst das Verfahren den Schritt des Bearbeitens der Glasschmelze, insbesondere mittels Down Draw, Overflow Fusion, Floaten oder Rohrzug, insbesondere Danner-Verfahren, Vello-Verfahren oder A-Zug-Verfahren (Vertikalzug-Verfahren).

In einigen Ausführungsformen umfasst das Verfahren den Schritt des Verarbeitens eines Lichtleitelements, mit einem Faserzugverfahren oder Wiederziehen zu einer Glasfaser bzw. Lichtleitfaser, zu einer Linsenvorform oder zu einem Behälter. In einigen Ausführungsformen umfasst das Verfahren den Schritt des Verarbeitens eines Glasartikels mit einem Faserzugverfahren zu einer Glasfaser.

### Verwendung

Die Erfindung betrifft auch die Verwendung eines erfindungsgemäßen Glases oder Glasartikels als Faserglas. Die Erfindung betrifft auch die Verwendung eines erfindungsgemäßen Glases oder Lichtleitelements in oder als Glasfaser. Die Erfindung betrifft auch die Verwendung eines erfindungsgemäßen Glases als Kernglas, insbesondere als Kernglas in einem Licht- und/oder Bildleiter. Die Erfindung betrifft auch die Verwendung eines erfindungsgemäßen Glases oder Glasartikels als oder in einem Licht- und/oder Bildleiter.

Die Erfindung betrifft auch die Verwendung eines erfindungsgemäßen Glases oder Glasartikels für die Bereiche Abbildung, Projektion, Telekommunikation, optische Nachrichtentechnik, Mobile Drive, Lasertechnologie und Desinfektion sowie optische Elemente bzw. Vorformen (so genannte "Preformen") solcher optischen Elemente.

Besonders vorteilhaft ist die Verwendung in endoskopischen Systemen für die Industrie- und/oder Medizintechnik, insbesondere für Einweg-Endoskope bzw. Single-Use-Endoskope im Medizinbereich.

### Beispielhafte Ausgestaltungen

Einige bevorzugte Ausführungsformen betreffen ein Glas oder einen Glasartikel, umfassend die folgenden Komponenten in den angegebenen Anteilen (in Gew.-):

| **Komponente** | **Min (Gew.-%)** | **Max (Gew.-%)** |
|---|---|---|
| SiO₂ | 18 | 35 |
| B₂O₃ | 1,0 | 13 |
| CaO | 0 | 0,5 |
| BaO | 2,0 | 30 |
| SrO | 0 | 2,0 |
| ZnO | 1,0 | 18 |
| La₂O₃ | 5,0 | 40 |
| Gd₂O₃ | 0 | 8,0 |
| Y₂O₃ | 0 | 8,0 |
| ZrO₂ | 1,0 | 7,0 |
| Ta₂O₅ | 0 | 7,0 |
| Nb₂O₅ | 0 | 7,0 |
| Σ R₂O | 0 | 2,0 |

wobei das Glas mindestens eine der beiden Komponenten Gd₂O₃ und Y₂O₃ umfasst, wobei das Verhältnis der Summe der Gewichtsanteile von Gd₂O₃ und Y₂O₃ zum Gewichtsanteil von SiO₂ mindestens 0,01 beträgt, und wobei das Glas bei einer Probendicke von 25 mm und einer Wellenlänge von 380 nm eine Reintransmission von mindestens 0,900 aufweist.

Einige bevorzugte Ausführungsformen betreffen ein Glas oder einen Glasartikel, umfassend die folgenden Komponenten in den angegebenen Anteilen (in Gew.-):

| **Komponente** | **Min (Gew.-%)** | **Max (Gew.-%)** |
|---|---|---|
| SiO₂ | 18 | 35 |
| B₂O₃ | 1,0 | 13 |
| CaO | 0 | 0,5 |
| BaO | 2,0 | 30 |
| SrO | 0 | 2,0 |
| ZnO | 1,0 | 18 |
| La₂O₃ | 5,0 | 40 |
| Gd₂O₃ | 0 | 8,0 |
| Y₂O₃ | 0 | 8,0 |
| ZrO₂ | 1,0 | 7,0 |
| Ta₂O₅ | 0 | 7,0 |
| Nb₂O₅ | 0 | 7,0 |
| Σ R₂O | 0 | 2,0 |

wobei das Glas mindestens eine der beiden Komponenten Gd₂O₃ und Y₂O₃ umfasst, wobei das Verhältnis der Summe der Gewichtsanteile von Gd₂O₃ und Y₂O₃ zum Gewichtsanteil von SiO₂ mindestens 0,01 beträgt, und wobei die untere Entglasungsgrenze UEG mindestens 650°C beträgt, wenn das Glas für eine Haltezeit von 5 Minuten in einem Gradientenofen mit aufsteigender Temperaturführung thermisch behandelt wird.

Einige bevorzugte Ausführungsformen betreffen ein Glas oder einen Glasartikel, umfassend die folgenden Komponenten in den angegebenen Anteilen (in Gew.-):

| **Komponente** | **Min (Gew.-%)** | **Max (Gew.-%)** |
|---|---|---|
| SiO₂ | 18 | 35 |
| B₂O₃ | 1,0 | 13 |
| CaO | 0 | 0,5 |
| BaO | 2,0 | 30 |
| SrO | 0 | 2,0 |
| ZnO | 1,0 | 18 |
| La₂O₃ | 5,0 | 40 |
| Gd₂O₃ | 0 | 8,0 |
| Y₂O₃ | 0 | 8,0 |
| ZrO₂ | 1,0 | 7,0 |
| Ta₂O₅ | 0 | 7,0 |
| Nb₂O₅ | 0 | 7,0 |
| Σ R₂O | 0 | 2,0 |

wobei das Glas mindestens eine der beiden Komponenten Gd₂O₃ und Y₂O₃ umfasst, wobei das Verhältnis der Summe der Gewichtsanteile von Gd₂O₃ und Y₂O₃ zum Gewichtsanteil von SiO₂ mindestens 0,01 beträgt, und wobei die Differenz zwischen der unteren Entglasungsgrenze UEG und dem Erweichungspunkt T7,6 mindestens 50 K beträgt.

Einige bevorzugte Ausführungsformen betreffen ein Glas oder einen Glasartikel, umfassend die folgenden Komponenten in den angegebenen Anteilen (in Gew.-):

| **Komponente** | **Min (Gew.-%)** | **Max (Gew.-%)** |
|---|---|---|
| SiO₂ | 18 | 35 |
| B₂O₃ | 1,0 | 13 |
| CaO | 0 | 0,5 |
| BaO | 2,0 | 30 |
| SrO | 0 | 2,0 |
| ZnO | 1,0 | 18 |
| La₂O₃ | 5,0 | 40 |
| Gd₂O₃ | 0 | 8,0 |
| Y₂O₃ | 0 | 8,0 |
| ZrO₂ | 1,0 | 7,0 |
| Ta₂O₅ | 0 | 7,0 |
| Nb₂O₅ | 0 | 7,0 |
| Σ R₂O | 0 | 2,0 |

wobei das Glas mindestens eine der beiden Komponenten Gd₂O₃ und Y₂O₃ umfasst, wobei das Verhältnis der Summe der Gewichtsanteile von Gd₂O₃ und Y₂O₃ zum Gewichtsanteil von SiO₂ mindestens 0,01 beträgt, und wobei der Brechungsindex n_{d} des Glases in einem Bereich von 1,65 bis 1,80 liegt.

Einige bevorzugte Ausführungsformen betreffen ein Glas oder einen Glasartikel, umfassend die folgenden Komponenten in den angegebenen Anteilen (in Gew.-):

| **Komponente** | **Min (Gew.-%)** | **Max (Gew.-%)** |
|---|---|---|
| SiO₂ | 18 | 35 |
| B₂O₃ | 1,0 | 13 |
| CaO | 0 | 0,5 |
| BaO | 2,0 | 30 |
| SrO | 0 | 2,0 |
| ZnO | 1,0 | 18 |
| La₂O₃ | 5,0 | 40 |
| Gd₂O₃ | 0 | 8,0 |
| Y₂O₃ | 0 | 8,0 |
| ZrO₂ | 1,0 | 7,0 |
| Ta₂O₅ | 0 | 7,0 |
| Nb₂O₅ | 0 | 7,0 |
| Σ R₂O | 0 | 2,0 |

wobei das Glas mindestens eine der beiden Komponenten Gd₂O₃ und Y₂O₃ umfasst, wobei das Verhältnis der Summe der Gewichtsanteile von Gd₂O₃ und Y₂O₃ zum Gewichtsanteil von SiO₂ mindestens 0,01 beträgt, wobei das Glas bei einer Probendicke von 25 mm und einer Wellenlänge von 380 nm eine Reintransmission von mindestens 0,900 aufweist und wobei die untere Entglasungsgrenze UEG mindestens 650°C beträgt, wenn das Glas für eine Haltezeit von 5 Minuten in einem Gradientenofen mit aufsteigender Temperaturführung thermisch behandelt wird.

Einige bevorzugte Ausführungsformen betreffen ein Glas oder einen Glasartikel, umfassend die folgenden Komponenten in den angegebenen Anteilen (in Gew.-):

| **Komponente** | **Min (Gew.-%)** | **Max (Gew.-%)** |
|---|---|---|
| SiO₂ | 18 | 35 |
| B₂O₃ | 1,0 | 13 |
| CaO | 0 | 0,5 |
| BaO | 2,0 | 30 |
| SrO | 0 | 2,0 |
| ZnO | 1,0 | 18 |
| La₂O₃ | 5,0 | 40 |
| Gd₂O₃ | 0 | 8,0 |
| Y₂O₃ | 0 | 8,0 |
| ZrO₂ | 1,0 | 7,0 |
| Ta₂O₅ | 0 | 7,0 |
| Nb₂O₅ | 0 | 7,0 |
| Σ R₂O | 0 | 2,0 |

wobei das Glas mindestens eine der beiden Komponenten Gd₂O₃ und Y₂O₃ umfasst, wobei das Verhältnis der Summe der Gewichtsanteile von Gd₂O₃ und Y₂O₃ zum Gewichtsanteil von SiO₂ mindestens 0,01 beträgt, wobei das Glas bei einer Probendicke von 25 mm und einer Wellenlänge von 380 nm eine Reintransmission von mindestens 0,900 aufweist und wobei die Differenz zwischen der unteren Entglasungsgrenze UEG und dem Erweichungspunkt T7,6 mindestens 50 K beträgt.

Einige bevorzugte Ausführungsformen betreffen ein Glas oder einen Glasartikel, umfassend die folgenden Komponenten in den angegebenen Anteilen (in Gew.-):

| **Komponente** | **Min (Gew.-%)** | **Max (Gew.-%)** |
|---|---|---|
| SiO₂ | 18 | 35 |
| B₂O₃ | 1,0 | 13 |
| CaO | 0 | 0,5 |
| BaO | 2,0 | 30 |
| SrO | 0 | 2,0 |
| ZnO | 1,0 | 18 |
| La₂O₃ | 5,0 | 40 |
| Gd₂O₃ | 0 | 8,0 |
| Y₂O₃ | 0 | 8,0 |
| ZrO₂ | 1,0 | 7,0 |
| Ta₂O₅ | 0 | 7,0 |
| Nb₂O₅ | 0 | 7,0 |
| Σ R₂O | 0 | 2,0 |

wobei das Glas mindestens eine der beiden Komponenten Gd₂O₃ und Y₂O₃ umfasst, wobei das Verhältnis der Summe der Gewichtsanteile von Gd₂O₃ und Y₂O₃ zum Gewichtsanteil von SiO₂ mindestens 0,01 beträgt, wobei das Glas bei einer Probendicke von 25 mm und einer Wellenlänge von 380 nm eine Reintransmission von mindestens 0,900 aufweist und wobei der Brechungsindex n_{d} des Glases in einem Bereich von 1,65 bis 1,80 liegt.

Einige bevorzugte Ausführungsformen betreffen ein Glas oder einen Glasartikel, umfassend die folgenden Komponenten in den angegebenen Anteilen (in Gew.-):

| **Komponente** | **Min (Gew.-%)** | **Max (Gew.-%)** |
|---|---|---|
| SiO₂ | 18 | 35 |
| B₂O₃ | 1,0 | 13 |
| CaO | 0 | 0,5 |
| BaO | 2,0 | 30 |
| SrO | 0 | 2,0 |
| ZnO | 1,0 | 18 |
| La₂O₃ | 5,0 | 40 |
| Gd₂O₃ | 0 | 8,0 |
| Y₂O₃ | 0 | 8,0 |
| ZrO₂ | 1,0 | 7,0 |
| Ta₂O₅ | 0 | 7,0 |
| Nb₂O₅ | 0 | 7,0 |
| Σ R₂O | 0 | 2,0 |

wobei das Glas mindestens eine der beiden Komponenten Gd₂O₃ und Y₂O₃ umfasst, wobei das Verhältnis der Summe der Gewichtsanteile von Gd₂O₃ und Y₂O₃ zum Gewichtsanteil von SiO₂ mindestens 0,01 beträgt, wobei die untere Entglasungsgrenze UEG mindestens 650°C beträgt, wenn das Glas für eine Haltezeit von 5 Minuten in einem Gradientenofen mit aufsteigender Temperaturführung thermisch behandelt wird und wobei die Differenz zwischen der unteren Entglasungsgrenze UEG und dem Erweichungspunkt T7,6 mindestens 50 K beträgt.

Einige bevorzugte Ausführungsformen betreffen ein Glas oder einen Glasartikel, umfassend die folgenden Komponenten in den angegebenen Anteilen (in Gew.-):

| **Komponente** | **Min (Gew.-%)** | **Max (Gew.-%)** |
|---|---|---|
| SiO₂ | 18 | 35 |
| B₂O₃ | 1,0 | 13 |
| CaO | 0 | 0,5 |
| BaO | 2,0 | 30 |
| SrO | 0 | 2,0 |
| ZnO | 1,0 | 18 |
| La₂O₃ | 5,0 | 40 |
| Gd₂O₃ | 0 | 8,0 |
| Y₂O₃ | 0 | 8,0 |
| ZrO₂ | 1,0 | 7,0 |
| Ta₂O₅ | 0 | 7,0 |
| Nb₂O₅ | 0 | 7,0 |
| Σ R₂O | 0 | 2,0 |

wobei das Glas mindestens eine der beiden Komponenten Gd₂O₃ und Y₂O₃ umfasst, wobei das Verhältnis der Summe der Gewichtsanteile von Gd₂O₃ und Y₂O₃ zum Gewichtsanteil von SiO₂ mindestens 0,01 beträgt, wobei die untere Entglasungsgrenze UEG mindestens 650°C beträgt, wenn das Glas für eine Haltezeit von 5 Minuten in einem Gradientenofen mit aufsteigender Temperaturführung thermisch behandelt wird und wobei der Brechungsindex n_{d} des Glases in einem Bereich von 1,65 bis 1,80 liegt.

Einige bevorzugte Ausführungsformen betreffen ein Glas oder einen Glasartikel, umfassend die folgenden Komponenten in den angegebenen Anteilen (in Gew.-):

| **Komponente** | **Min (Gew.-%)** | **Max (Gew.-%)** |
|---|---|---|
| SiO₂ | 18 | 35 |
| B₂O₃ | 1,0 | 13 |
| CaO | 0 | 0,5 |
| BaO | 2,0 | 30 |
| SrO | 0 | 2,0 |
| ZnO | 1,0 | 18 |
| La₂O₃ | 5,0 | 40 |
| Gd₂O₃ | 0 | 8,0 |
| Y₂O₃ | 0 | 8,0 |
| ZrO₂ | 1,0 | 7,0 |
| Ta₂O₅ | 0 | 7,0 |
| Nb₂O₅ | 0 | 7,0 |
| Σ R₂O | 0 | 2,0 |

wobei das Glas mindestens eine der beiden Komponenten Gd₂O₃ und Y₂O₃ umfasst, wobei das Verhältnis der Summe der Gewichtsanteile von Gd₂O₃ und Y₂O₃ zum Gewichtsanteil von SiO₂ mindestens 0,01 beträgt, wobei die Differenz zwischen der unteren Entglasungsgrenze UEG und dem Erweichungspunkt T7,6 mindestens 50 K beträgt und wobei der Brechungsindex n_{d} des Glases in einem Bereich von 1,65 bis 1,80 liegt.

Einige bevorzugte Ausführungsformen betreffen ein Glas oder einen Glasartikel, umfassend die folgenden Komponenten in den angegebenen Anteilen (in Gew.-):

| **Komponente** | **Min (Gew.-%)** | **Max (Gew.-%)** |
|---|---|---|
| SiO₂ | 18 | 35 |
| B₂O₃ | 1,0 | 13 |
| CaO | 0 | 0,5 |
| BaO | 2,0 | 30 |
| SrO | 0 | 2,0 |
| ZnO | 1,0 | 18 |
| La₂O₃ | 5,0 | 40 |
| Gd₂O₃ | 0 | 8,0 |
| Y₂O₃ | 0 | 8,0 |
| ZrO₂ | 1,0 | 7,0 |
| Ta₂O₅ | 0 | 7,0 |
| Nb₂O₅ | 0 | 7,0 |
| Σ R₂O | 0 | 2,0 |

wobei das Glas mindestens eine der beiden Komponenten Gd₂O₃ und Y₂O₃ umfasst, wobei das Verhältnis der Summe der Gewichtsanteile von Gd₂O₃ und Y₂O₃ zum Gewichtsanteil von SiO₂ mindestens 0,01 beträgt, wobei das Glas bei einer Probendicke von 25 mm und einer Wellenlänge von 380 nm eine Reintransmission von mindestens 0,900 aufweist, wobei die untere Entglasungsgrenze UEG mindestens 650°C beträgt, wenn das Glas für eine Haltezeit von 5 Minuten in einem Gradientenofen mit aufsteigender Temperaturführung thermisch behandelt wird und wobei die Differenz zwischen der unteren Entglasungsgrenze UEG und dem Erweichungspunkt T7,6 mindestens 50 K beträgt.

Einige bevorzugte Ausführungsformen betreffen ein Glas oder einen Glasartikel, umfassend die folgenden Komponenten in den angegebenen Anteilen (in Gew.-):

| **Komponente** | **Min (Gew.-%)** | **Max (Gew.-%)** |
|---|---|---|
| SiO₂ | 18 | 35 |
| B₂O₃ | 1,0 | 13 |
| CaO | 0 | 0,5 |
| BaO | 2,0 | 30 |
| SrO | 0 | 2,0 |
| ZnO | 1,0 | 18 |
| La₂O₃ | 5,0 | 40 |
| Gd₂O₃ | 0 | 8,0 |
| Y₂O₃ | 0 | 8,0 |
| ZrO₂ | 1,0 | 7,0 |
| Ta₂O₅ | 0 | 7,0 |
| Nb₂O₅ | 0 | 7,0 |
| Σ R₂O | 0 | 2,0 |

wobei das Glas mindestens eine der beiden Komponenten Gd₂O₃ und Y₂O₃ umfasst, wobei das Verhältnis der Summe der Gewichtsanteile von Gd₂O₃ und Y₂O₃ zum Gewichtsanteil von SiO₂ mindestens 0,01 beträgt, wobei das Glas bei einer Probendicke von 25 mm und einer Wellenlänge von 380 nm eine Reintransmission von mindestens 0,900 aufweist, wobei die untere Entglasungsgrenze UEG mindestens 650°C beträgt, wenn das Glas für eine Haltezeit von 5 Minuten in einem Gradientenofen mit aufsteigender Temperaturführung thermisch behandelt wird und wobei der Brechungsindex n_{d} des Glases in einem Bereich von 1,65 bis 1,80 liegt.

Einige bevorzugte Ausführungsformen betreffen ein Glas oder einen Glasartikel, umfassend die folgenden Komponenten in den angegebenen Anteilen (in Gew.-):

| **Komponente** | **Min (Gew.-%)** | **Max (Gew.-%)** |
|---|---|---|
| SiO₂ | 18 | 35 |
| B₂O₃ | 1,0 | 13 |
| CaO | 0 | 0,5 |
| BaO | 2,0 | 30 |
| SrO | 0 | 2,0 |
| ZnO | 1,0 | 18 |
| La₂O₃ | 5,0 | 40 |
| Gd₂O₃ | 0 | 8,0 |
| Y₂O₃ | 0 | 8,0 |
| ZrO₂ | 1,0 | 7,0 |
| Ta₂O₅ | 0 | 7,0 |
| Nb₂O₅ | 0 | 7,0 |
| Σ R₂O | 0 | 2,0 |

wobei das Glas mindestens eine der beiden Komponenten Gd₂O₃ und Y₂O₃ umfasst, wobei das Verhältnis der Summe der Gewichtsanteile von Gd₂O₃ und Y₂O₃ zum Gewichtsanteil von SiO₂ mindestens 0,01 beträgt, wobei das Glas bei einer Probendicke von 25 mm und einer Wellenlänge von 380 nm eine Reintransmission von mindestens 0,900 aufweist, wobei die Differenz zwischen der unteren Entglasungsgrenze UEG und dem Erweichungspunkt T7,6 mindestens 50 K beträgt und wobei der Brechungsindex n_{d} des Glases in einem Bereich von 1,65 bis 1,80 liegt.

Einige bevorzugte Ausführungsformen betreffen ein Glas oder einen Glasartikel, umfassend die folgenden Komponenten in den angegebenen Anteilen (in Gew.-):

| **Komponente** | **Min (Gew.-%)** | **Max (Gew.-%)** |
|---|---|---|
| SiO₂ | 18 | 35 |
| B₂O₃ | 1,0 | 13 |
| CaO | 0 | 0,5 |
| BaO | 2,0 | 30 |
| SrO | 0 | 2,0 |
| ZnO | 1,0 | 18 |
| La₂O₃ | 5,0 | 40 |
| Gd₂O₃ | 0 | 8,0 |
| Y₂O₃ | 0 | 8,0 |
| ZrO₂ | 1,0 | 7,0 |
| Ta₂O₅ | 0 | 7,0 |
| Nb₂O₅ | 0 | 7,0 |
| Σ R₂O | 0 | 2,0 |

wobei das Glas mindestens eine der beiden Komponenten Gd₂O₃ und Y₂O₃ umfasst, wobei das Verhältnis der Summe der Gewichtsanteile von Gd₂O₃ und Y₂O₃ zum Gewichtsanteil von SiO₂ mindestens 0,01 beträgt, wobei die untere Entglasungsgrenze UEG mindestens 650°C beträgt, wenn das Glas für eine Haltezeit von 5 Minuten in einem Gradientenofen mit aufsteigender Temperaturführung thermisch behandelt wird, wobei die Differenz zwischen der unteren Entglasungsgrenze UEG und dem Erweichungspunkt T7,6 mindestens 50 K beträgt und wobei der Brechungsindex n_{d} des Glases in einem Bereich von 1,65 bis 1,80 liegt.

Einige bevorzugte Ausführungsformen betreffen ein Glas oder einen Glasartikel, umfassend die folgenden Komponenten in den angegebenen Anteilen (in Gew.-):

| **Komponente** | **Min (Gew.-%)** | **Max (Gew.-%)** |
|---|---|---|
| SiO₂ | 18 | 35 |
| B₂O₃ | 1,0 | 13 |
| CaO | 0 | 0,5 |
| BaO | 2,0 | 30 |
| SrO | 0 | 2,0 |
| ZnO | 1,0 | 18 |
| La₂O₃ | 5,0 | 40 |
| Gd₂O₃ | 0 | 8,0 |
| Y₂O₃ | 0 | 8,0 |
| ZrO₂ | 1,0 | 7,0 |
| Ta₂O₅ | 0 | 7,0 |
| Nb₂O₅ | 0 | 7,0 |
| Σ R₂O | 0 | 2,0 |

wobei das Glas mindestens eine der beiden Komponenten Gd₂O₃ und Y₂O₃ umfasst, wobei das Verhältnis der Summe der Gewichtsanteile von Gd₂O₃ und Y₂O₃ zum Gewichtsanteil von SiO₂ mindestens 0,01 beträgt, wobei das Glas bei einer Probendicke von 25 mm und einer Wellenlänge von 380 nm eine Reintransmission von mindestens 0,900 aufweist, wobei die untere Entglasungsgrenze UEG mindestens 650°C beträgt, wenn das Glas für eine Haltezeit von 5 Minuten in einem Gradientenofen mit aufsteigender Temperaturführung thermisch behandelt wird, wobei die Differenz zwischen der unteren Entglasungsgrenze UEG und dem Erweichungspunkt T7,6 mindestens 50 K beträgt und wobei der Brechungsindex n_{d} des Glases in einem Bereich von 1,65 bis 1,80 liegt.

Wie zuvor beschrieben ist es besonders vorteilhaft, wenn sowohl Gd₂O₃ und Y₂O₃ in dem erfindungsgemäßen Glas vorhanden sind.

### Beispiele

### 1. Beispielgläser und deren Eigenschaften

Die folgende Tabelle zeigt Synthesezusammensetzungen und Eigenschaften ausgewählter Gläser. Die Anteile der einzelnen Komponenten bezeichnen die Anteile in der Synthesezusammensetzung in Gew.-%. Sie sind jeweils auf 100% normiert und mit Ausnahme des Läutermittels auf eine Nachkommastelle gerundet angegeben. Abweichungen von 100% sind rundungsbedingt. Die Reintransmission Ti bei 380 nm und bei 600 nm ist jeweils für eine Probendicke von 25 mm und auf drei Nachkommastellen gerundet angegeben. Die Beispiele wurden unter Laborbedingungen hergestellt, was mit verminderter innerer Qualität einhergeht. Unter Produktionsbedingungen werden mit den Glaszusammensetzungen der Erfindung noch höhere Reintransmissionen erreicht werden als in den vorliegenden Beispielen.

| **Komponente (Gew.-%)** | **Vgl.-Bsp. A** | **Vgl.-Bsp. B** | **Bsp. 1** | **Bsp. 2** | **Bsp. 3** |
|---|---|---|---|---|---|
| SiO₂ | 29,5 | 29,5 | 30,3 | 30,2 | 28,1 |
| B₂O₃ | 3,8 | 3,8 | 3,1 | 4,0 | 3,4 |
| Li₂O | 0,2 | 0,2 | 1,0 | 1,0 | 0,8 |
| Na₂O | 0,6 | 0,6 | 1,0 | | |
| BaO | 21,4 | 21,5 | 14,4 | 21,6 | 21,2 |
| ZnO | 13,8 | 13,9 | 1,9 | 2,1 | 13,5 |
| La₂O₃ | 21,8 | 21,8 | 24,8 | 26,9 | 20,7 |
| Gd₂O₃ | | | 5,2 | | 2,9 |
| Y₂O₃ | | | 6,8 | 1,0 | 1,0 |
| ZrO₂ | 2,4 | 2,4 | 2,6 | 5,7 | 3,4 |
| Ta₂O₅ | | | 2,6 | 2,4 | 4,8 |
| Nb₂O₅ | 6,2 | 6,2 | 6,2 | 5,1 | 0,4 |
| Sb₂O₃ | 0,30 | 0,05 | 0,05 | 0,05 | 0,05 |

| **Eigenschaften** | | | | | |
|---|---|---|---|---|---|
| Ti (380 nm) | 0,749 | 0,895 | 0,857 | 0,866 | 0,889 |
| Ti (600 nm) | 0,995 | 0,997 | 0,997 | 0,998 | 1,000 |
| n_{d} | 1,718 | 1,717 | 1,730 | 1,726 | 1,731 |
| Vd | 45,5 | 45,6 | 45,7 | | 47,4 |
| Dichte [g/cm³] | 4,12 | | 4,15 | 4,13 | 4,20 |
| CTE [ppm/K] | 7,2 | | 7,6 | 7,4 | 7,5 |
| Tg [°C] | 641 | | 665 | 668 | 635 |

Wie die Tabelle zeigt, stimmen die Beispiele und die Vergleichsbeispiele hinsichtlich Brechungsindex n_{d}, Abbe-Zahl v_{d}, Dichte, mittlerem thermischen Längenausdehnungskoeffizienten im Temperaturbereich von 20°C bis 300°C (englisch: "Coefficient of Thermal Expansion", CTE) und Glasübergangstemperatur Tg weitestgehend überein. CTE, Dichte und Tg wurden an Proben bestimmt, die vor der Messung mit einer Kühlrate von 120 K/h gekühlt wurden. Brechungsindex n_{d} und Abbe-Zahl v_{d} wurden an Proben bestimmt, die vor der Messung mit einer Kühlrate von 30 K/h gekühlt wurden. Werden derartige Proben vor der Messung nochmals gekühlt so erfolgt deren Kühlung in der Regel ab einer Temperatur, die oberhalb des Tg (ca. 100 K), aber unterhalb der Erweichungstemperatur, des Glases liegt.

Die folgende Tabelle zeigt Synthesezusammensetzungen und Eigenschaften weiterer Beispielgläser.

| **Komponente (Gew.-%)** | **Bsp. 4** | **Bsp. 5** | **Bsp. 6** | **Bsp. 7** | **Bsp. 8** | **Bsp. 9** |
|---|---|---|---|---|---|---|
| SiO₂ | 27,0 | 28,7 | 20,1 | 29,2 | 27,9 | 28,4 |
| B₂O₃ | 5,0 | 3,4 | 13,0 | 3,5 | 3,4 | 3,4 |
| Li₂O | | 0,3 | 1,1 | 0,3 | 0,8 | |
| Na₂O | | | 0,5 | | | |
| BaO | 26,4 | 21,2 | 11,6 | 21,6 | 21,2 | 20,2 |
| SrO | 2,0 | | | | | |
| ZnO | 1,1 | 14,6 | 7,9 | 12,7 | 13,9 | 14,4 |
| La₂O₃ | 34,2 | 20,2 | 29,5 | 20,6 | 19,7 | 20,2 |
| Gd₂O₃ | 0,5 | 1,9 | 5,7 | | 3,8 | 2,9 |
| Y₂O₃ | 0,6 | 1,4 | 6,3 | 1,0 | 1,0 | 1,0 |
| ZrO₂ | 1,9 | 2,9 | 2,1 | 3,4 | 3,4 | 2,9 |
| Ta₂O₅ | 0,3 | 4,8 | | 6,9 | 4,8 | 6,7 |
| Nb₂O₅ | 1,0 | 0,6 | 2,1 | 0,8 | 0,2 | |
| Sb₂O₃ | 0,05 | 0,05 | 0,05 | 0,05 | 0,05 | 0,05 |

| **Eigenschaften** | | | | | | |
|---|---|---|---|---|---|---|
| Ti (380 nm) | 0,921 | 0,927 | 0,939 | 0,941 | 0,944 | 0,946 |
| Ti (600 nm) | 0,997 | 1,000 | 0,997 | 0,997 | 1,000 | 0,994 |
| n_{d} | 1,720 | | 1,730 | 1,714 | 1,721 | 1,717 |
| Vd | 50,0 | | 49,3 | 47,4 | 47,8 | 47,6 |
| Dichte [g/cm³] | 4,34 | 4,20 | 4,20 | 4,24 | 4,24 | 4,33 |
| CTE [ppm/K] | 8,5 | 7,0 | 7,8 | 6,9 | 7,2 | 6,8 |
| Tg [°C] | 707 | 655 | 597 | 667 | 638 | 693 |

Die folgende Tabelle zeigt Synthesezusammensetzungen und Eigenschaften weiterer Beispielgläser.

| **Komponente (Gew.-%)** | **Bsp. 10** | **Bsp. 11** | **Bsp. 12** | **Bsp. 13** | **Bsp. 14** | **Bsp. 15** |
|---|---|---|---|---|---|---|
| SiO₂ | 28,7 | 28,7 | 28,0 | 28,6 | 28,7 | 28,0 |
| B₂O₃ | 3,4 | 3,5 | 3,4 | 3,4 | 3,4 | 3,4 |
| Li₂O | 0,3 | | 0,8 | 0,3 | 0,3 | 0,8 |
| BaO | 21,2 | 20,5 | 21,2 | 21,2 | 21,2 | 21,2 |
| ZnO | 13,5 | 13,6 | 13,9 | 13,5 | 13,5 | 13,9 |
| La₂O₃ | 19,2 | 20,5 | 19,7 | 19,3 | 20,2 | 19,7 |
| Gd₂O₃ | 2,9 | 3,9 | 3,8 | 2,9 | 2,9 | 3,8 |
| Y₂O₃ | 1,0 | | 1,0 | 1,0 | 1,4 | 1,0 |
| ZrO₂ | 2,9 | 2,9 | 3,4 | 2,9 | 3,2 | 3,4 |
| Ta₂O₅ | 6,3 | 6,3 | 4,7 | 6,3 | 4,8 | 4,7 |
| Nb₂O₅ | 0,8 | | 0,2 | 0,8 | 0,4 | 0,2 |
| Sb₂O₃ | 0,05 | 0,05 | 0,05 | 0,05 | 0,05 | 0,05 |

| **Eigenschaften** | | | | | | |
|---|---|---|---|---|---|---|
| Ti (380 nm) | 0,946 | 0,947 | 0,953 | 0,966 | 0,969 | 0,971 |
| Ti (600 nm) | 0,997 | 0,991 | 0,998 | 0,998 | 0,999 | 1,000 |
| n_{d} | 1,717 | 1,713 | 1,719 | 1,718 | 1,717 | 1,719 |
| Vd | 47,3 | 47,9 | 47,9 | 47,3 | 47,9 | 47,9 |
| Dichte [g/cm³] | 4,30 | 4,30 | 4,32 | 4,31 | 4,30 | |
| CTE [ppm/K] | 6,9 | 6,8 | 7,3 | 7,0 | 7,0 | |
| Tg [°C] | 663 | 690 | 635 | 659 | 662 | |

Schmelzbedingungen können auch bei gleicher Zusammensetzung zu leicht abweichenden Transmissionswerten führen. Eine Abnahme der Transmission ist beispielsweise beim Schmelzen in oder mit Pt beobachtbar. Dies gilt insbesondere bei Schmelzen mit kleinen Volumina, beispielsweise bei Laborschmelzen.

Die folgende Tabelle zeigt die Analysezusammensetzungen ausgewählter Gläser (in Gew.-%). Die Analyse der Zusammensetzungen erfolgte mittels Röntgenfluoreszenzspektroskopie (englisch: "X-ray Fluorescence Spectroscopy", XRF).

| **Komponente (Gew.-%)** | **Vgl.-Bsp. A** | **Vgl.-Bsp. B** | **Bsp. 12** | **Bsp. 13** | **Bsp. 14** | **Bsp. 15** |
|---|---|---|---|---|---|---|
| SiO₂ | 29,8 | 29,9 | 28,8 | 29,6 | 29,6 | 28,8 |
| B₂O₃ | 3,9 | 3,6 | 3,2 | 3,4 | 3,2 | 3,2 |
| Li₂O | 0,2 | 0,2 | 0,7 | 0,3 | 0,3 | 0,7 |
| Na₂O | 0,7 | 0,7 | | | | |
| BaO | 21,9 | 21,8 | 21,4 | 21,3 | 21,4 | 21,5 |
| SrO | | | 0,02 | 0,2 | 0,02 | 0,02 |
| ZnO | 13,7 | 13,9 | 13,5 | 13,0 | 13,0 | 13,4 |
| La₂O₃ | 21,3 | 21,7 | 19,7 | 19,2 | 20,2 | 19,7 |
| Gd₂O₃ | | | 3,8 | 2,8 | 2,9 | 3,8 |
| Y₂O₃ | | | 0,9 | 0,9 | 1,4 | 0,9 |
| ZrO₂ | 2,3 | 2,4 | 3,2 | 2,7 | 3,0 | 3,2 |
| Ta₂O₅ | | | 4,5 | 6,0 | 4,6 | 4,5 |
| Nb₂O₅ | 6,0 | 6,1 | 0,2 | 0,7 | 0,4 | 0,2 |
| Sb₂O₃ | 0,27 | 0,04 | 0,04 | 0,04 | 0,04 | 0,04 |

Die folgende Tabelle zeigt weitere Eigenschaften der Beispiele 12 bis 14.

| **Eigenschaft** | **Bsp. 12** | **Bsp. 13** | **Bsp. 14** |
|---|---|---|---|
| Oberer Kühlpunkt T13 [°C] | 643 | 669 | 671 |
| Erweichungspunkt T7,6 [°C] | 776 | 804 | 806 |
| Verarbeitungspunkt T4 [°C] | 952 | 979 | 982 |

Der obere Kühlpunkt T13 (englisch: "annealing point") ist die Temperatur, bei der die Viskosität 10¹³ dPas beträgt. Der Erweichungspunkt T7,6 ist die Temperatur, bei der die Viskosität 10^{7.6} dPas beträgt. Der Verarbeitungspunkt T4 ist die Temperatur, bei der die Viskosität 10⁴ dPas beträgt.

### 2. Kristallisationsbeständigkeit

Die Kristallisationsbeständigkeit der Beispielgläser 12 bis 14 wurde getestet.

Die Kristallisationsgeschwindigkeit wurde bestimmt, indem das Glas für eine Haltezeit von 5 Minuten oder von 60 Minuten in einem Gradientenofen mit aufsteigender Temperaturführung thermisch behandelt wurde. Die Kristallisationsgeschwindigkeit wurde unter Verwendung von Glasgries mit einem Durchmesser von 1,6 mm bis 4 mm bestimmt. Glasgries wurde für die thermische Behandlung im Gradientenofen auf einem Platinträger platziert. Der Träger wies jeweils eine Vertiefung zur Aufnahme eines Glaskorns auf. Durch ein Loch an der Unterseite jeder Vertiefung wurde die Kristallisationsgeschwindigkeit im Anschluss an die thermische Behandlung mikroskopisch bestimmt. Die größte Kristallisationsgeschwindigkeit, die festgestellt wurde, ist die maximale Kristallisationsgeschwindigkeit KGₘₐₓ. UEG und OEG wurden als Unter- bzw. Obergrenze des Temperaturbereichs bestimmt, in dem Kristallisation auftrat.

Die Ergebnisse sind in der folgenden Tabelle zusammengefasst

| **Eigenschaft** | **Bsp. 12** | **Bsp. 13** | **Bsp. 14** |
|---|---|---|---|
| ***5 Minuten Haltezeit*** | | | |
| UEG [°C] | < 895 | < 885 | < 895 |
| OEG [°C] | 1075 | 1090 | 1115 |
| KGₘₐₓ [µm/min] | 9,6 | 3,9 | 11,4 |
| T (KGₘₐₓ) [°C] | 1035 | 1050 | 1030 |

| ***60 Minuten Haltezeit*** | | | |
|---|---|---|---|
| UEG [°C] | n.b. | n.b. | n.b. |
| OEG [°C] | 1160 | 1130 | 1155 |
| KGₘₐₓ [µm/min] | 2,4 | 3,1 | 1,8 |
| T (KGₘₐₓ) [°C] | 985 | 1035 | 980 |

Bei 60 Minuten Haltezeit war UEG nicht bestimmbar (n.b.).

### 3. Mantelglas und Kernglas

Als Mantelglas für die Kombination mit den hierin beschriebenen Gläserm eignen sich insbesondere Borosilikatgläser. Borosilikatgläser sind allgemein Gläser enthaltend SiO₂ und B₂O₃. Borosilikatgläser enthalten insbesondere (in Gew.-%) SiO₂ von 60 bis 75%, B₂O₃ von 7 bis 25% und Al₂O₃ von 5 bis 17%. Weitere Komponenten wie insbes, Alkalien können ebenso vorhanden sein.

Abwandlungen von Borosilikatgläser sind ebenfalls möglich, die beispielsweise geringere Gehalte an B₂O₃ aufweisen können. Vorteilhaft für die Kombination mit den hierin beschriebenen Gläsern sind die in der Tabelle aufgelisteten Zusammensetzungsbereiche für Komponenten, die in insbesondere als Mantelglas für die beschriebenen Gläser als Kerngläser enthalten sein können (Angaben in Gew.-%):

| **Komposition** | **B1** | **B2** |
|---|---|---|
| SiO₂ | 60 - 75 | 60 - 75 |
| B₂O₃ | 7-25 | 0,5 - 15 |
| Na₂O | 0-8 | 1 - 15 |
| K₂O | 0-8 | 0-15 |
| Al₂O₃ | 5-17 | 3 - 10 |

Weitere optionale Komponenten wie insbesondere MgO und/oder TiO₂ und/oder CaO sind selbstverständlich möglich.

In der folgenden Tabelle sind zwei Mantelgläser mit ihrer Zusammensetzung (gemäß Analyse in Gew.-% auf Oxidbasis) als weitere Ausführungsbeispiele aufgeführt. Diese fallen prinzipiell in die Gruppe B2 der vorgenannten Tabelle. Weiterhin bedeuten n_{d} den Brechungsindex, CTE den mittleren thermischen Längenausdehnungskoeffizienten im Bereich von 20°C bis 300°C, der Erweichungspunkt T7,6 die Temperatur bei einer Viskosität von 10^{7,6} dPas, S die Säurebeständigkeit (Gewichtsverlust nach Säureangriff zur Einteilung der Gläser in Säureklassen), L die Laugenbeständigkeit (Beständigkeit von Gläsern gegen siedende wäßrige Mischlauge).

| **Beispiel** | **I** | **II** |
|---|---|---|
| **Mantelglastyp** | **Gruppe 1** | **Gruppe 2** |
| SiO₂ | 73,9 | 69,9 |
| B₂O₃ | 9,60 | 1,0 |
| Na₂O | 6,60 | 12,6 |
| K₂O | 2,56 | 3,2 |
| MgO | 0,01 | 2,7 |
| CaO | 0,63 | 5,1 |
| BaO | 0,04 | 2,1 |
| Al₂O₃ | 6,62 | 4,0 |
| TiO₂ | | 0,1 |
| F | 0,08 | 0,2 |
| CI | 0,18 | |
| Fe₂O₃ | 0,04 | |
| Sb₂O₃ | <0,005 | |
| As₂O₃ | <0,005 | 0,1 |
| Summe | 100,26 | 101 |

| Eigenschaften | | |
|---|---|---|
| n_{d} | 1,49 | 1,514 |
| CTE [ppm/K] | 5,5 | 9,1 |
| T7,6 [°C] | 790 | 720 |
| S [Klasse] | 1 | 1 |
| L [Klasse] | 2 | 2 |

Basierend auf dem Unterschied des Brechungsindex zwischen Kernglas und Mantelglas lassen sich die numerische Apertur (NA) und der Öffnungswinkel eines faseroptischen Lichtleiters (Lichtleitfaser) berechnen. Die Ergebnisse sind in der folgenden Tabelle für Kombinationen der Gläser der Erfindung als Kernglas (siehe oben, Bsp. 1 bis 4 und 6 bis 15) mit den Mantelgläsern I und II zusammengefasst. Der Ausdruck "Δ CTE" bezeichnet die Differenz zwischen CTE des Kernglases und CTE des Mantelglases.

| **Kernglas** | **Mantelglas** I | | | **Mantelglas II** | |
|---|---|---|---|---|---|
| | NA | Öffnungswinkel [°] | Δ CTE [ppm/K] | NA | Öffnungswinkel [°] |
| **Bsp. 1** | 0,88 | 123 | 2,1 | 0,84 | 114 |
| **Bsp. 2** | 0,87 | 121 | 1,9 | 0,83 | 112 |
| **Bsp. 3** | 0,88 | 124 | 2,0 | 0,84 | 114 |
| **Bsp. 4** | 0,86 | 119 | 3,0 | 0,82 | 109 |
| **Bsp. 6** | 0,88 | 123 | 2,3 | 0,84 | 114 |
| **Bsp. 7** | 0,85 | 116 | 1,4 | 0,80 | 107 |
| **Bsp. 8** | 0,86 | 119 | 1,7 | 0,82 | 110 |
| **Bsp. 9** | 0,85 | 117 | 1,3 | 0,81 | 108 |
| **Bsp. 10** | 0,85 | 117 | 1,4 | 0,81 | 108 |
| **Bsp. 11** | 0,85 | 115 | 1,3 | 0,80 | 107 |
| **Bsp. 12** | 0,86 | 118 | 1,8 | 0,81 | 109 |
| **Bsp. 13** | 0,85 | 117 | 1,5 | 0,81 | 108 |
| **Bsp. 14** | 0,85 | 117 | 1,5 | 0,81 | 108 |
| **Bsp. 15** | 0,86 | 118 | | 0,81 | 109 |

Die Erfindung soll im Weiteren anhand der Figuren erläutert werden. Die Figuren sind gleichfalls Ausführungsbeispiele.

Es zeigen
Fig. 1 in schematischer Darstellung eine Glasfaser,
Fig. 2 in einem 1. Verlaufsdiagramm die spektrale Dämpfung der erfindungsgemäßen Glasfaser im Vergleich zu einer herkömmlichen Glasfaser und
Fig. 3 in einem 2. Verlaufsdiagramm die gemessene Intensität in Abhängigkeit vom Winkel zur Abstrahlachse der Glasfaser zur Bestimmung des Öffnungswinkels.

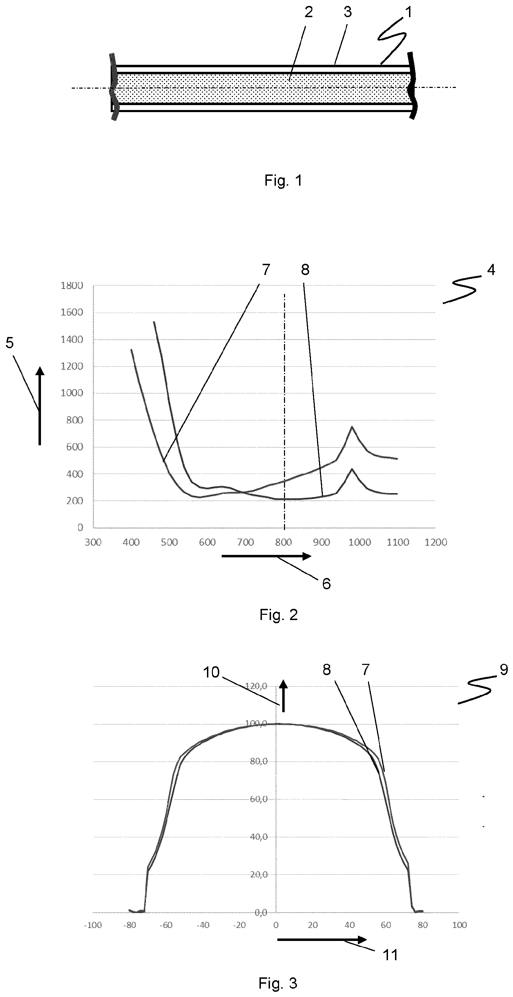

In Fig. 1 ist schematisch eine als Lichtleitelement 1 ausgebildete Glasfaser dargestellt, wobei die Glasfaser ein Kernglas 2 und ein Mantelglas 3 aufweist. Der Gesamtdurchmesser dieser so hergestellten Faser beträgt 70 µm. Im gezeigten Ausführungsbeispiel wurde als Kernglas 2 das Glas des vorgenannten Bsp. 15 eingesetzt.

Das Mantelglas 3 besteht aus einem Borosilikat-Glas, welches zuvor beschrieben wurde, insbesondere mit Zusammensetzungen enthaltend die Komponenten der Gruppe B1 oder B2, und typischerweise als Glasrohr ausgebildet ist.

Fig. 2 zeigt in einem 1. Verlaufsdiagramm 4 die Ergebnisse von spektralen Dämpfungsmessungen für eine herkömmliche Glasfaser 7 und der erfindungsgemäßen Glasfaser 8, wie sie zuvor zu Figur 1 beschrieben wurde. Hierbei ist die spektrale Dämpfung 5 in Abhängigkeit von der Wellenlänge 6 in nm des übertragenen Lichtes aufgezeichnet. Dazu werden sogenannte Mess-Lichtleiter mit bestimmter Länge, z.B. 1 m oder 3 m Länge, hergestellt und die Transmission unter Berücksichtigung der Reflektionsverluste and den Endflächen gemessen. Aus der der Transmission und der der Länge des Lichtleiters lässt sich dann die längenunabhängige spektrale Dämpfung errechnen, welche im Diagramm in dB/km angegeben ist, woraus sich dann auch die für derartige Lichtleiter eher gebräuchliche Angabe dB/m mit 1000 dB/km = 1 dB/m ableiten lässt.

Besonders vorteilhaft ist hierbei, dass insbesondere die spektrale Dämpfung 5 der erfindungsgemäßen Glasfaser 7 im Bereich von nahem Infrarotlicht (NIR), z.B. bei 800 nm Wellenlänge 6 niedriger liegt als bei einer herkömmlichen Glasfaser 8, wie sie beispielsweise eingangs beschrieben wurde. Diese liegt im gezeigten Beispiel bei 200 db/km bzw. 0,2 dB/m ggü. ca. 350 dB/km bzw. 0,35 dB/m bei der herkömmlichen Glasfaser. Dies ist insbesondere bei spektroskopischen Untersuchen in medizintechnischen Anwendungen, wie beispielsweise bei Endoskopen von Vorteil, da insbesondere Gewebe-Analysen im NIR-Bereich ein verbessertes Signal-Rausch-Verhältnis ermöglichen und damit z.B. insbesondere Kontraste in einer Bildgebung erhöht werden können. Zudem hat sich gezeigt, dass etwaige Schwankungen bei unterschiedlichen Schmelzen in diesem Wellenlängenbereich wenig stark ausfallen, als bei herkömmlichen Glasfasern, was insbesondere der erfindungsgemäßen Zusammensetzung zu geschrieben wird.

Andererseits zeigt aber das 1. Verlaufsdiagramm 4 auch, dass die sogenannte UV- oder Blaukante für die zuvor beschriebene erfindungsgemäße Glasfaser 8 noch deutlich zu höheren Wellenlängen ggü. der herkömmlichen Glasfaser 7 verschoben ist, d.h. die spektrale Dämpfung 5 im blauen Wellenlängenbereich zwischen 400 nm und 500 nm deutlich höher ist, als bei der herkömmlichen Glasfaser 7. Die Lage der UV- oder Blaukante lässt sich, wie oben beschrieben, durch den Y₂O₃- oder Gd₂O₃-Gehalt oder deren Kombination einstellen.

Eine etwas höhere spektrale Dämpfung 5 im "Blauen" wirkt sich insbesondere dann nicht nachteilig aus, wenn derartige Glasfasern insbesondere in endoskopischen Geräten eingesetzt werden. Hier liegt die typische Anwendungslänge bei max. 1 m bis 2 m, sodass der sogenannte "Yellow-Shift" bzw. eine Farbverschiebung zu Gelb eher gering ausfällt, was insbesondere für Single-Use-Endoskope mit typ. Anwendungslängen von < 1 m unkritisch ist. Vorteilhaft kann die leicht erhöhte Dämpfung im Blauen sogar genutzt werden, wenn der das Lichtleitelement, beispielsweise eine Glasfaser, mit einer Lichtquelle gekoppelt ist, welche stärker im Blauen emittiert, und/oder wenn Gewebe untersucht werden soll, welches empfindlich auf blaues Licht und damit höher energetische Anteile des Spektrums reagieren.

Figur 3 zeigt in einem 2. Verlaufsdiagramm 9 die Ergebnisse zur Bestimmung des Öffnungswinkels der Glasfasern. Dazu wird üblicherweise mit einem Sensor die Intensität 10 des vom Lichtleiter abgestrahlten Lichtes in Abhängigkeit vom Winkel 11 zur Abstrahlachse des Lichtleiters gemessen, wie dies für eine herkömmliche Glasfaser 7 und der erfindungsgemäßen Glasfaser 8 gemäß der o.g. und in Figur 1 beschriebenen Glasfaser dargestellt ist. Der sogenannte 2α-Öffnungswinkel ergibt sich dann definitionsgemäß aus den Winkeln 11, bei denen die Intensität 10 auf 50% des Maximalwertes bei 0° abgefallen ist.

Wie Figur 3 zeigt, weisen die beiden Glasfasern 7,8 einen nahezu identischen Öffnungswinkel von 2α = 2 x ca. 60° = 120° auf, was einer numerischen Apertur NA = 0,86 entspricht, wobei gilt NA = sin⁻¹(α), und damit beide Glasfasern als Weitwinkel-Glasfasern bezeichnet werden können, wie sie in endoskopischen Anwendungen vorteilhaft eingesetzt werden können, um entsprechend der diagonalen Öffnungswinkel von Kamera-Chips, das Gesichtsfeld derartiger Kameras schattenfrei ausleuchten zu können.

Weitere Untersuchungen an der erfindungsgemäßen Glasfaser 8 im Vergleich zur herkömmlichen Glasfaser 7, welche beide als Weit-Winkelfasern gelten und einen Faserdurchmesser von 70 µm aufweisen, betrifft das Festigkeitsniveau. Dazu wurden jeweils 30 Glasfaserproben mit gleicher Länge in einer Zugprüfmaschine eingespannt und die Spannung bis zum Bruch der Fasern gemessen. Demnach wurden für beide Glasfaser 7,8 nahezu identische Zugfestigkeiten um die 1000 MPa Bruchspannung gemessen, wobei sich die statistischen Schwankungsbereiche derart überlappten, dass von dem gleichen Zugfestigkeitsniveau auszugehen ist.

### Bezugszeichen:

- 1: Glasartikel, Lichteitelement
- 2: Kernglas
- 3: Mantelglas
- 4: 1. Verlaufsdiagramm
- 5: Spektrale Dämpfung
- 6: Wellenlänge
- 7: Herkömmliche Glasfaser
- 8: Erfindungsgemäßes Lichtleitelement, Glasfaser
- 9: 2. Verlaufsdiagramm
- 10: Intensität
- 11: Winkel

## Patentansprüche

1. Glas umfassend SiO₂ sowie mindestens eine der beiden Komponenten Gd₂O₃ und Y₂O₃, wobei das Verhältnis der Summe der Gewichtsanteile von Gd₂O₃ und Y₂O₃ zum Gewichtsanteil von SiO₂ mindestens 0,01 beträgt, wobei der Anteil an Ta₂O₅ höchstens 10 Gew.-% beträgt, wobei der Anteil an ZrO₂ mindestens 0,1 Gew.-% beträgt, bevorzugt von 0,1 - 10 Gew.-%, wobei das Verhältnis des Gewichtsanteils an B₂O₃ zum Gewichtsanteil an SiO₂ höchstens 0,50 beträgt.

2. Glas nach mindestens einem der vorhergehenden Ansprüche, wobei die Summe der Gewichtsanteile von Gd₂O₃ und Y₂O₃ mindestens 0,2 Gew.-% beträgt.

3. Glas nach mindestens einem der vorhergehenden Ansprüche, wobei die Summe der Anteile von BaO und La₂O₃ mindestens 20 Gew.-% beträgt.

4. Glas nach mindestens einem der vorhergehenden Ansprüche, umfassend die folgenden Komponenten in den angegebenen Anteilen (in Gew.-):
| Komponente | Min (Gew.-%) | Max (Gew.-%) |
|---|---|---|
| SiO₂ | 10 | 55 |
| B₂O₃ | 0 | 25 |
| CaO | 0 | 5,0 |
| BaO | 0 | 50 |
| SrO | 0 | 5,0 |
| ZnO | 0 | 30 |
| La₂O₃ | 0 | 70 |
| Gd₂O₃ | 0 | 15 |
| Y₂O₃ | 0 | 15 |
| ZrO₂ | 0,1 | 10 |
| Ta₂O₅ | 0 | 10 |
| Nb₂O₅ | 0 | 10 |
| Σ R₂O | 0 | 10 |

5. Glas nach mindestens einem der vorhergehenden Ansprüche, wobei der Anteil an Y₂O₃ und Gd₂O₃ jeweils mindestens 0,1 Gew.-% beträgt, bevorzugt jeweils mindestens 0,2 Gew.-%.

6. Glas nach mindestens einem der vorhergehenden Ansprüche, wobei der Gewichtsanteil von Y₂O₃ größer ist als der Gewichtsanteil von Nb₂O₅.

7. Glas nach mindestens einem der vorhergehenden Ansprüche, wobei der Anteil von LaₛO₃ mindestens 15 Gew.-%, bevorzugt mindestens 16 Gew.-%, besonders bevorzugt mindestes 17 Gew.-% beträgt.

8. Glas nach Anspruch 7, wobei der Anteil an La₂O₃ höchstens 40 Gew.-% beträgt, bevorzugt höchstens 38 Gew.-%, besonders bevorzugt höchstens 37 Gew.-%.

9. Glas nach mindestens einem der vorhergehenden Ansprüche, wobei das Verhältnis des Gewichtsanteils von La₂O₃ zur Summe der Gewichtsanteile von Gd₂O₃ und Y₂O₃ höchstens 10 beträgt.

10. Glas nach mindestens einem der vorhergehenden Ansprüche, wobei die Summe der Anteile von La₂O₃, Gd₂O₃ und Y₂O₃ mindestens 10 Gew.-% beträgt.

11. Glas nach mindestens einem der vorhergehenden Ansprüche, wobei die untere Entglasungsgrenze UEG mindestens 650°C beträgt, wenn das Glas für eine Haltezeit von 5 Minuten in einem Gradientenofen mit aufsteigender Temperaturführung thermisch behandelt wird.

12. Glas nach mindestens einem der vorhergehenden Ansprüche, wobei die Differenz zwischen der unteren Entglasungsgrenze UEG und dem Erweichungspunkt T7,6 mindestens 50 K beträgt.

13. Lichtleitelement (1), insbesondere Glasfaser oder Lichtleitstab, umfassend oder bestehend aus einem Glas nach mindestens einem der vorhergehenden Ansprüche, wobei der Brechungsindex n_{d} des Glases in einem Bereich von 1,65 bis 1,80 liegt.

14. Lichtleitelement (1) nach Anspruch 13, wobei das Lichtleitelement (1) das Glas nach mindestens einem der Ansprüche 1 bis 12 als Kernglas (2) umfasst und wobei das Lichtleitelement (1) ein Mantelglas (3) umfasst, dass das Kernglas (2) ummantelt.

15. Lichtleitelement (1) nach Anspruch 14, wobei das Mantelglas (3) aus einem Borosilikatglas besteht.

16. Lichtleitelement (1) nach Anspruch 14, wobei das Mantelglas (2) die folgenden Komponenten der Kompositionen B1 oder B2 umfasst
| **Komposition** | **B1** | **B2** |
|---|---|---|
| SiO₂ | 60 - 75 | 60 - 75 |
| B₂O₃ | 7-25 | 0,5 - 15 |
| Na₂O | 0-8 | 1 - 15 |
| K₂O | 0-8 | 0-15 |
| Al₂O₃ | 5 - 17 | 3-10 |

17. Lichtleitelement (1) nach mindestens einem der Ansprüche 13 bis 16 mit einer numerischen Apertur (NA) von mindestens 0,82, vorzugsweise mindestens 0,85.

18. Lichtleitelement (1) nach mindestens einem der Ansprüche 13 bis 17 mit einer spektralen Dämpfung im nahen IR-Bereich bei einer Wellenlänge (6) von 800 nm von höchstens 0,3 dB/m, vorzugsweise höchstens 0,2 dB/m.

19. Verfahren zur Herstellung des Glases nach mindestens einem der Ansprüche 1 bis 12 oder eines Lichtleitelements (1) nach mindestens einem der Ansprüche 13 bis 18, umfassend die folgenden Schritte:
• Schmelzen der Glasrohstoffe,
• Kühlen des erhaltenen Glases oder Glasartikels,
• Bearbeiten der Glasschmelze, insbesondere mittels Down Draw, Overflow Fusion, Floaten oder Rohrzug, insbesondere Danner-Verfahren, Vello-Verfahren oder A-Zug-Verfahren (Vertikalzug-Verfahren).

20. Verwendung eines Glases nach mindestens einem der Ansprüche 1 bis 12 als Kernglas (2) in einem Licht- und/oder Bildleiter.

21. Verwendung eines Lichtleitelements (1) nach mindestens einem der Ansprüche 13 bis 18 in endoskopische Anwendungen, insbesondere Endoskopen, vorteilhaft Single-Use-Endoskopen, in Projektionseinrichtungen, in der optische Nachrichtentechnik, Automotive Anwendungen, Lasertechnologie und Desinfektion.
